(19) Europäisches Patentamt
European Patent Office
Office européen des brevets

(11) EP 3 328 850 B1

(12) EUROPÄISCHE PATENTSCHRIFT

(45) Veröffentlichungstag und Bekanntmachung des Hinweises auf die Patenterteilung:
**11.11.2020 Patentblatt 2020/46**

(21) Anmeldenummer: **16745623.5**

(22) Anmeldetag: **28.07.2016**

(51) Int Cl.:
***C07D 401/14*** (2006.01) ***C07D 401/04*** (2006.01)
***C07D 403/10*** (2006.01) ***C07D 405/04*** (2006.01)
***C07D 405/10*** (2006.01) ***C07D 409/04*** (2006.01)
***C07D 239/74*** (2006.01) ***C07D 239/82*** (2006.01)
***H01L 51/00*** (2006.01)

(86) Internationale Anmeldenummer:
**PCT/EP2016/001308**

(87) Internationale Veröffentlichungsnummer:
**WO 2017/016667 (02.02.2017 Gazette 2017/05)**

# (54) VERBINDUNGEN MIT FLUOREN-STRUKTUREN

COMPOUNDS HAVING FLUORENE STRUCTURES

COMPOSÉS À STRUCTURES FLUORÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2015 EP 15178816**

(43) Veröffentlichungstag der Anmeldung:
**06.06.2018 Patentblatt 2018/23**

(73) Patentinhaber: **Merck Patent GmbH**
**64293 Darmstadt (DE)**

(72) Erfinder:
- **PARHAM, Amir Hossain**
  **60486 Frankfurt am Main (DE)**
- **EBERLE, Thomas**
  **76829 Landau (DE)**
- **JATSCH, Anja**
  **60489 Frankfurt am Main (DE)**
- **GROSSMANN, Tobias**
  **64297 Darmstadt (DE)**
- **KROEBER, Jonas Valentin**
  **60311 Frankfurt am Main (DE)**

(56) Entgegenhaltungen:
**WO-A1-2012/141499**
**WO-A1-2012/165832**
**WO-A2-2016/003225**
**KR-A- 20120 116 272**
**KR-A- 20140 020 208**
**KR-A- 20140 030 786**
**US-A1- 2014 284 580**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 328 850 B1

Printed by Jouve, 75001 PARIS (FR)

## Beschreibung

Die vorliegende Erfindung beschreibt Fluorenderivate, welche mit elektronentransportierenden Gruppen substituiert sind, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden als Matrixmaterialien für phosphoreszierende Verbindungen sowie als Elektronentransportmaterialien häufig heteroaromatische Verbindungen eingessetzt, wie zum Beispiel Triazinderivate oder Benzimidazolderivate. Als Matrixmaterialien für phosphoreszierende Verbindungen eignen sich auch Carbazolderivate. Bekannt für diese Funktion sind beispielsweise Spirobifluorenderivate, welche in 2-Position mit Triazingruppen substituiert sind, wie in WO 2010/015306 und WO 2010/072300 offenbart. Bei diesen Verbindungen gibt es sowohl bei fluoreszierenden wie auch bei phosphoreszierenden OLEDs weiterhin Verbesserungsbedarf, insbesondere hinsichtlich Effizienz, Lebensdauer und Betriebsspannung bei Verwendung in einer organischen Elektrolumineszenzvorrichtung. Ferner sind aus der JP 2014183315A heterocyclische Verbindungen bekannt, die Fluoren-Strukturen aufweisen. Ähnliche Verbindungen sind weiterhin aus der EP 2842954 A1 bekannt.

WO 2012/165832 A1, WO 2012/141499 A1 und KR20120116272 A offenbaren Fluorene, die mit Chinazolinen und carbazolhaltigen Gruppen substiuiert sind, sowie deren Verwendung in organischen Elektrolumneszenzvorrichtungen.

Generell besteht bei diesen Materialien für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Ferner sollten die Matrixmaterialien insbesondere für phosphoreszierende Emitter, welche Ketoketonatliganden enthalten, geeignet sein.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Es wurde überraschend gefunden, dass Vorrichtungen, die Verbindungen umfassend Strukturen gemäß der folgenden Formeln enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß Anspruch 1,
Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen

der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

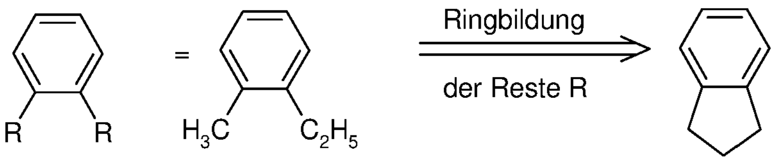


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

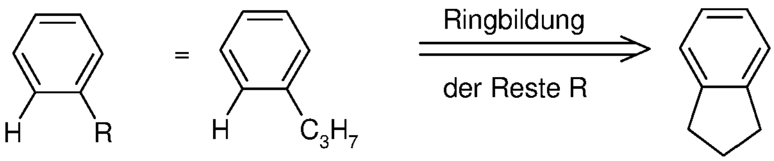


Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Darüber hinaus sind Verbindungen bevorzugt, die dadurch gekennzeichnet sind, dass in Formeln (III) und/oder (IV) nicht mehr als zwei, vorzugsweise nicht mehr als eine Gruppe X für N steht, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

Ferner kann vorgesehen sein, dass die Reste $R^1$ der Gruppen X in den Formeln (III) und/oder (IV) mit den Ringatomen der Fluorenstruktur kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ der Gruppen X in den Formeln (III) und/oder (IV) mit den Ringatomen der Fluorenstruktur kein Ringsystem bilden. Dies schließt die Bildung eines Ringsystems mit möglichen Substituenten $R^2$, $R^3$ ein, die an die Reste $R^1$ gebunden sein können.

Vorzugsweise können die erfindungsgemäßen Verbindungen Strukturen gemäß Formeln (IIIa) und/oder (IVa) umfassen

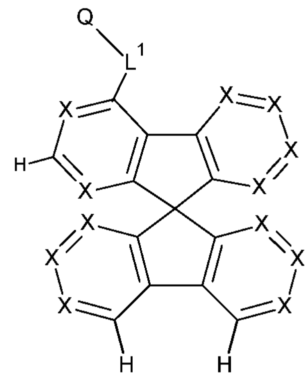

Formel (IIIa)

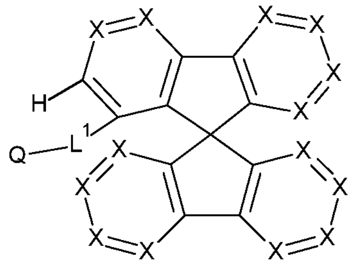

Formel (IVa)

worin die Symbole X, $R^1$, $L^1$ und Q die bereits dargelegte Bedeutung aufweisen.

Weiterhin kann vorgesehen sein, dass die Substitutenten $R^1$ der Fluorenstruktur in den Formeln (IIIa) und/oder (IVa) mit den Ringatomen der Fluorenstruktur kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substitutenten $R^1$ der Fluorenstruktur in den Formeln (IIIa) und/oder (IVa) mit den Ringatomen der Fluorenstruktur kein Ringsystem bilden. Dies schließt die Bildung eines Ringsystems mit möglichen Substituenten $R^2$, $R^3$ ein, die an die Reste $R^1$ gebunden sein können.

Ferner kann vorgesehen sein, dass in Formeln (IIIa) und (IVa) nicht mehr als zwei, vorzugsweise nicht mehr als eine Gruppe X für N steht, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

In einer weiterhin bevorzugten Ausführungsform können die erfindungsgemäßen Verbindungen Strukturen gemäß Formeln (IIIb) und/oder (IVb) umfassen

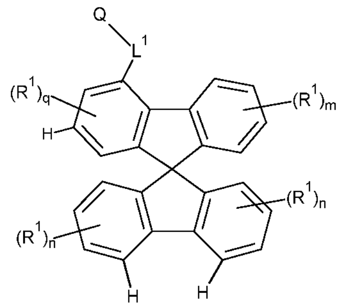

Formel (IIIb)

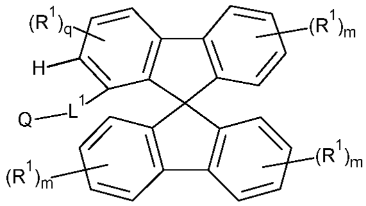

Formel (IVb)

worin die Symbole Q, $L^1$ und $R^1$ die zuvor dargelegte Bedeutung aufweisen und m 0, 1, 2, 3 oder 4, vorzugsweise 0, 1 oder 2, n 0, 1, 2 oder 3, vorzugsweise 0, 1 oder 2 und q 0, 1 oder 2, vorzugsweise 0 oder 1 ist.

Gemäß einer bevorzugten Ausgestaltung sind Verbindungen, umfassend Strukturen gemäß Formel (III), (lila), (IIIb), (IV), (IVa) und (IVb), durch Strukturen der Formel (III), (lila), (IIIb), (IV), (IVa) und (IVb) darstellbar. Vorzugsweise weisen Verbindungen, umfassend Strukturen gemäß Formel (III), (lila), (IIIb), (IV), (IVa) und (IVb), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Die Gruppe Q stellt eine elektronentransportierende Gruppe dar. Elektronentransportierende Gruppen sind in der Fachwelt weithin bekannt und fördern die Fähigkeit von Verbindungen Elektronen zu transportieren und/oder zu leiten.

Weiterhin zeigen die erfindungsgemäßen Verbindungen gemäß bei denen in Formeln (III), (lila), (IIIb), (IV), (IVa) und (IVb) die Gruppe Q mindestens eine Struktur umfasst, die aus der Gruppe Pyrimidine, Chinazoline, Chinoxaline, Chinoline und Isochinoline ausgewählt ist.

Darüber hinaus sind Verbindungen bevorzugt, die dadurch gekennzeichnet sind, dass die Gruppe Q in Formel (III), (lila), (IIIb), (IV), (IVa) und (IVb) ein heteroaromatisches Ringsystem mit mindestens zwei kondensierten Ringen ist, welches durch einen oder mehrere Reste $R^1$ substituiert sein kann, vorzugsweise jedoch unsubstituiert ist, wobei die Ringatome der mindestens zwei kondensierten Ringe mindestens ein Stickstoffatom umfassen, wobei $R^1$ die zuvor dargelegte Bedeutung aufweist.

In einer weiteren Ausgestaltung kann vorgesehen sein, dass die unter anderem in den Formeln (III), (lila), (IIIb), (IV), (IVa) und (IVb) dargelegte Gruppe Q ein heteroaromatisches Ringsystem darstellt, wobei die Ringatome 1 bis 4 Stickstoffatome umfassen und das Ringsystem durch einen oder mehrere Reste $R^1$ substituiert sein kann, vorzugsweise jedoch unsubstituiert ist, wobei $R^1$ die zuvor dargelegte Bedeutung aufweist.

Ferner kann vorgesehen sein, dass die unter anderem in den Formeln (III), (lila), (IIIb), (IV), (IVa) und (IVb) dargelegte Gruppe Q ein heteroaromatisches Ringsystem mit 9 bis 14, vorzugsweise 10 Ringatomen darstellt, welches durch einen oder mehrere Reste $R^1$ substituiert sein kann, wobei $R^1$ die zuvor dargelegte Bedeutung aufweist, vorzugsweise jedoch unsubstituiert ist.

Vorzugsweise kann die unter anderem in den Formeln (III), (lila), (IIIb), (IV), (IVa) und (IVb) dargelegte Gruppe Q ausgewählt sein aus Strukturen der Formeln (Q-1), (Q-2) und/oder (Q-3)

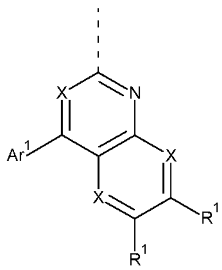

Formel (Q-1)

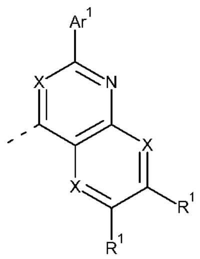

Formel (Q-2)

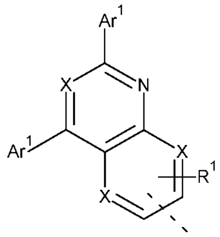

Formel (Q-3)

wobei die Symbole X und $R^1$ genannte Bedeutung aufweisen, die gestrichelte Bindung die Anbindungsposition markiert und $Ar^1$ ein aromatisches oder heteroaromatisches Ringsystem mit 6 bis 40 C-Atomen, das jeweils mit einem oder

mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^1$ bzw. $R^2$ ein mono- oder polycyclisches, aliphatisches Ringsystem bilden können, das mit einem oder mehreren Resten $R^3$ substituiert sein kann, wobei die Symbole $R^1$ und $R^2$ die zuvor genannte Bedeutung aufweisen.

In einer weiteren Ausführungsform kann unter anderem in den Formeln (III), (Iila), (IIIb), (IV), (IVa) und (IVb) dargelegte Gruppe Q ausgewählt sein aus Strukturen der Formeln (Q-4), (Q-5), (Q-6), (Q-7), (Q-8) (Q-9), (Q-10), (Q-11), (Q-12), (Q-13), (Q-14), und/oder (Q-15)

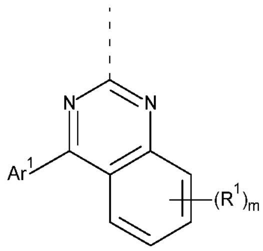

Formel (Q-4)

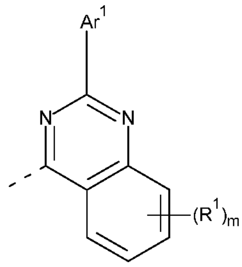

Formel (Q-5)

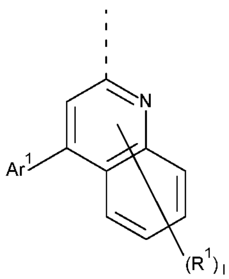

Formel (Q-6)

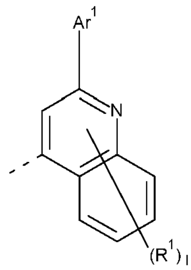

Formel (Q-7)

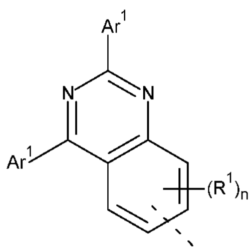

Formel (Q-8)

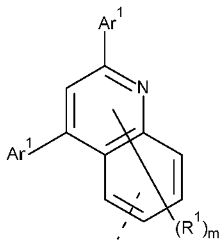

Formel (Q-9)

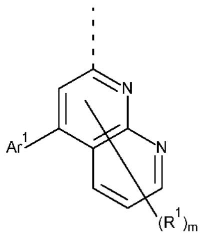

Formel (Q-10)

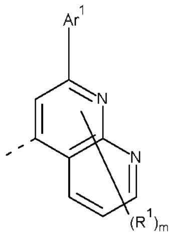

Formel (Q-11)

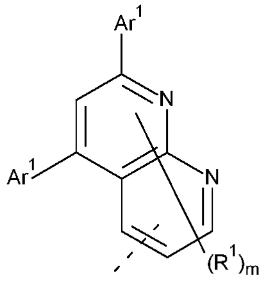

Formel (Q-12)

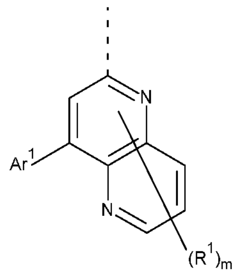

Formel (Q-13)

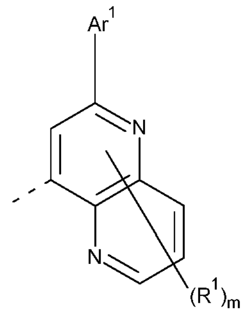

Formel (Q-14)

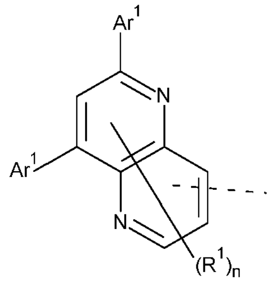

Formel (Q-15)

worin die Symbole $Ar^1$ und $R^1$ die zuvor dargelegt Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1 und l1, 2, 3, 4 oder 5, vorzugsweise 0, 1 oder 2 ist.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise das C- oder N-Atom der zuvor dargestellten Gruppen (Q-1) bis (Q-15).

In einer weiteren bevorzugten Ausführungsform der Erfindung steht $Ar^1$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^2$ substituiert sein kann, bevorzugt aber unsubstituiert ist, wobei $R^2$ die zuvor dargestellte Bedeutung aufweisen kann. Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

In einer bevorzugten Ausführungsform kann der Rest $Ar^1$ und/oder ein an $Ar^1$ gebundener Substituent $R^2$ gemäß den Formeln (Q-1) bis (Q-15) ein Strukturelement umfassen, welches ausgewählt ist aus Strukturen gemäß Formeln (Q-1) bis (Q-15) und/oder aus Strukturen gemäß Formel (Q-16) oder (Q-17)

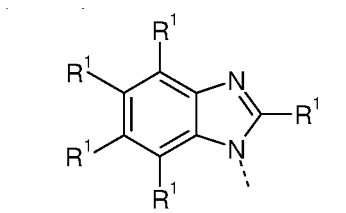

Formel (Q-16)

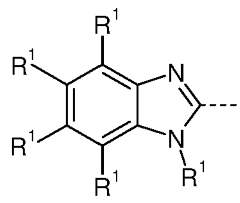

Formel (Q-17)

wobei das Symbol $R^1$ die zuvor, insbesondere für Formel (I) und/oder (II) genannte Bedeutung aufweist und die gestrichelten Bindungen jeweils die Anbindungspositionen markieren, an die das Strukturelement gemäß Formel (Q-16) oder (Q-17) an andere Strukturelemente des Rests $Ar^1$ oder an den Substituent $R^2$ oder an eine Struktur gemäß Formeln (Q-1) bis (Q-15) bindet.

Mit Vorteil stellt $Ar^1$ in den Formeln (Q-1) bis (Q-15) ein aromatisches Ringsystem mit 6 bis 12 aromatischen Ringatomen dar, welches mit einem oder mehreren Resten $R^2$ substituiert sein kann, vorzugsweise aber unsubstituiert ist, wobei $R^2$ die zuvor dargestellte Bedeutung aufweisen kann.

Bevorzugt bilden die Reste $R^1$ in den Formeln (Q-1) bis (Q-17) mit den Ringatomen der Heteroarylgruppe, an die die Reste $R^1$ gebunden sind, kein kondensiertes Ringsystem. Dies schließt die Bildung eines kondensierten Ringsystems mit möglichen Substituenten $R^2$, $R^3$ ein, die an die Reste $R^1$ gebunden sein können.

Bevorzugt bilden die Reste $R^2$ in den Formeln (Q-1) bis (Q-15) mit den Ringatomen der Arylgruppe oder Heteroarylgruppe $Ar^1$, an die die Reste $R^2$ gebunden sind, kein kondensiertes Ringsystem. Dies schließt die Bildung eines kondensierten Ringsystems mit möglichen Substituenten $R^3$ ein, die an die Reste $R^2$ gebunden sein können.

Wenn X für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann. Die Gruppe $Ar^1$ kann die zuvor, insbesondere für Struktur (Q-1) genannte Bedeutung aufweisen. Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise das C-, N- oder das P-Atom der Gruppen $N(Ar^1)_2$, $C(=O)Ar^1$, $P(=O)(Ar^1)2$.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist. Die Gruppe $Ar^1$ kann die zuvor, insbesondere für Struktur (Q-1) genannte Bedeutung aufweisen. Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise das N- Atom der Gruppe $N(Ar^1)_2$.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (III), (IV), (lila), (IVa), (IIIb) und/oder (IVb) mindestens ein Rest $R^1$ und/oder $Ar^1$ für eine Gruppe steht, die ausgewählt ist aus den Formeln ($R^1$-1) bis ($R^1$-79)

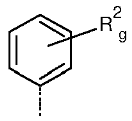

Formel ($R^1$-1)

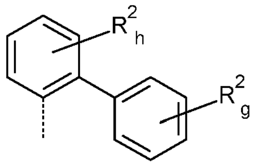

Formel ($R^1$-2)

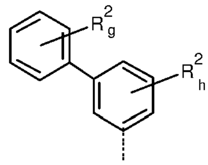

Formel ($R^1$-3)

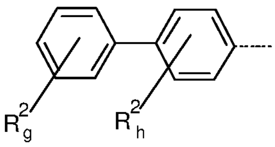

Formel ($R^1$-4)

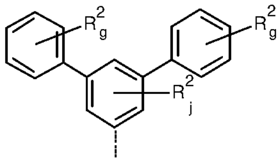

Formel ($R^1$-5)

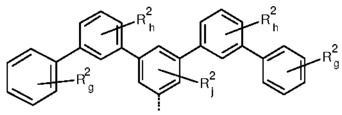

Formel ($R^1$-6)

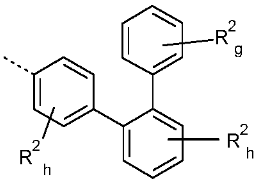

Formel ($R^1$-7)

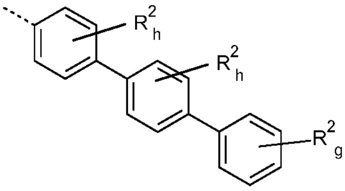

Formel ($R^1$-8)

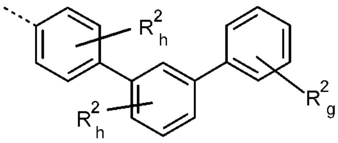

Formel ($R^1$-9)

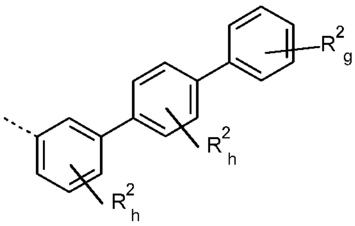

Formel ($R^1$-10)

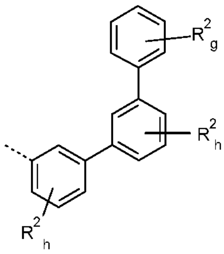

Formel ($R^1$-11)

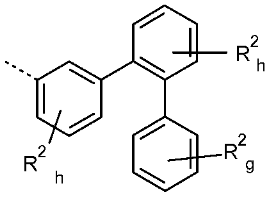

Formel ($R^1$-12)

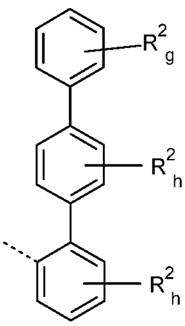

Formel ($R^1$-13)

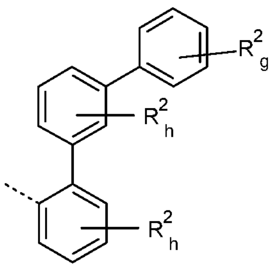

Formel ($R^1$-14)

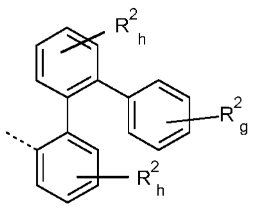

Formel ($R^1$-15)

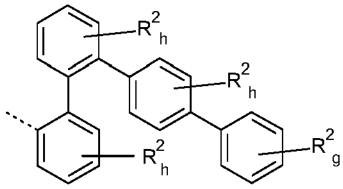

Formel ($R^1$-16)

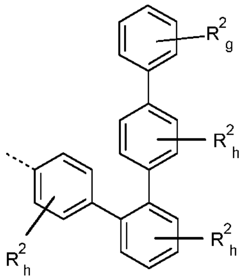

Formel ($R^1$-17)

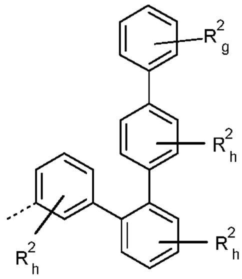

Formel ($R^1$-18)

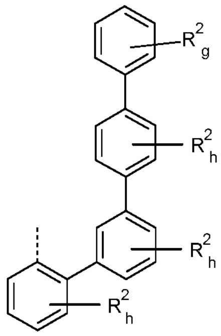

Formel ($R^1$-19)

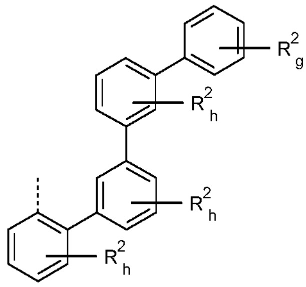

Formel ($R^1$-20)

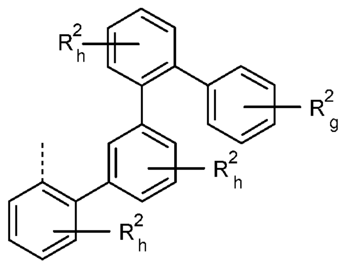

Formel ($R^1$-21)

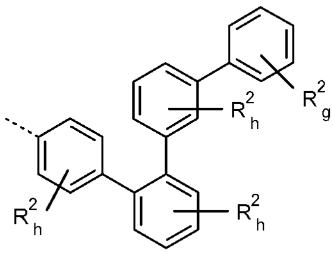

Formel ($R^1$-22)

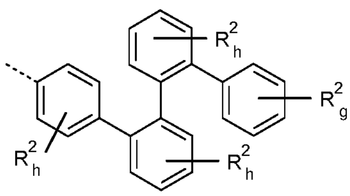

Formel ($R^1$-23)

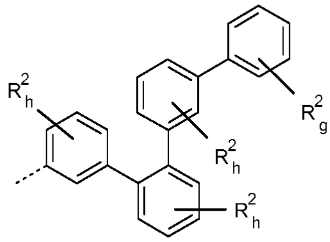

Formel ($R^1$-24)

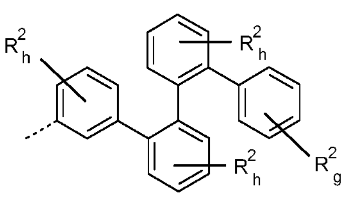

Formel ($R^1$-25)

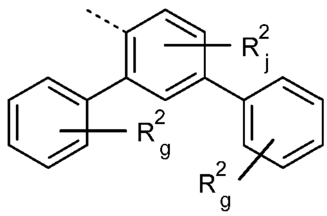

Formel ($R^1$-26)

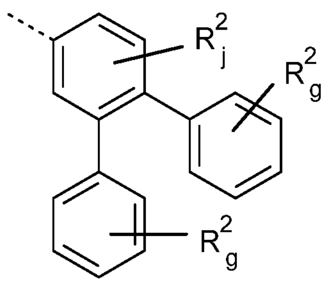

Formel ($R^1$-27)

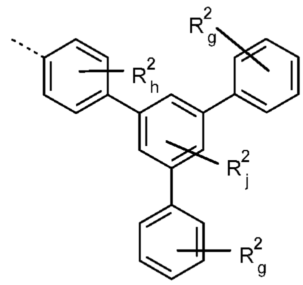

Formel ($R^1$-28)

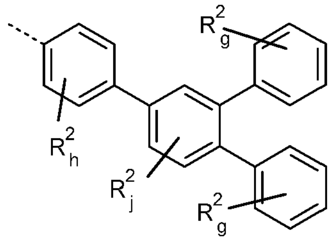

Formel ($R^1$-29)

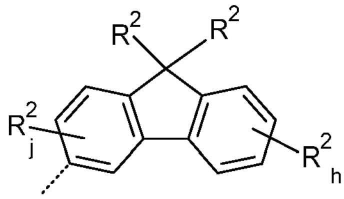

Formel ($R^1$-30)

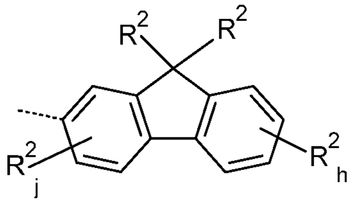

Formel ($R^1$-31)

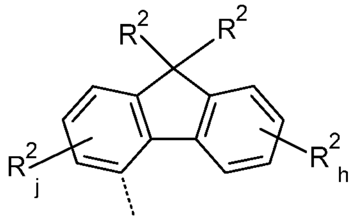

Formel ($R^1$-32)

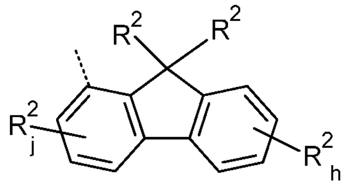

Formel ($R^1$-33)

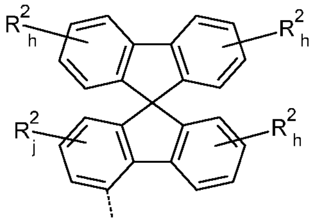

Formel ($R^1$-34)

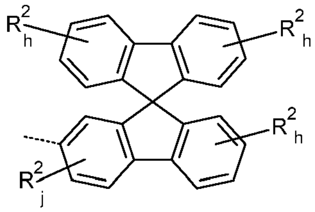

Formel ($R^1$-35)

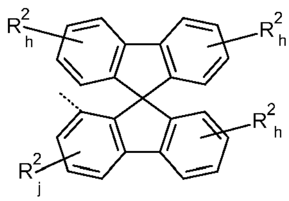

Formel ($R^1$-36)

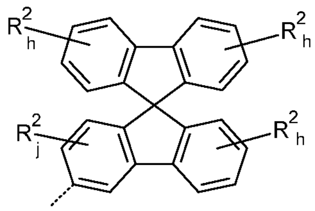

Formel ($R^1$-37)

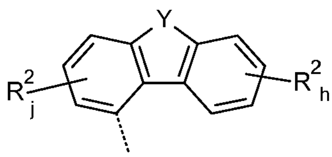

Formel ($R^1$-38)

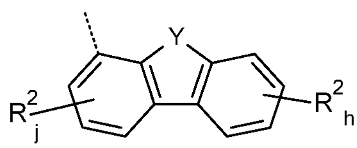

Formel ($R^1$-39)

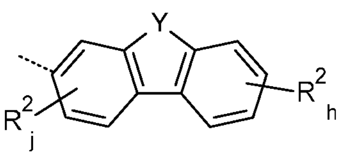

Formel ($R^1$-40)

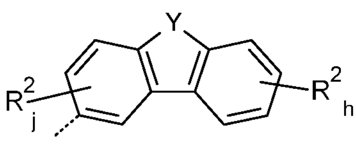

Formel ($R^1$-41)

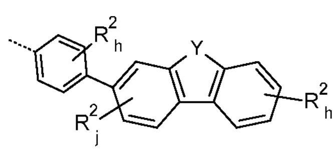

Formel ($R^1$-42)

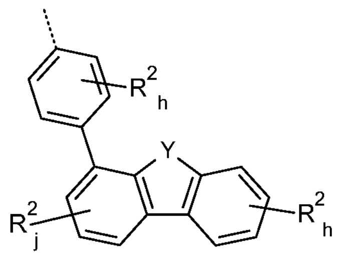

Formel ($R^1$-43)

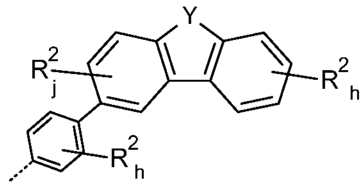

Formel ($R^1$-44)

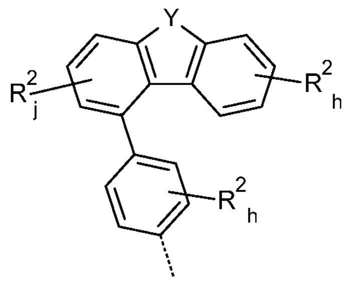

Formel ($R^1$-45)

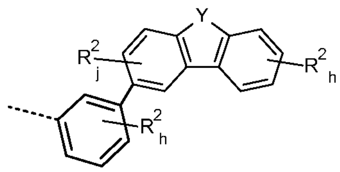

Formel ($R^1$-46)

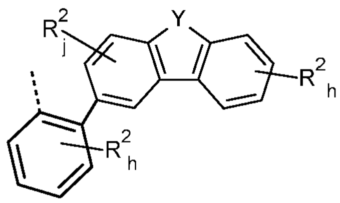

Formel ($R^1$-47)

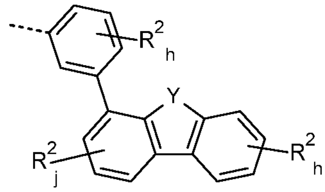

Formel ($R^1$-48)

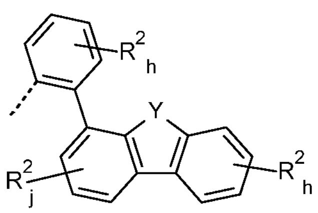

Formel ($R^1$-49)

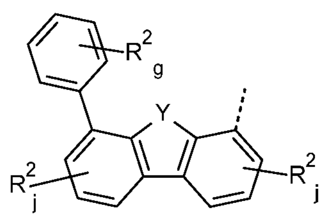

Formel ($R^1$-50)

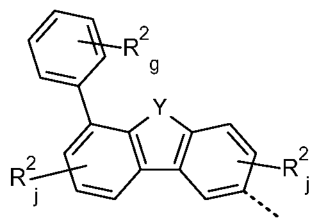

Formel ($R^1$-51)

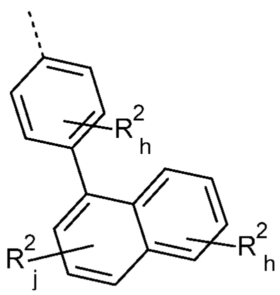

Formel ($R^1$-52)

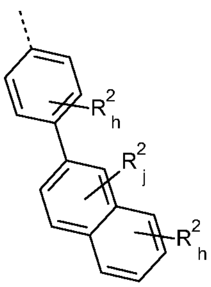

Formel ($R^1$-53)

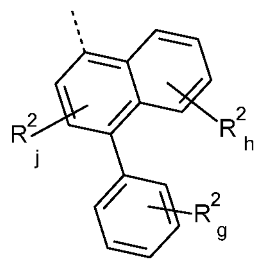

Formel ($R^1$-54)

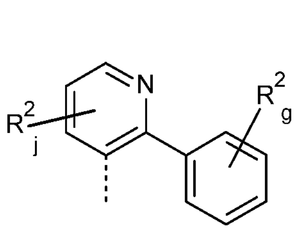

Formel ($R^1$-55)

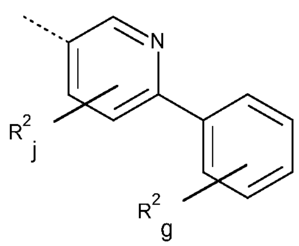

Formel ($R^1$-56)

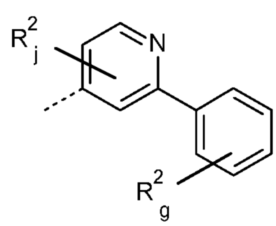

Formel ($R^1$-57)

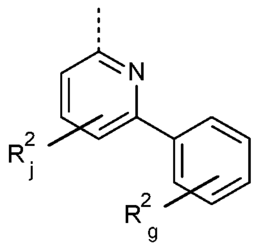

Formel ($R^1$-58)

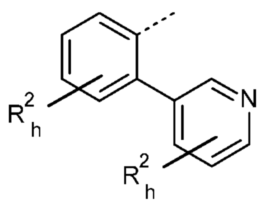

Formel ($R^1$-59)

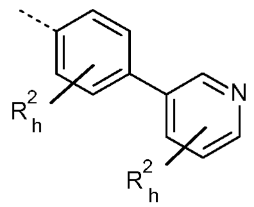

Formel ($R^1$-60)

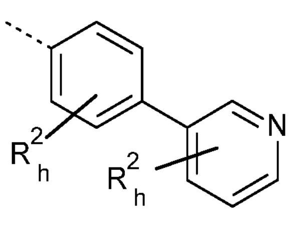

Formel ($R^1$-61)

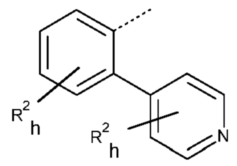

Formel ($R^1$-62)

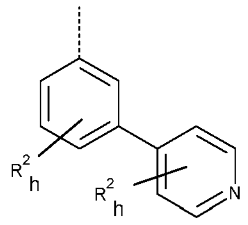

Formel ($R^1$-63)

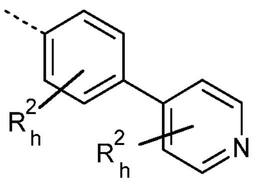

Formel ($R^1$-64)

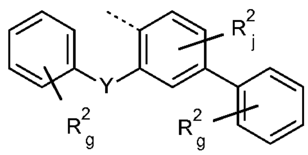

Formel ($R^1$-65)

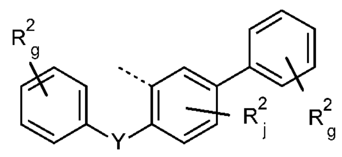

Formel ($R^1$-66)

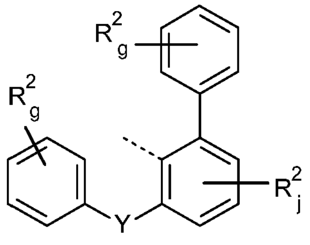

Formel ($R^1$-67)

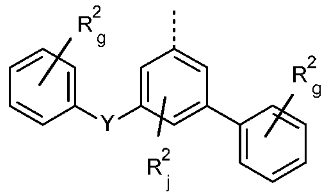

Formel ($R^1$-68)

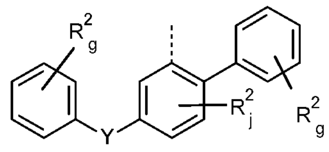

Formel ($R^1$-69)

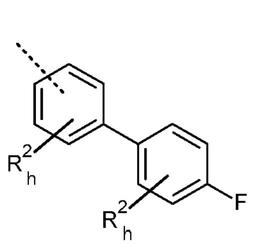

Formel ($R^1$-70)

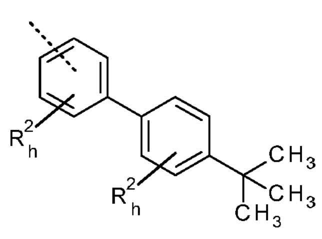

Formel ($R^1$-71)

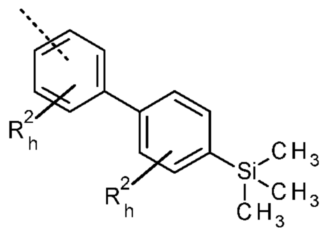

Formel ($R^1$-72)

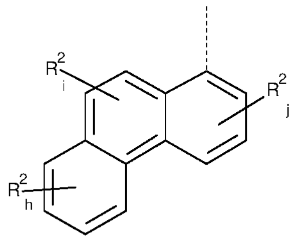

Formel ($R^1$-73)

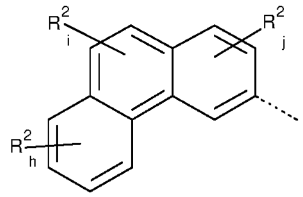

Formel ($R^1$-74)

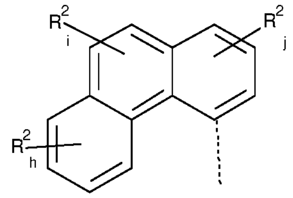

Formel ($R^1$-75)

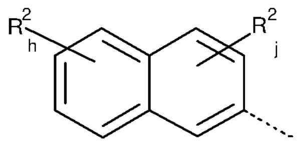

Formel ($R^1$-76)

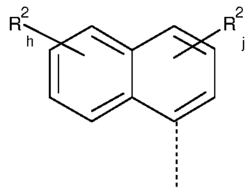

Formel ($R^1$-77)

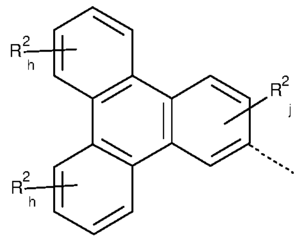

Formel ($R^1$-78)

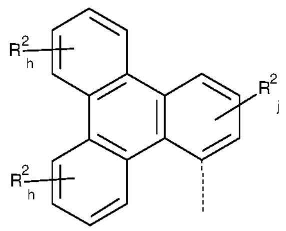

Formel ($R^1$-79)

wobei für die verwendeten Symbole gilt:

Y ist O, S oder $NR^2$, vorzugsweise O oder S;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
$R^2$ kann die zuvor genannte, insbesondere für Formel (I) und/oder (II) genannte Bedeutung aufweisen und
die gestrichelte Bindung markiert die Anbindungsposition und.

Vorzugsweise kann vorgesehen sein, dass die Summe der Indices i, j, h und g in den Strukturen der Formel ($R^1$-1) bis ($R^1$-79) jeweils höchstens 3, vorzugsweise höchstens 2 und besonders bevorzugt höchstens 1 beträgt.

Bevorzugt kann die Gruppe $L^1$ mit der elektronentransportierenden Gruppe Q und der Fluorenstruktur der Formel (III), (IV), (lila), (IVa), (IIIb) und/oder (IVb) eine durchgängige Konjugation ausbilden. Eine durchgängie Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen der elektronentransportierenden Gruppe Q und der Fluorenstruktur liegt. Im Gegensatz hierzu kann bei einer Spirobifluorenstruktur eine durchgängie Konjugation ausgebildet werden, falls die Verbindung zwischen der elektronentransportierenden Gruppe Q und der Fluorenstruktur der Formel (III), (IV), (lila), (IVa), (IIIb) und/oder (IVb) über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der elektronentransportierenden Gruppe Q und der Fluorenstruktur der Formel (III), (IV), (IIIa), (IVa), (IIIb) und/oder (IVb) über verschiedene Phenylgruppen der Spirobifluorenstruktur erfolgt, die über das $sp^3$ hybridisierte Kohlenstoffatom in Position 9 verbunden sind, ist die Konjugation unterbrochen.

In einer weiteren bevorzugten Ausführungsform der Erfindung steht $L^1$ gleich oder verschieden bei jedem Auftreten für eine Einfachbindung oder ein aromatisches oder heteroaromatisches Ringsystem mit 5 bis 24 aromatischen Ringatomen, das durch einen oder mehrere Reste $R^2$ substituiert sein kann. Besonders bevorzugt steht $L^1$ gleich oder verschieden bei jedem Auftreten für eine Einfachbindung oder ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste $R^2$ substituiert sein kann, bevorzugt aber unsubstituiert ist, wobei $R^2$ die zuvor genannte Bedeutung aufweisen kann. Weiterhin bevorzugt steht das Symbol $L^1$ gleich oder verschieden bei jedem Auftreten für eine Einfachbindung oder einen Aryl- oder Heteroarylrest, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist. Ganz besonders bevorzugt steht $L^1$ für eine Einfachbindung. Beispiele für geeignete aromatische oder heteroaromatische Ringsysteme $L^1$ sind ausgewählt aus der Gruppe bestehend aus ortho-, meta- oder para-Phenylen, Biphenyl, Fluoren, Pyridin, Pyrimidin, Triazin, Dibenzofuran und Dibenzothiophen, die jeweils durch einen oder mehrere Reste $R^2$ substituiert sein können, bevorzugt aber unsubstituiert sind.

Bevorzugt sind Verbindungen umfassend Strukturen der Formeln (III), (IV), (IIIa), (IVa), (IIIb) und/oder (IVb), in denen die Gruppe $L^1$ gemäß Formel (III), (IV), (IIIa), (IVa), (IIIb) und/oder (IVb) für eine Bindung oder eine Gruppe steht, die ausgewählt ist aus den Formeln (L-1) bis (L-70)

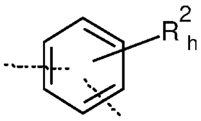

Formel (L-1)

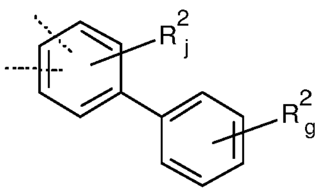

Formel (L-2)

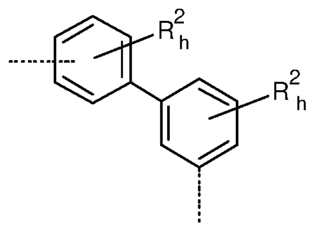

Formel (L-3)

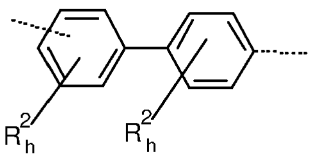

Formel (L-4)

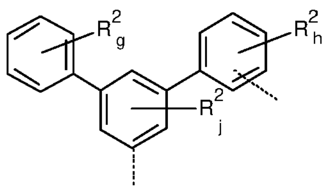

Formel (L-5)

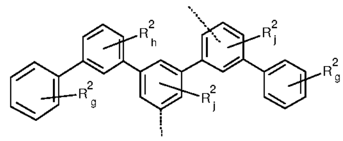

Formel (L-6)

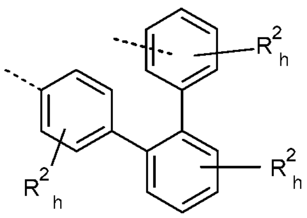

Formel (L-7)

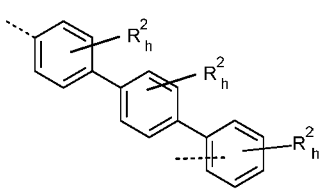

Formel (L-8)

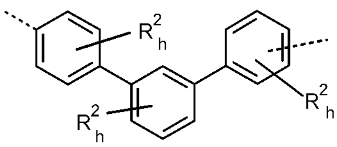

Formel (L-9)

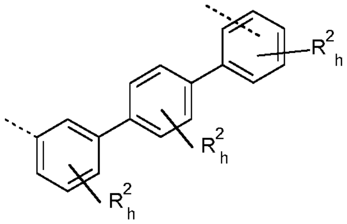

Formel (L-10)

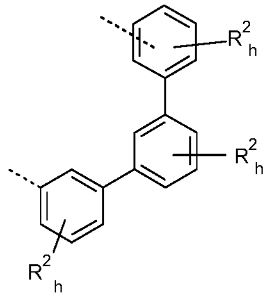

Formel (L-11)

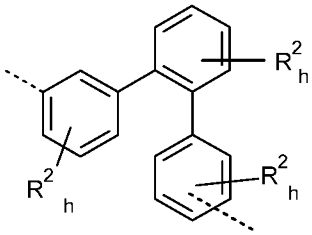

Formel (L-12)

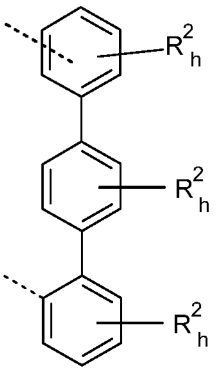

Formel (L-13)

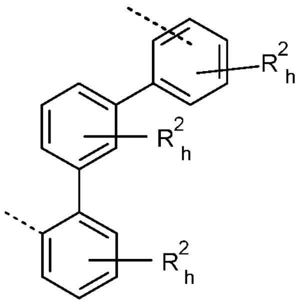

Formel (L-14)

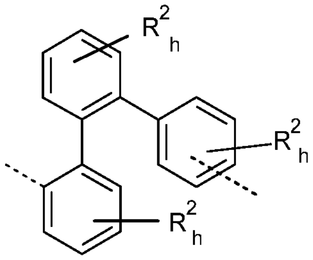

Formel (L-15)

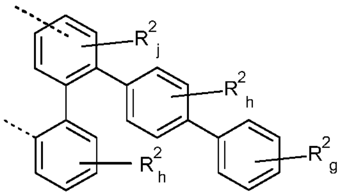

Formel (L-16)

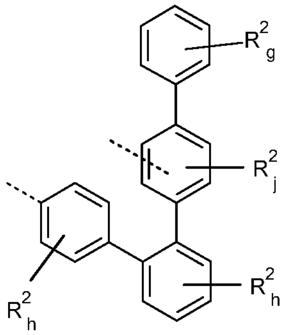

Formel (L-17)

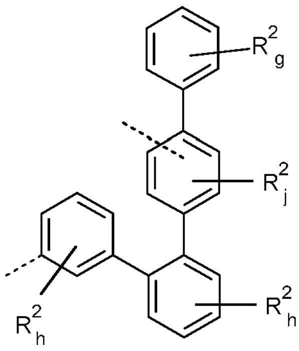

Formel (L-18)

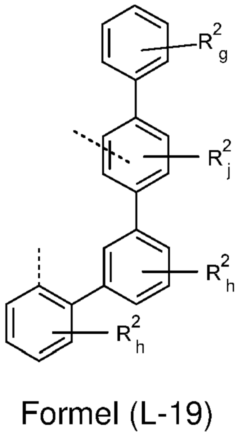

Formel (L-19)

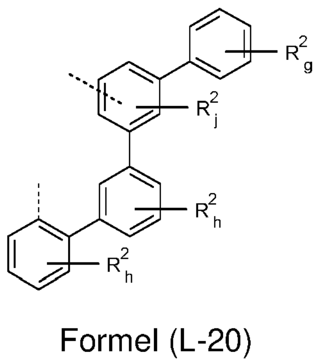

Formel (L-20)

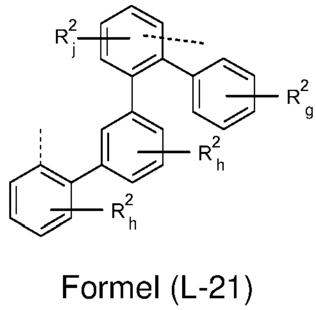

Formel (L-21)

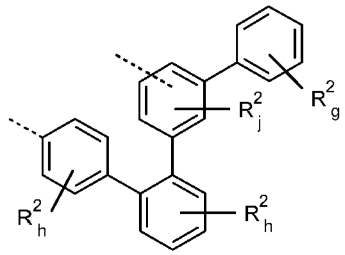

Formel (L-22)

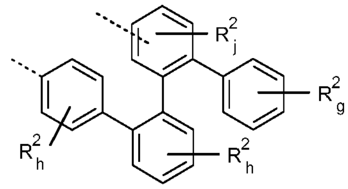

Formel (L-23)

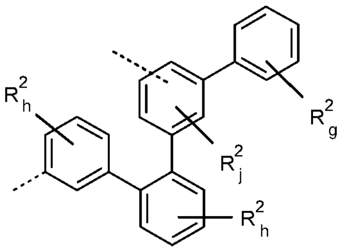

Formel (L-24)

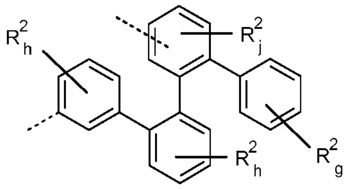

Formel (L-25)

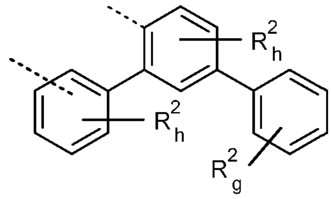

Formel (L-26)

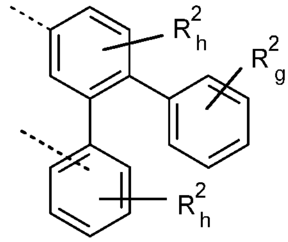

Formel (L-27)

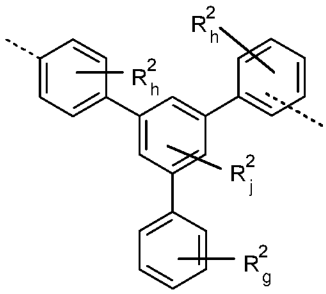

Formel (L-28)

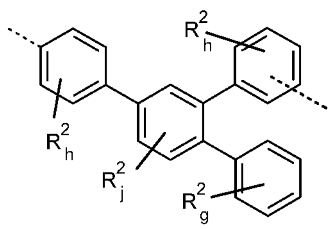

Formel (L-29)

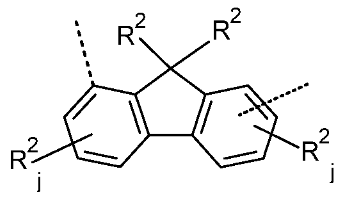

Formel (L-30)

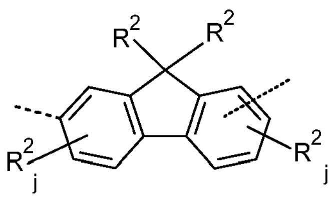

Formel (L-31)

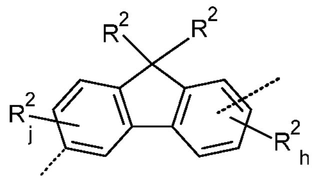

Formel (L-32)

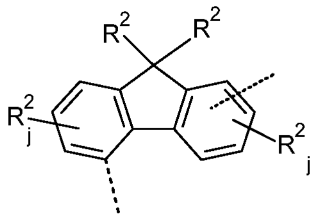

Formel (L-33)

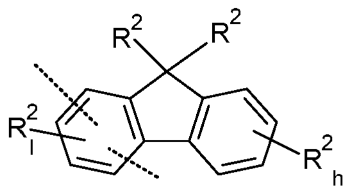

Formel (L-34)

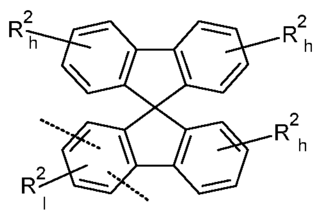

Formel (L-35)

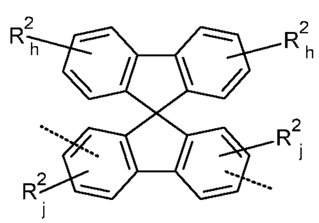

Formel (L-36)

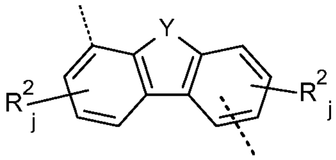

Formel (L-37)

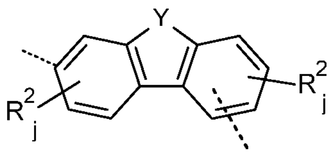

Formel (L-38)

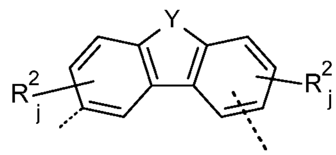

Formel (L-39)

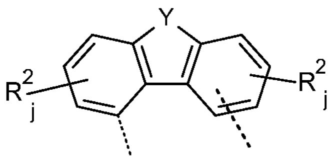

Formel (L-40)

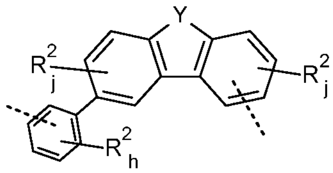

Formel (L-41)

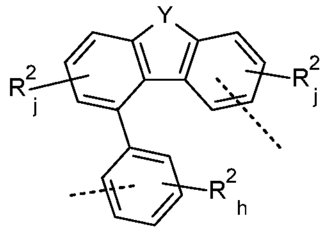

Formel (L-42)

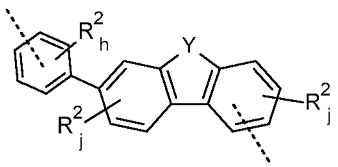

Formel (L-43)

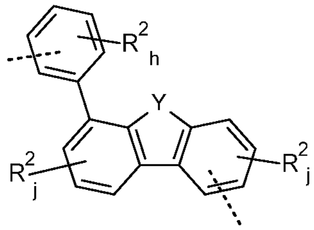

Formel (L-44)

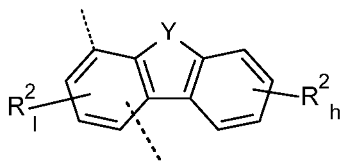

Formel (L-45)

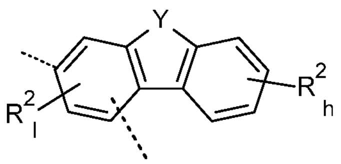

Formel (L-46)

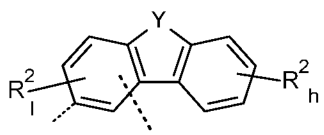

Formel (L-47)

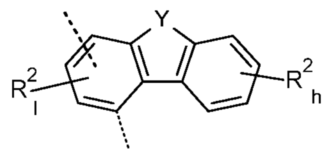

Formel (L-48)

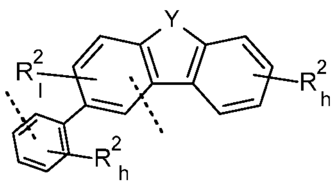

Formel (L-49)

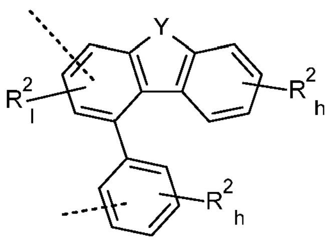

Formel (L-50)

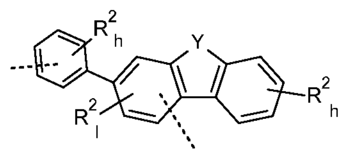

Formel (L-51)

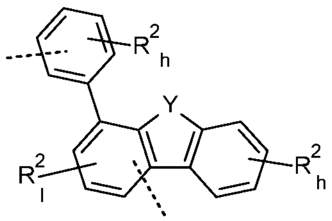

Formel (L-52)

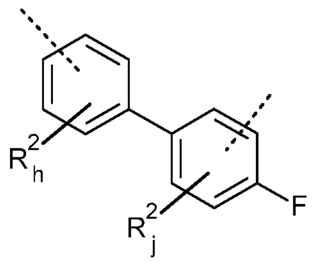

Formel (L-53)

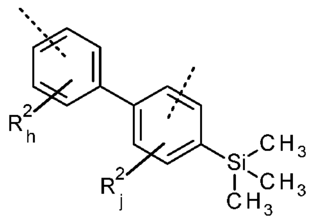

Formel (L-54)

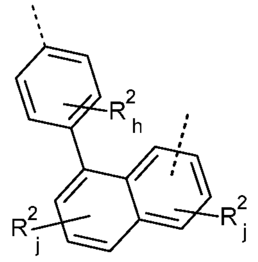

Formel (L-55)

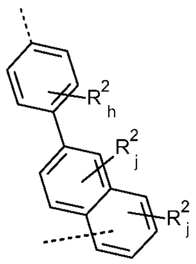

Formel (L-56)

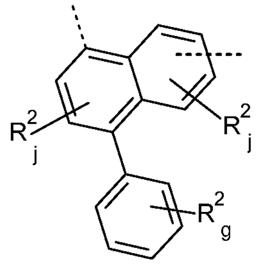

Formel (L-57)

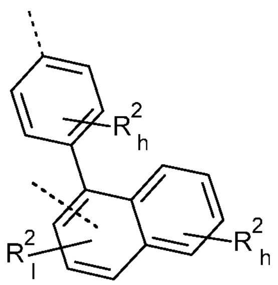

Formel (L-58)

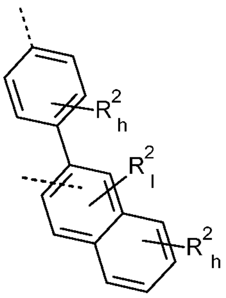

Formel (L-59)

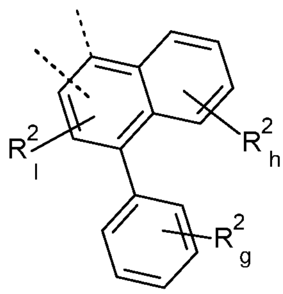

Formel (L-60)

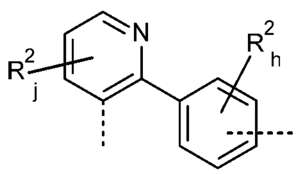

Formel (L-61)

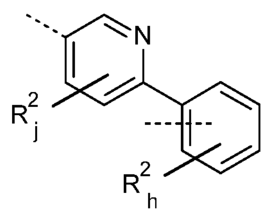

Formel (L-62)

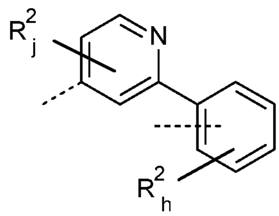

Formel (L-63)

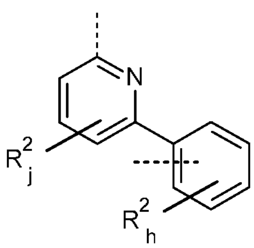

Formel (L-64)

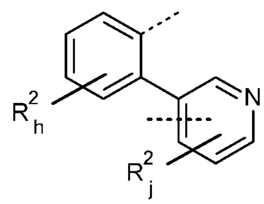

Formel (L-65)

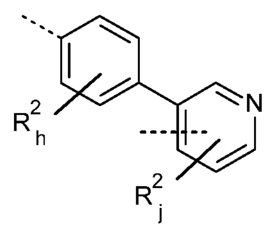

Formel (L-66)

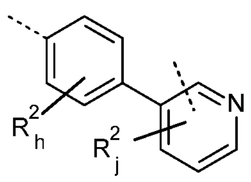

Formel (L-67)

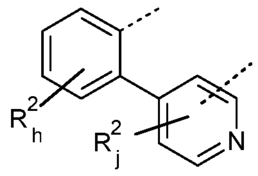

Formel (L-68)

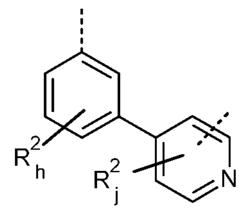

Formel (L-69)

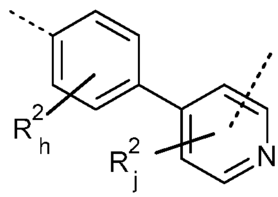

Formel (L-70)

wobei die gestrichelten Bindungen jeweils die Anbindungspositionen markieren, der Index l 0, 1 oder 2 ist, der Index g 0, 1, 2, 3, 4 oder 5 ist, j bei jedem Auftreten unabhängig 0, 1, 2 oder 3 ist; h bei jedem Auftreten unabhängig 0, 1, 2, 3 oder 4 ist; Y O, S oder $NR^2$, vorzugsweise O oder S ist; und $R^2$ die zuvor genannte Bedeutung aufweist.

Vorzugsweise kann vorgesehen sein, dass die Summe der Indices l, g, h und j in den Strukturen der Formel (L-1) bis (L-70) jeweils höchstens 3, vorzugsweise höchstens 2 und besonders bevorzugt höchstens 1 beträgt.

In einer weiteren Ausführungsform kann vorgesehen sein, dass eine erfindungsgemäße Verbindung, umfassend mindestens eine Struktur gemäß Formel (III), (IV), (Iiia), (IVa), (IIIb) und/oder (IVb) zwei oder mehr Elektronentransportgruppen umfasst.

In einer bevorzugten Ausgestaltung können die erfindungsgemäßen Verbindungen eine Struktur gemäß Formel (VII) und/oder (VIII) bilden

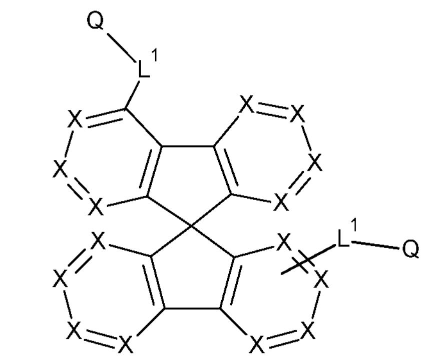

Formel (VII)

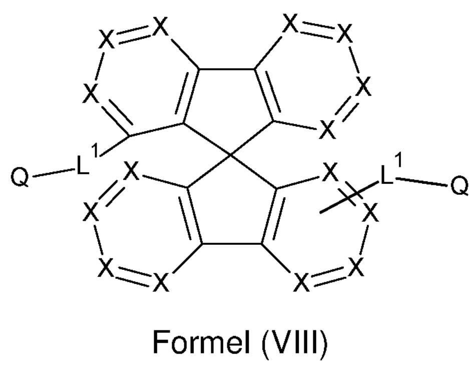

Formel (VIII)

wobei die Symbole X, $L^1$ und Q bei jedem Auftreten jeweils gleich oder verschieden die zuvor, insbesondere für Formel (I) genannte Bedeutung haben und X für die Anbindungsstelle der Gruppe ($-L^1-Q$) C ist.

Weiterhin sind Verbindungen bevorzugt, umfassend Strukturen der Formeln (VIIa) und (VIIb)

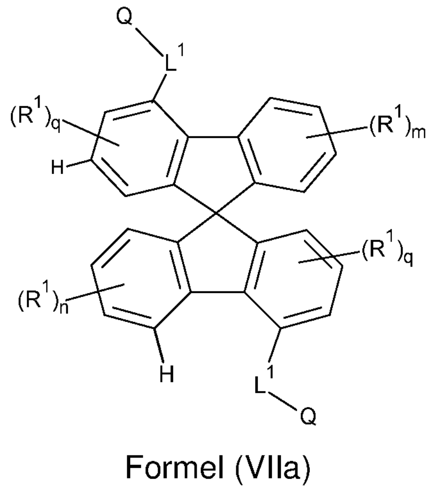

Formel (VIIa)

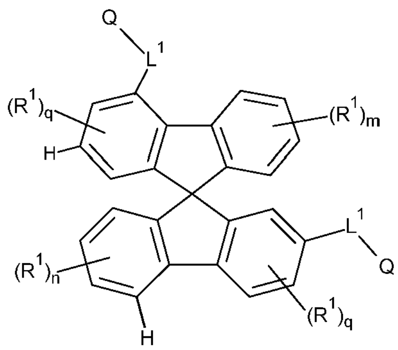

Formel (VIIb)

worin die Symbole Q, $L^1$ und $R^1$ die zuvor dargelegte Bedeutung aufweisen und m 0, 1, 2, 3 oder 4, vorzugsweise 0, 1 oder 2, n 0, 1, 2 oder 3, vorzugsweise 0, 1 oder 2 und q 0, 1 oder 2, vorzugsweise 0 oder 1 ist.

Mit Vorteil kann vorgesehen sein, dass eine erfindungsgemäße Verbindung, umfassend mindestens eine Struktur gemäß Formel (III), (IV), (lila), (IVa), (IIIb) und/oder (IVb) keine Carbazol- und/oder Triarylamin-Gruppe umfasst. Besonders bevorzugt umfasst eine erfindungsgemäße Verbindung keine lochtransportierende Gruppe. Lochtransportierende Gruppen sind in der Fachwelt bekannt, wobei diese Gruppen vielfach Carbazol-, Indenocarbazol-, Indolocarbazol-, Arylamin- oder eine Diarylaminstrukturen.

In einer weiteren Ausgestaltung kann vorgesehen sein, dass eine erfindungsgemäße Verbindung, umfassend mindestens eine Struktur gemäß Formel (III), (IV), (lila), (IVa), (IIIb) und/oder (IVb) mindestens eine lochtransportierende Gruppe umfasst, vorzugsweise eine Carbazol- und/oder Triarylamin-Gruppe. Ferner kann als Lochtransportierende Gruppe auch eine Indenocarbazol-, Indolocarbazol-, Arylamin- oder eine Diarylamin-Gruppe vorgesehen sein.

In einer weiteren bevorzugten Ausführungsform der Erfindung ist $R^2$ in den erfindungsgemäßen Verbindungen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$ in den erfindungsgemäßen Verbindungen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen $R^1$ bzw. $R^2$ bzw. $Ar^1$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Beispiele für geeignete erfindungsgemäße und nicht-erfindungsgemäße Verbindungen sind die nachstehend gezeigten Strukturen gemäß den folgenden Formeln 1 bis 216:

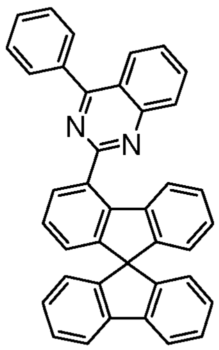

Formel 1

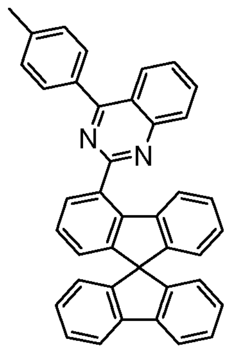

Formel 2

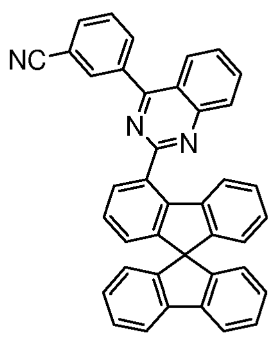

Formel 3

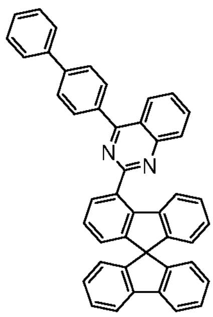

Formel 4

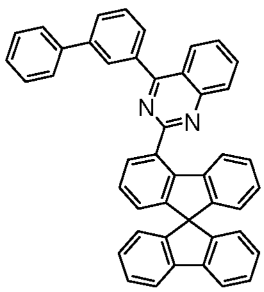

Formel 5

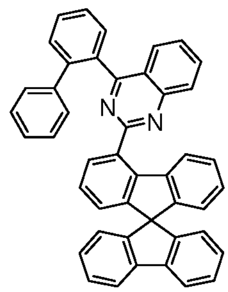

Formel 6

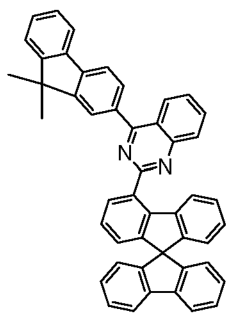

Formel 7

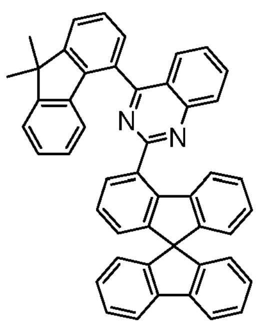

Formel 8

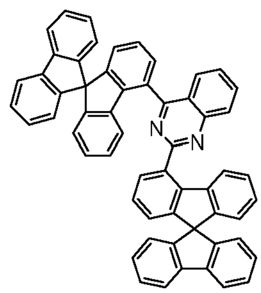

Formel 9

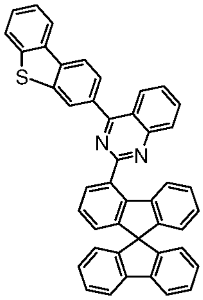

Formel 10

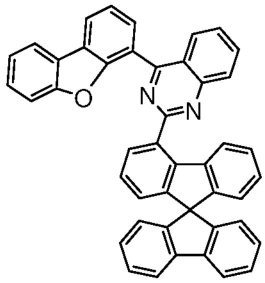

Formel 11

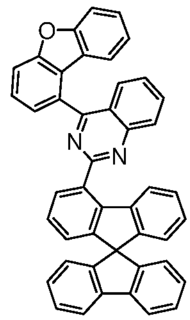

Formel 12

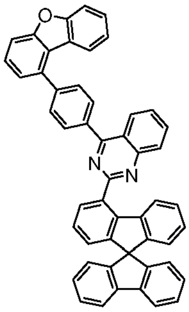

Formel 13

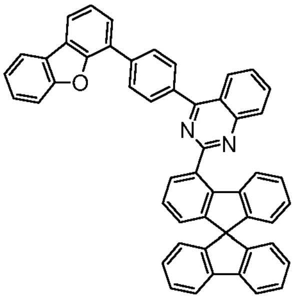

Formel 14

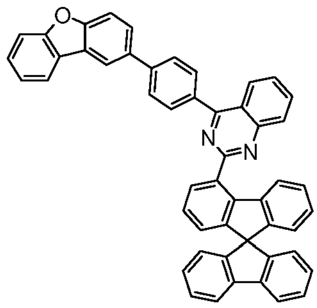

Formel 15

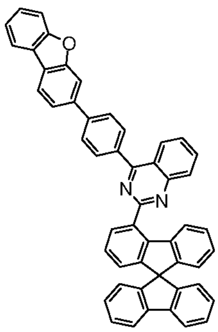

Formel 16

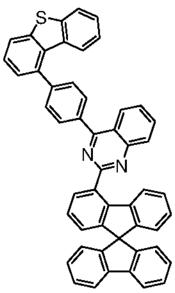

Formel 17

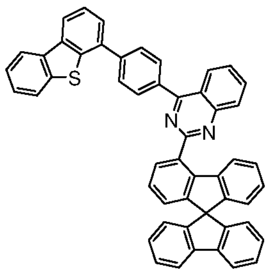

Formel 18

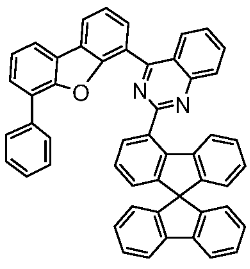

Formel 19

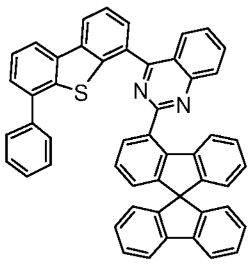

Formel 20

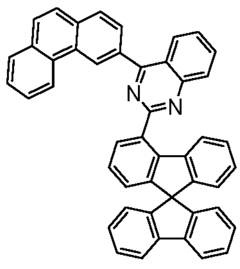

Formel 21

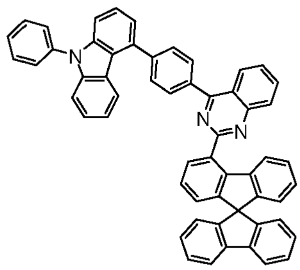

Formel 22

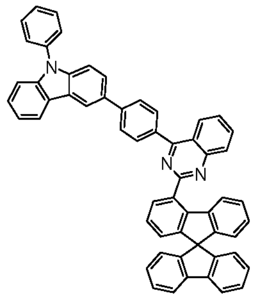

Formel 23

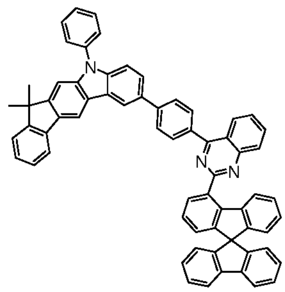

Formel 24

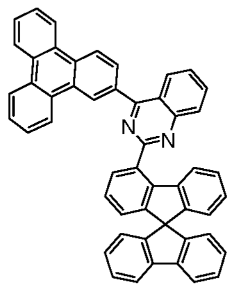

Formel 25

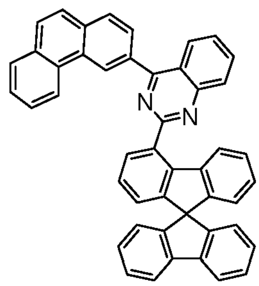

Formel 26

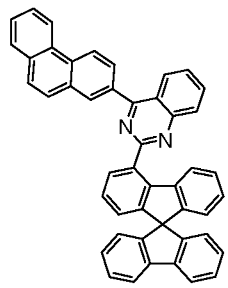

Formel 27

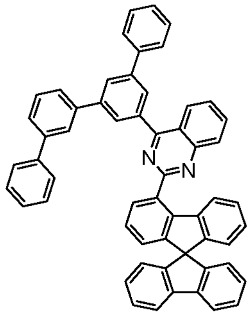

Formel 28

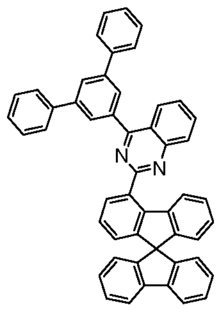

Formel 29

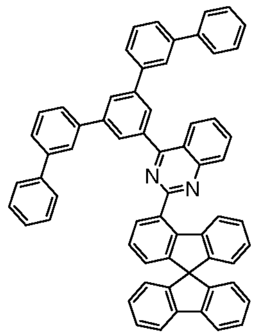

Formel 30

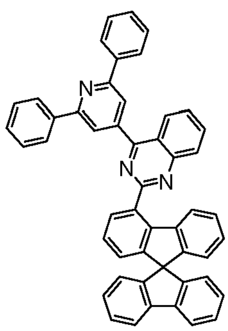

Formel 31

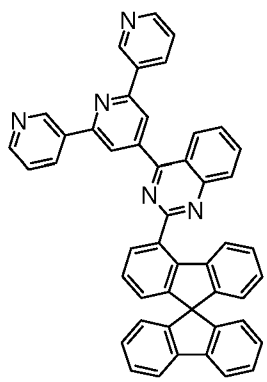

Formel 32

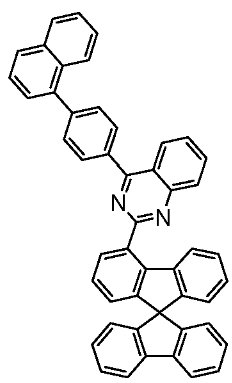

Formel 33

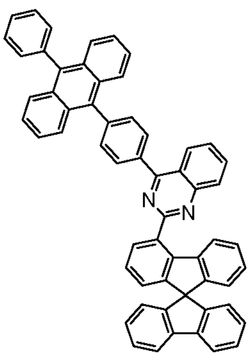

Formel 34

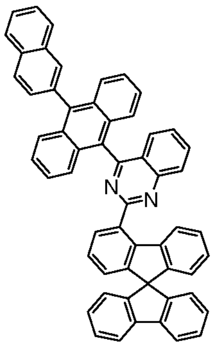

Formel 35

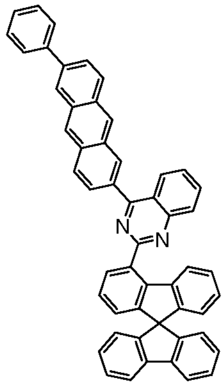

Formel 36

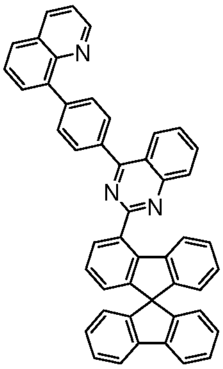

Formel 37

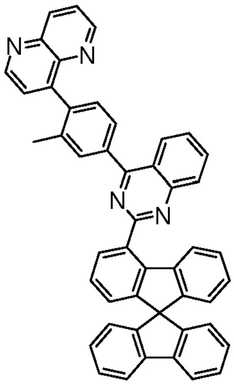

Formel 38

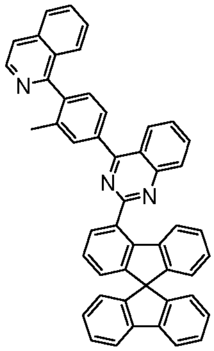

Formel 39

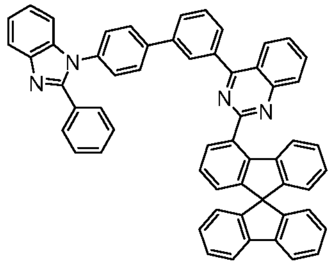

Formel 40

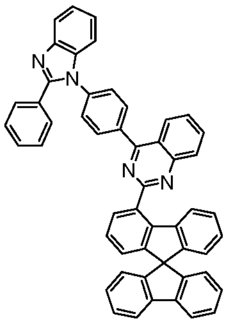

Formel 41

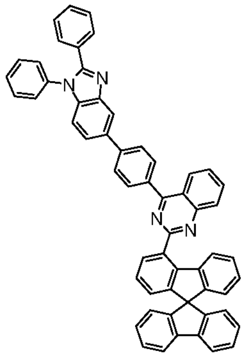

Formel 42

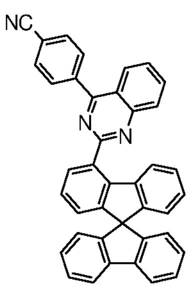

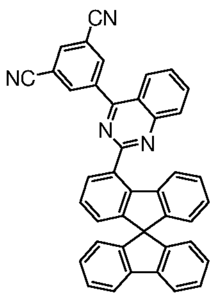

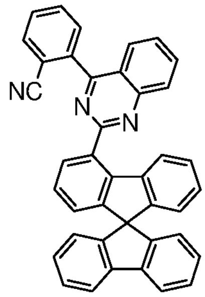

Formel 43 Formel 44 Formel 45

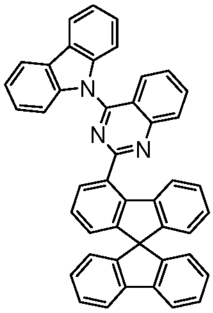 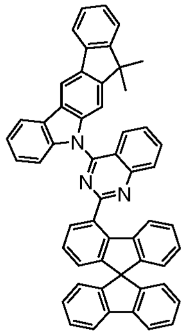 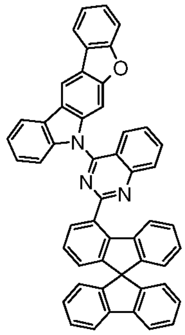

Formel 46 Formel 47 Formel 48

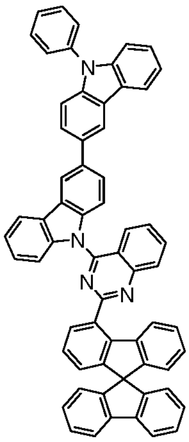 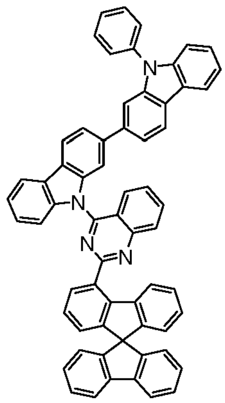 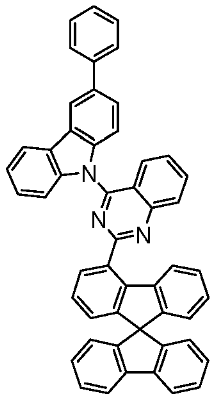

Formel 49 Formel 50 Formel 51

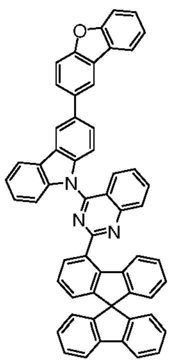 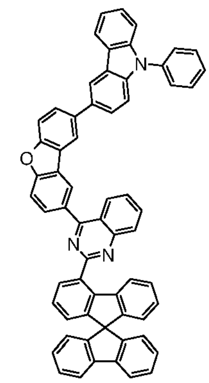 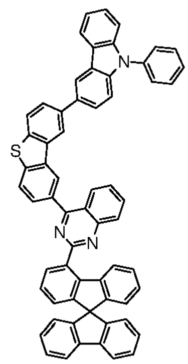

Formel 52 Formel 53 Formel 54

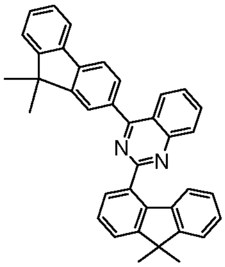

Formel 55

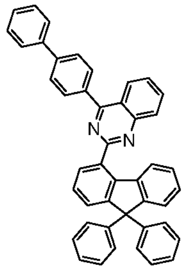

Formel 56

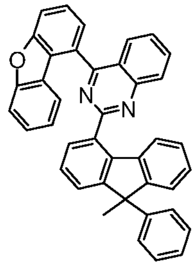

Formel 57

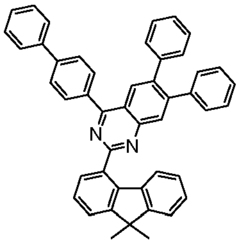

Formel 58

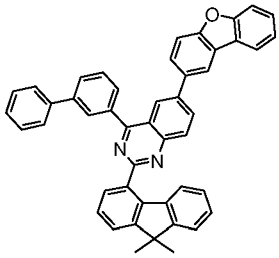

Formel 59

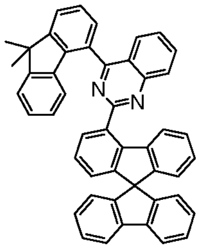

Formel 60

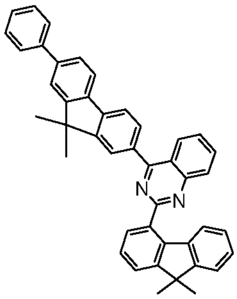

Formel 61

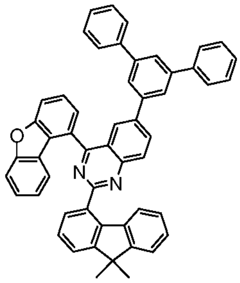

Formel 62

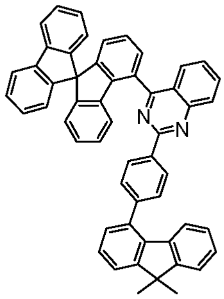

Formel 63

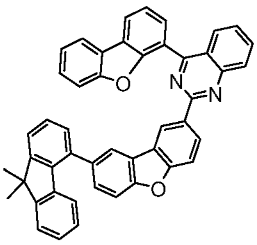

Formel 64

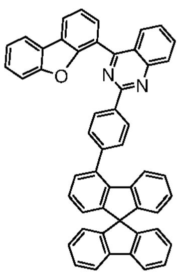

Formel 65

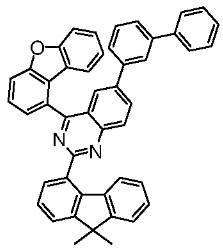

Formel 66

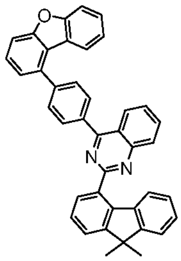

Formel 67

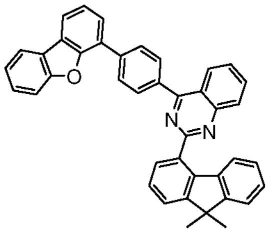

Formel 68

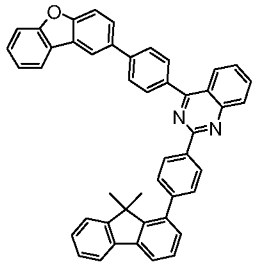

Formel 69

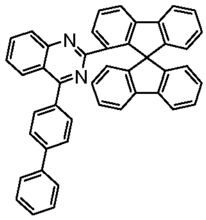

Formel 70

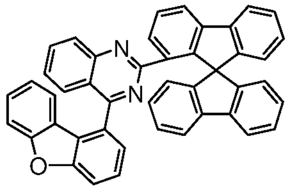

Formel 71

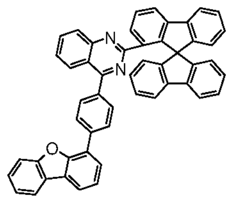

Formel 72

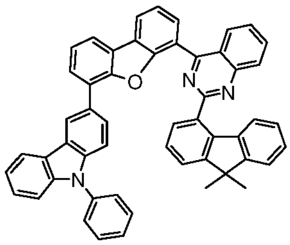

Formel 73

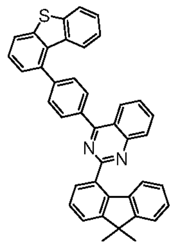

Formel 74

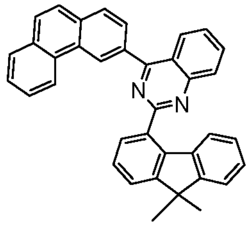

Formel 75

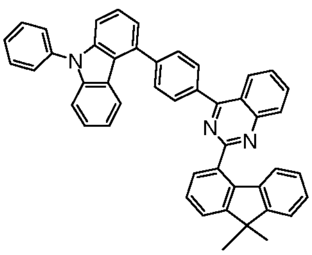

Formel 76

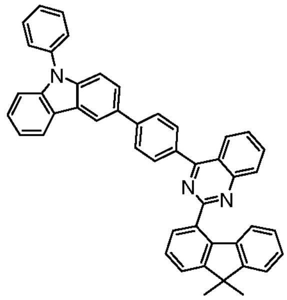

Formel 77

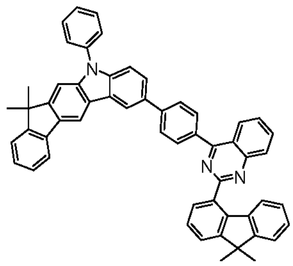

Formel 78

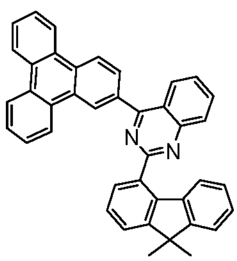

Formel 79

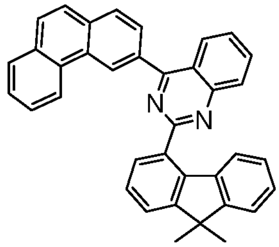

Formel 80

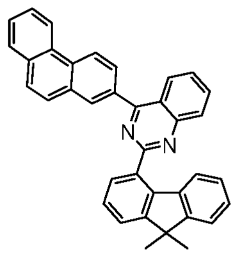

Formel 81

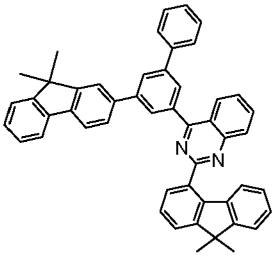
Formel 82

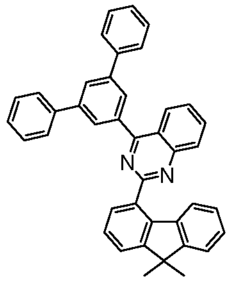
Formel 83

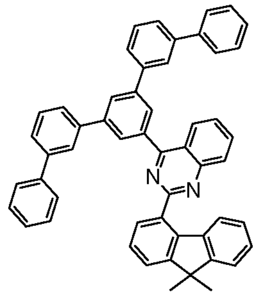
Formel 84

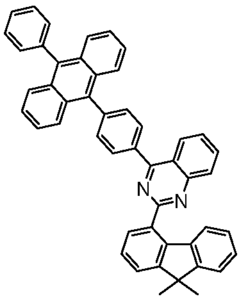
Formel 85

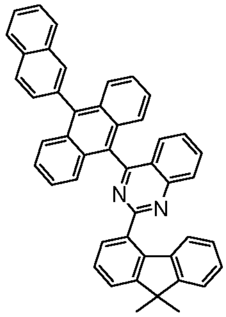
Formel 86

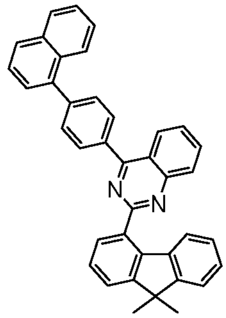
Formel 87

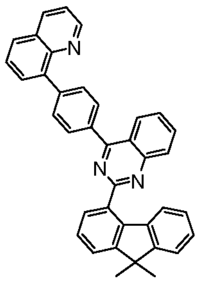
Formel 88

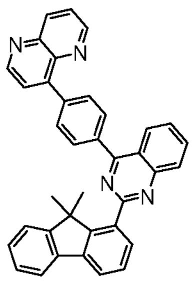
Formel 89

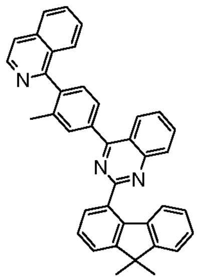
Formel 90

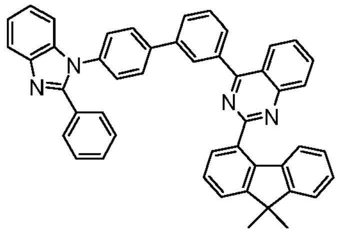
Formel 91

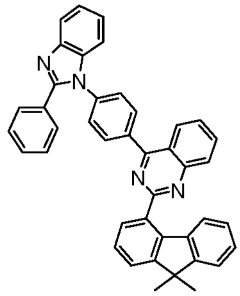
Formel 92

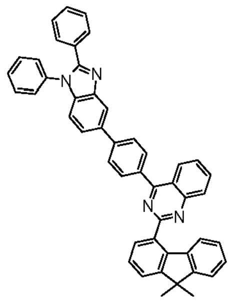
Formel 93

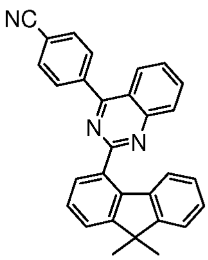

Formel 94

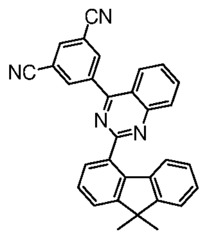

Formel 95

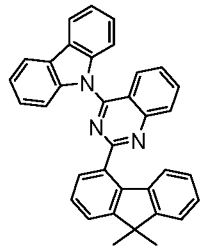

Formel 96

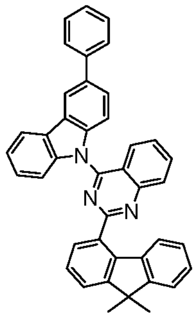

Formel 97

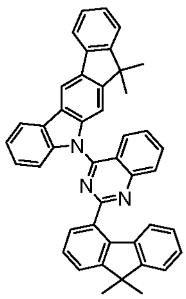

Formel 98

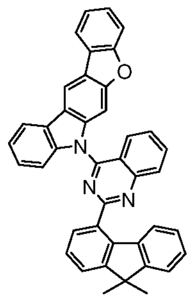

Formel 99

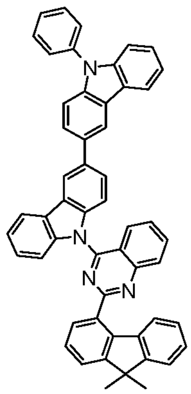

Formel 100

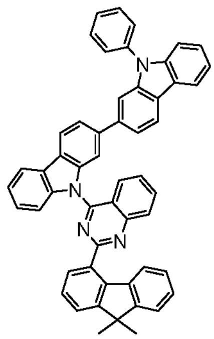

Formel 101

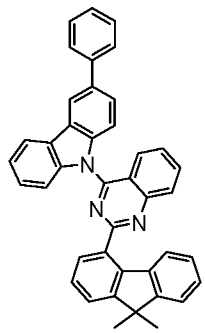

Formel 102

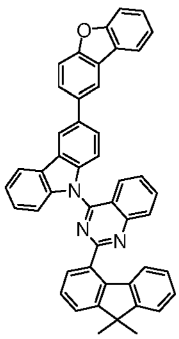

Formel 103

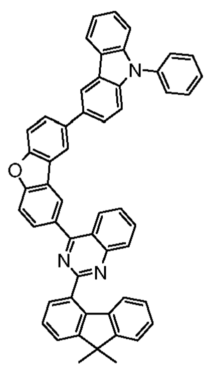

Formel 104

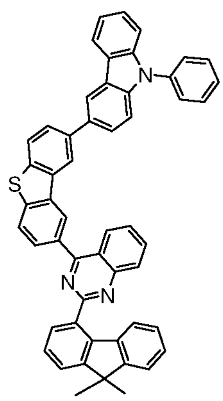

Formel 105

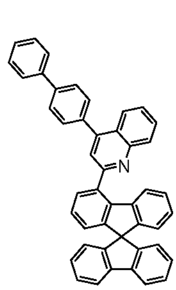

Formel 106

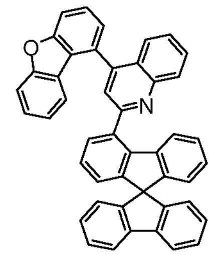

Formel 107

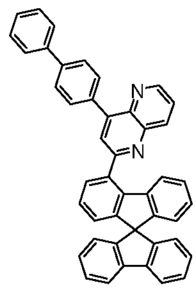

Formel 108

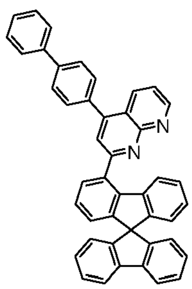

Formel 109

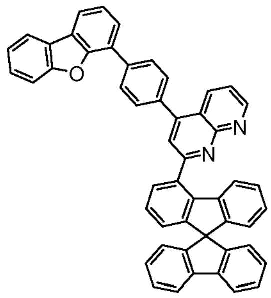

Formel 110

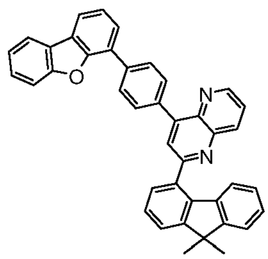

Formel 111

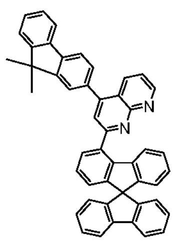

Formel 112

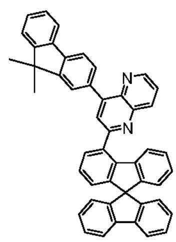

Formel 113

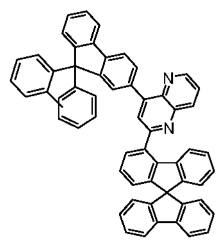

Formel 114

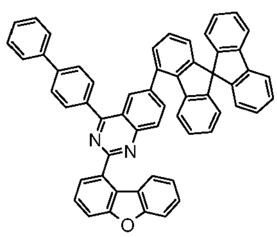

Formel 115

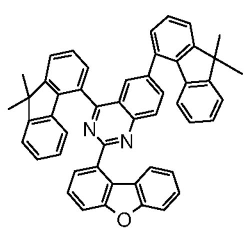

Formel 116

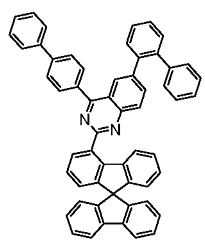

Formel 117

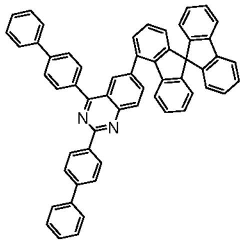

Formel 118

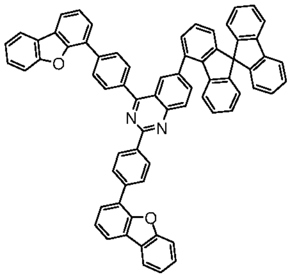

Formel 119

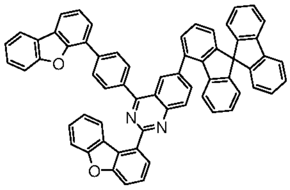

Formel 120

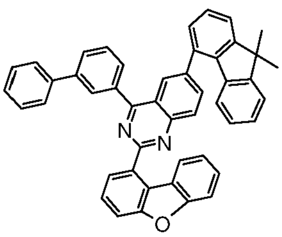

Formel 121

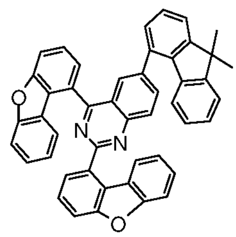

Formel 122

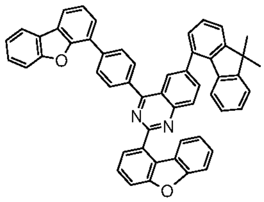

Formel 123

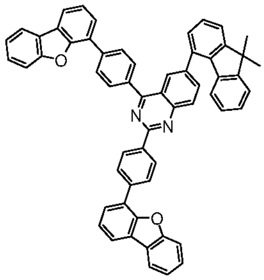

Formel 124

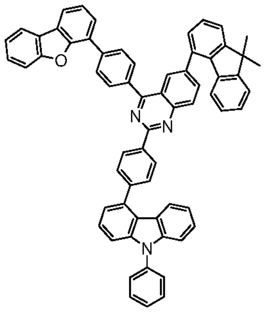

Formel 125

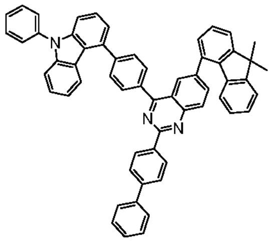

Formel 126

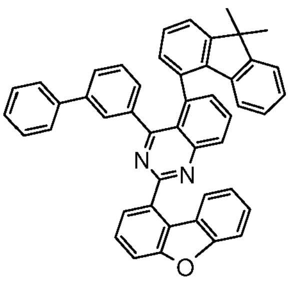

Formel 127

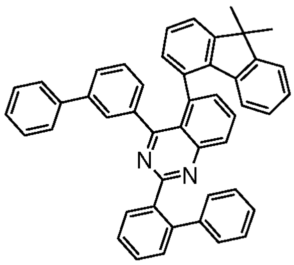

Formel 128

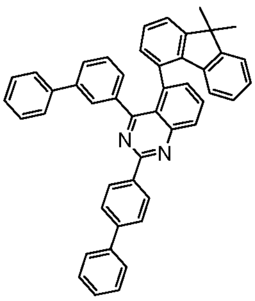

Formel 129

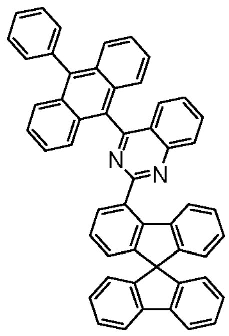

Formel 130

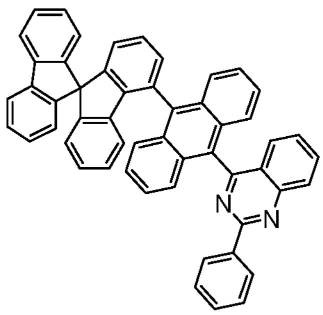

Formel 131

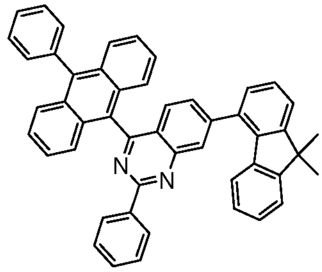

Formel 132

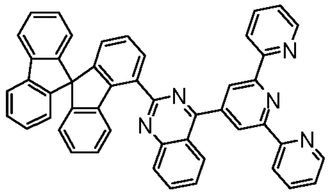

Formel 133

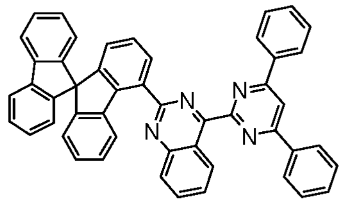

Formel 134

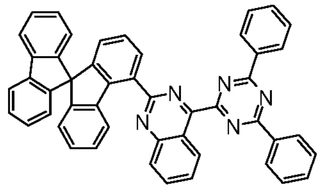

Formel 135

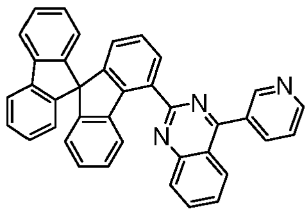

Formel 136

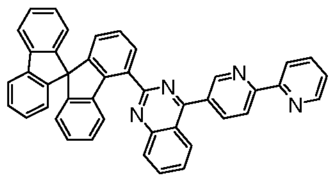

Formel 137

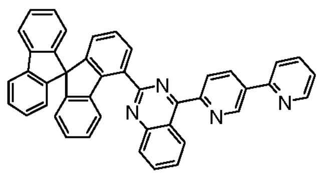

Formel 138

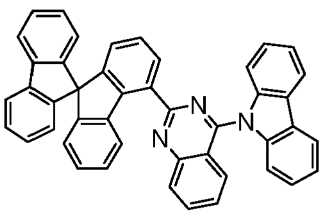

Formel 139

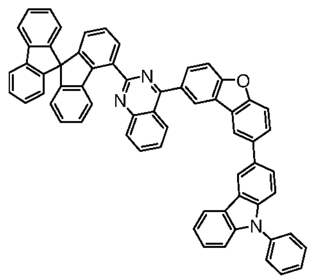

Formel 140

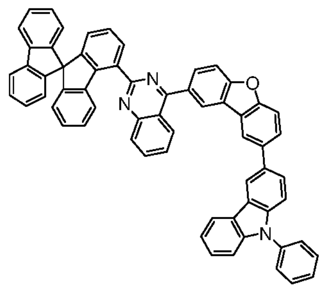

Formel 141

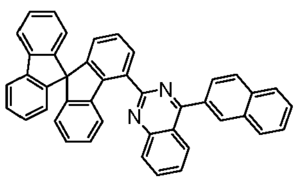

Formel 142

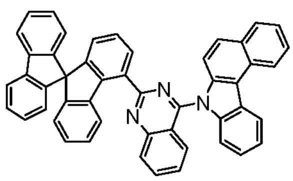

Formel 143

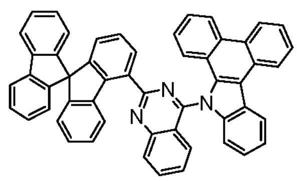

Formel 144

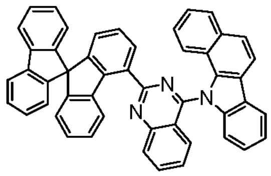

Formel 145

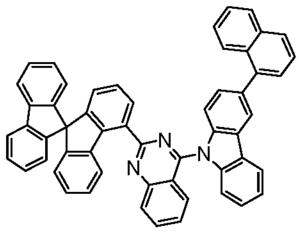

Formel 146

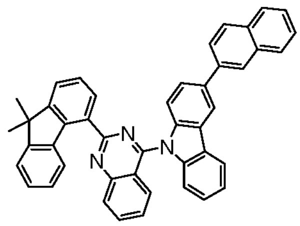

Formel 147

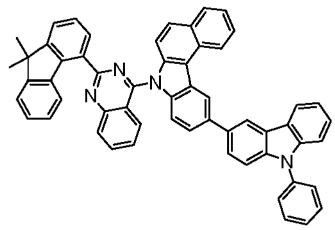

Formel 148

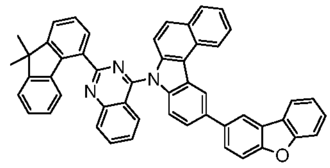

Formel 149

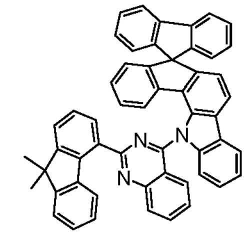

Formel 150

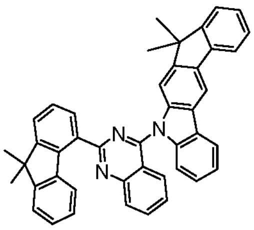

Formel 151

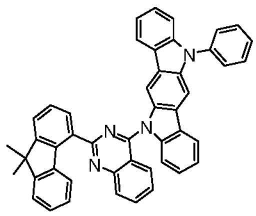

Formel 152

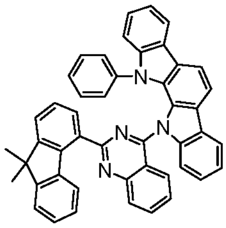

Formel 153

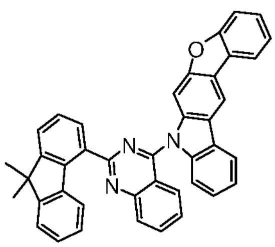

Formel 154

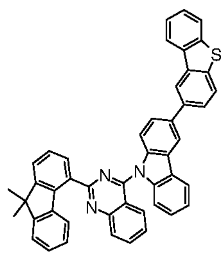

Formel 155

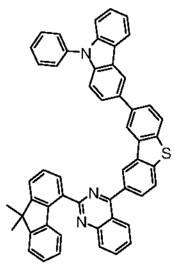

Formel 156

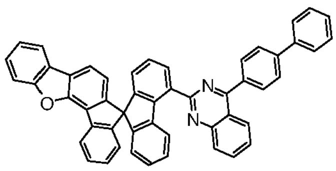

Formel 157

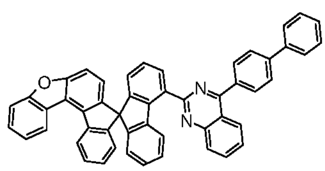

Formel 158

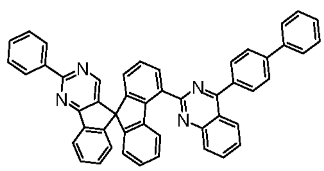

Formel 159

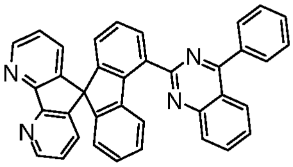

Formel 160

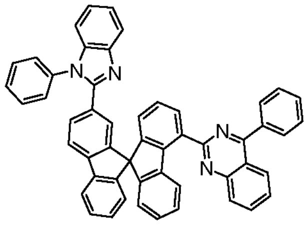

Formel 161

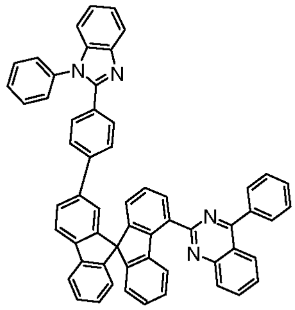

Formel 162

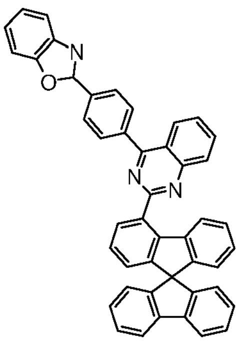

Formel 163

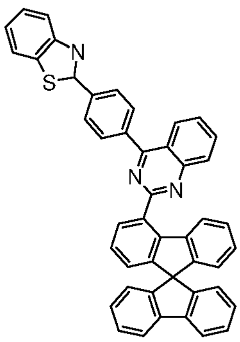

Formel 164

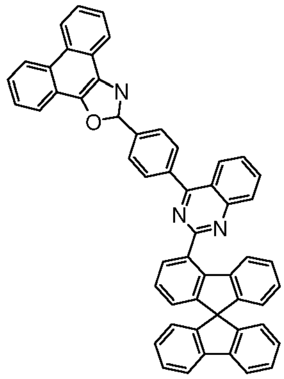

Formel 165

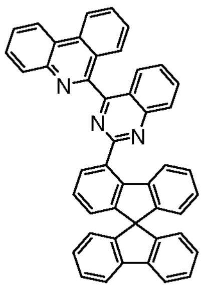

Formel 166

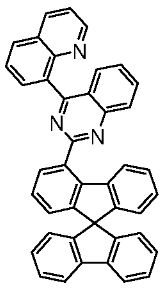

Formel 167

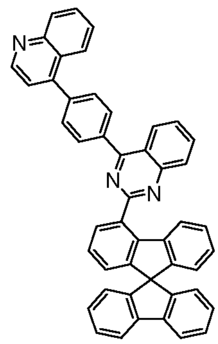

Formel 168

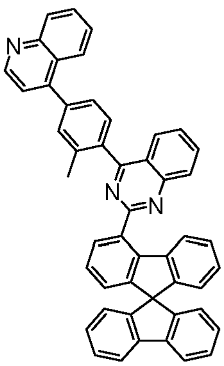

Formel 169

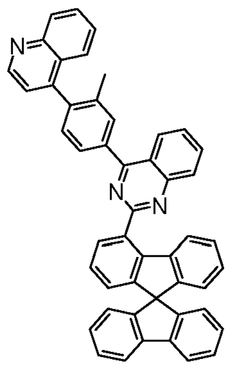

Formel 170

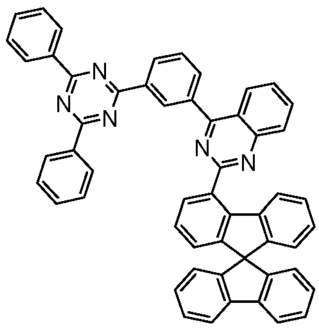

Formel 171

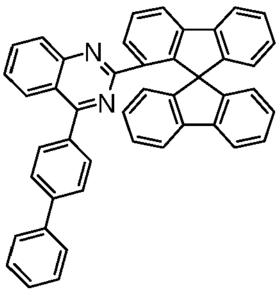

Formel 172

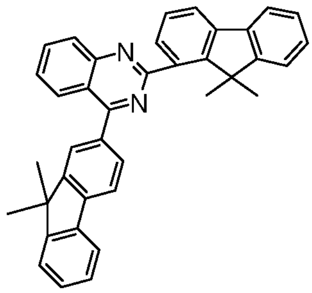

Formel 173

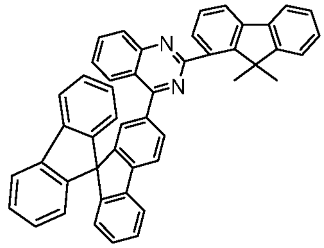

Formel 174

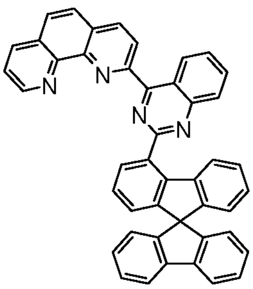

Formel 175

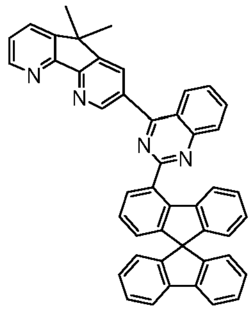

Formel 176

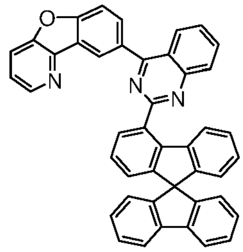

Formel 177

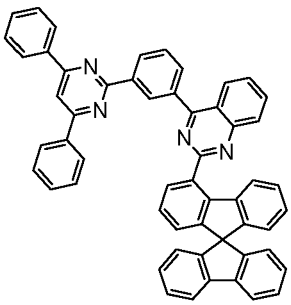

Formel 178

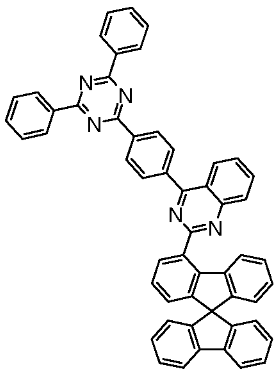

Formel 179

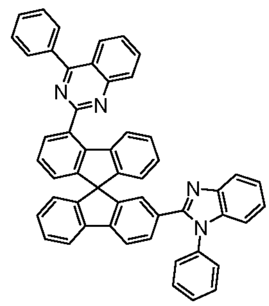

Formel 180

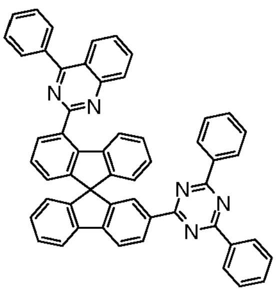

Formel 181

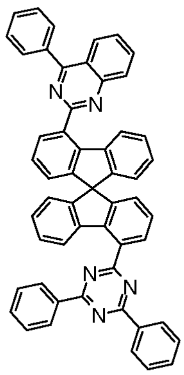

Formel 182

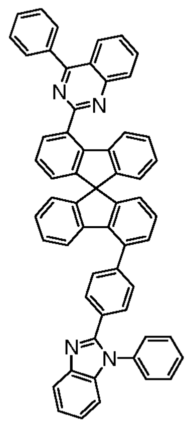

Formel 183

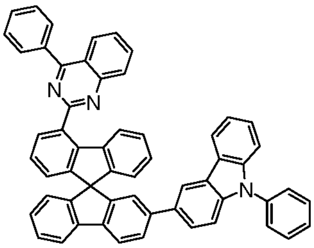

Formel 184

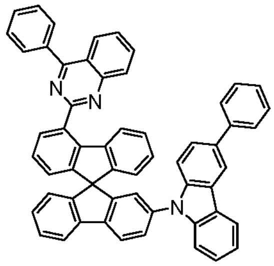

Formel 185

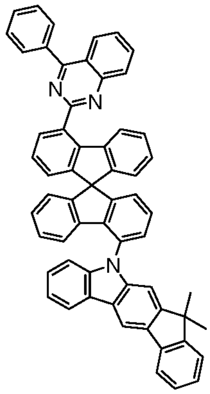

Formel 186

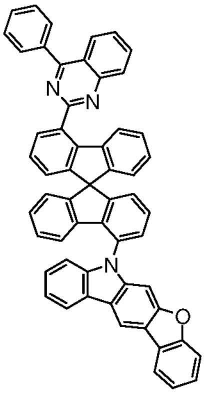

Formel 187

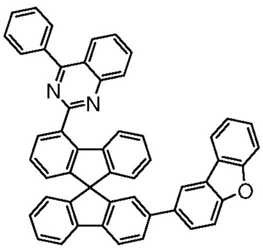

Formel 188

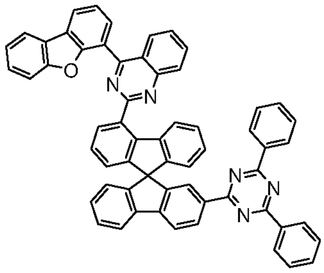

Formel 189

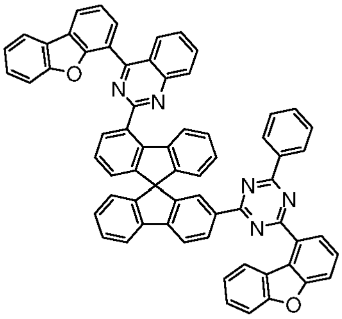

Formel 190

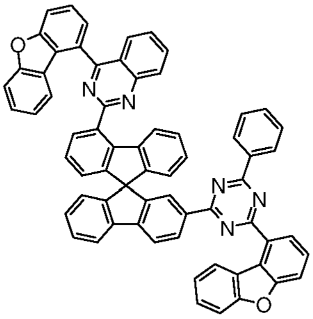

Formel 191

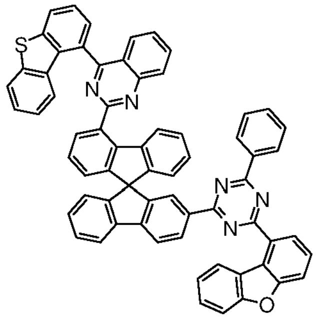

Formel 192

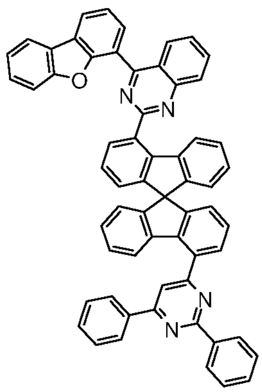

Formel 193

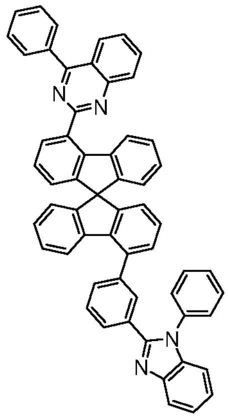

Formel 194

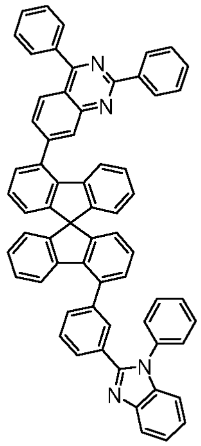

Formel 195

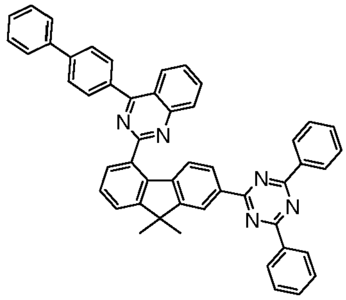

Formel 196

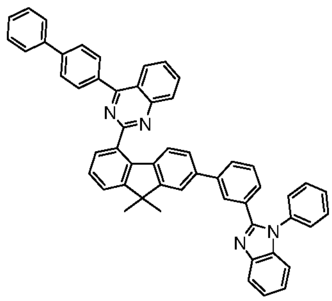

Formel 197

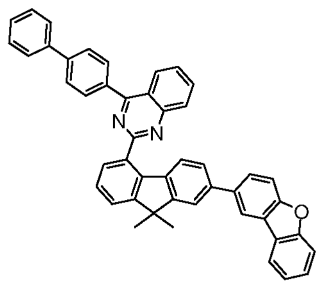

Formel 198

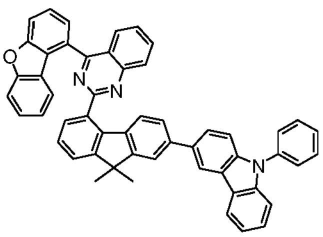

Formel 199

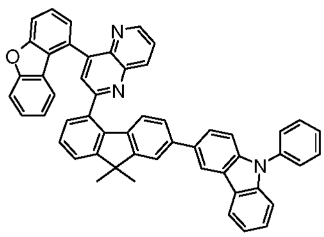

Formel 200

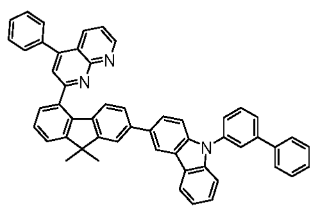

Formel 201

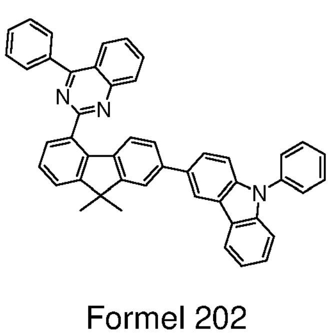

Formel 202

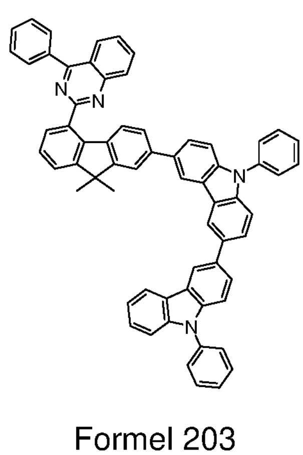

Formel 203

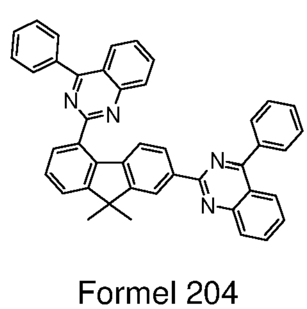

Formel 204

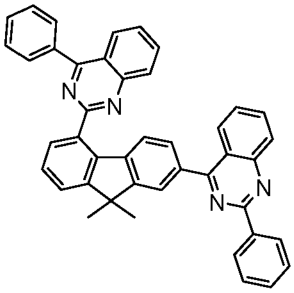

Formel 205

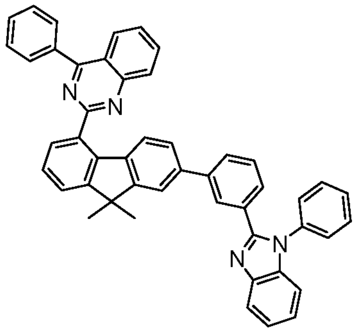

Formel 206

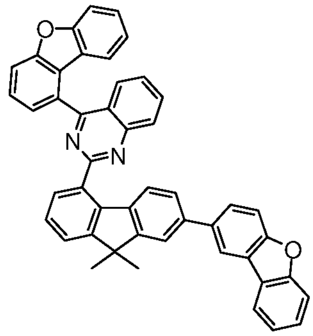

Formel 207

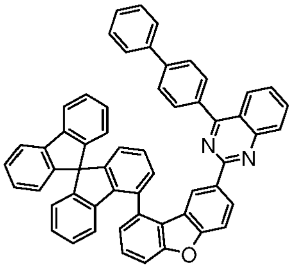

Formel 208

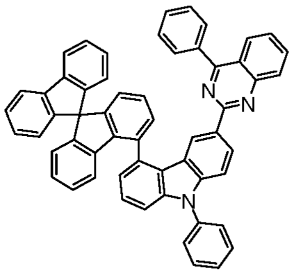

Formel 209

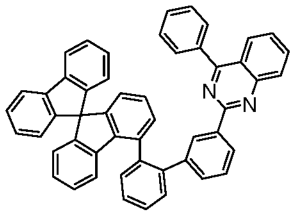

Formel 210

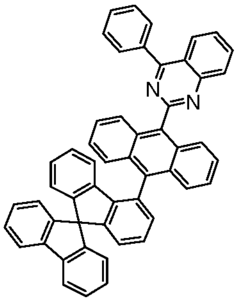

Formel 211

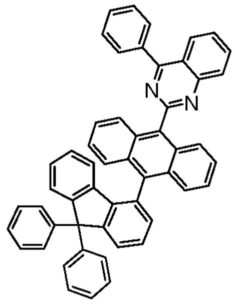

Formel 212

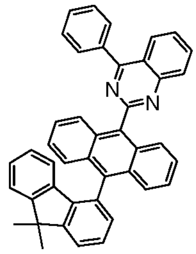

Formel 213

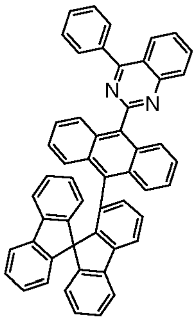

Formel 214

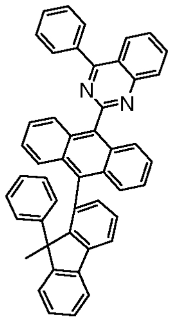

Formel 215

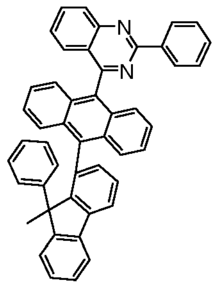

Formel 216

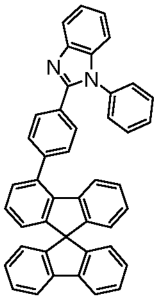
Formel 217

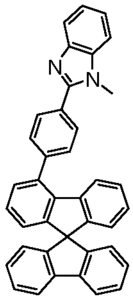
Formel 218

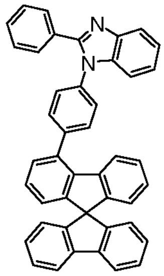
Formel 219

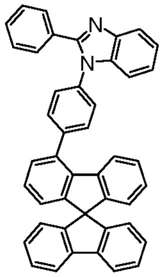
Formel 220

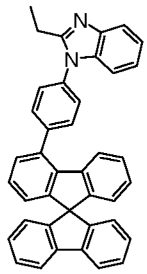
Formel 221

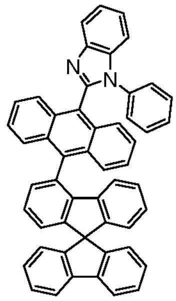
Formel 222

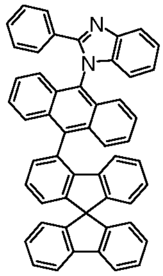
Formel 223

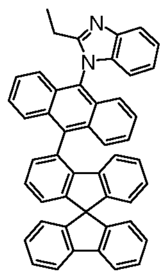
Formel 224

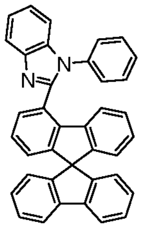
Formel 225

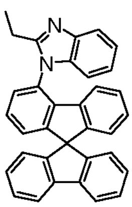
Formel 226

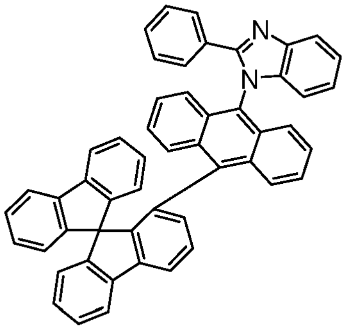
Formel 14

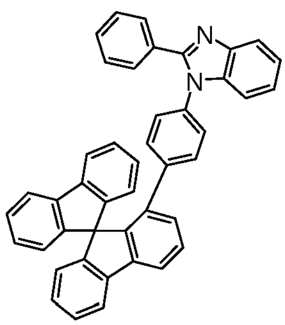
Formel 15

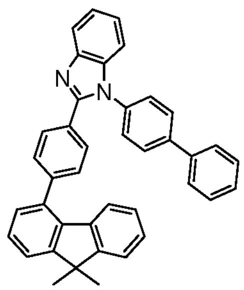

Formel 217

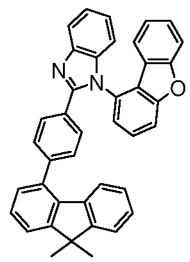

Formel 218

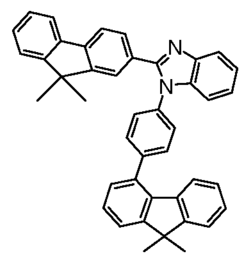

Formel 219

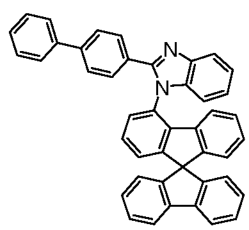

Formel 220

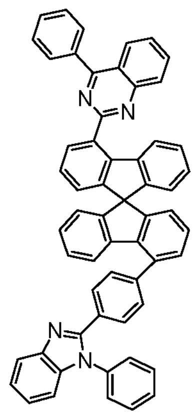

Formel 221

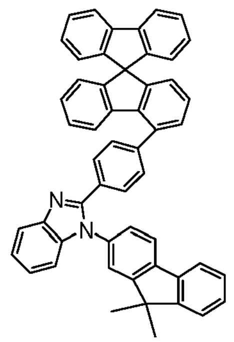

Formel 222

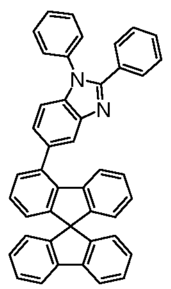

Formel 220

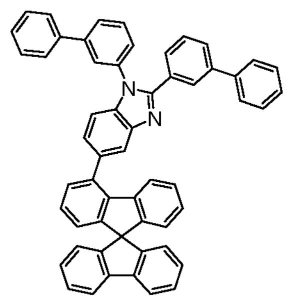

Formel 221

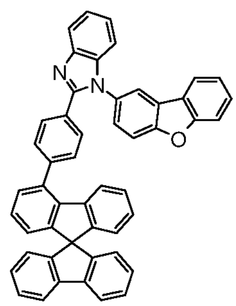

Formel 222

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem in einer Kupplungsreaktion eine Verbindung, umfassend mindestens eine elektronentransportierende Gruppe, mit einer Verbindung, umfassend mindestens einen Fluoren-Rest, umgesetzt wird.

Geeignete Verbindungen mit einer elektronentransportierenden Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt

werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemta, ohne dass hierdurch eine Beschränkung erfolgen soll. Die Teilschritte der einzelnen Schemata können hierbei beliebig kombiniert werden.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Schema 1

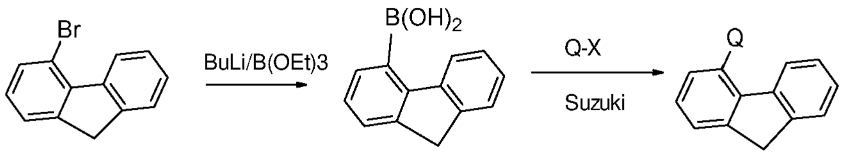


Die Gruppe Q stellt eine Elektronentransportgruppe dar, X eine Abgangsgruppe, beispielsweise Halogen.

Das folgende Schema 2 erläutert die in Schema 1 dargelegte Umsetzung für mehrere verschiedene Elektronentransportgruppen.

Schema 2

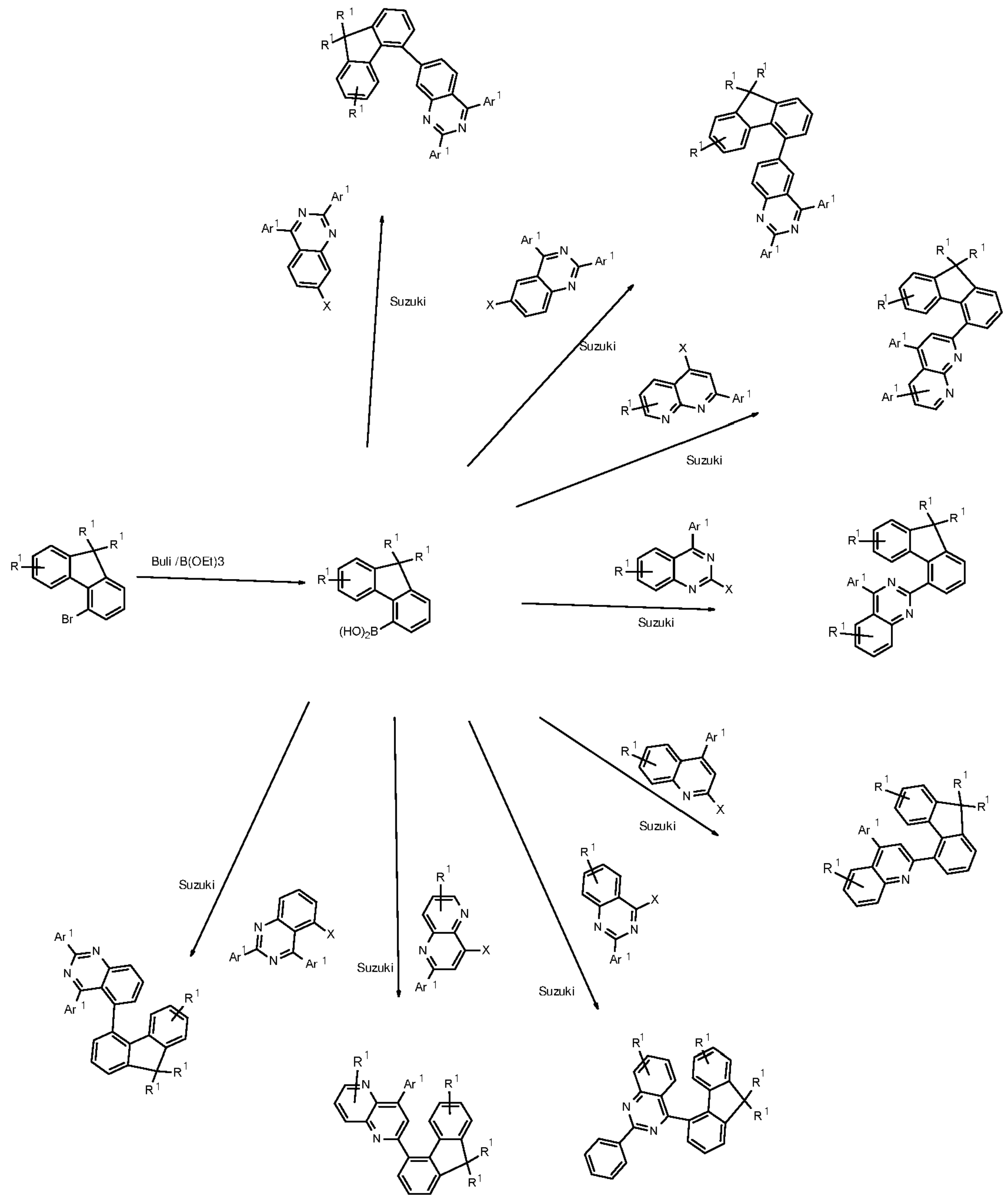


Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) und/oder Formel (II) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei ein oder mehrere Bindungen der erfindungsgemäßen Verbindungen zum Polymer, Oligomer oder Dendrimer vorhanden sind. Je nach Verknüpfung der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß den erfindungsgemäßen Verbindungenbzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, die eine Glasübergangstemperatur von mindestens 70°C, besond ers bevorzugt von mindestens 110°C, ganz besonders bevorzugt von mind estens 125°C und insbesondere bevorzugt von mindestens 150°C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialien sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, n-Dotanden, Wide-Band-Gap-Materialien, Elektronenblockiermaterialien und Lochblockiermaterialien.

Die vorliegenden Erfindung betrifft daher auch eine Zusammensetzung enthaltend wenigstens eine erfindungsgemäße Verbindung sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine erfindungsgemäße Verbindung sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\mathrm{HOMO(eV)} = ((\mathrm{HEh}*27.212)-0.9899)/1.1206$$

$$\mathrm{LUMO(eV)} = ((\mathrm{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine erfindungsgemäße Verbindung sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden

Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960 und den noch nicht offen gelegten Anmeldungen EP 13004411.8, EP 14000345.0, EP 14000417.7 und EP 14002623.8 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

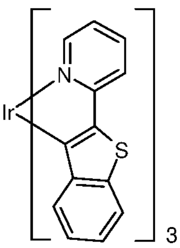
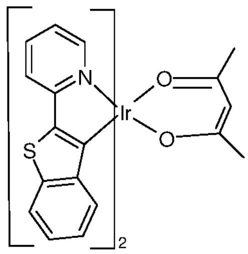
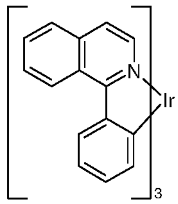

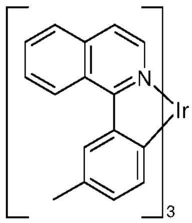
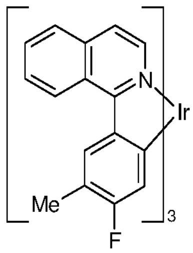
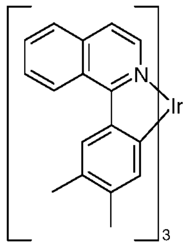

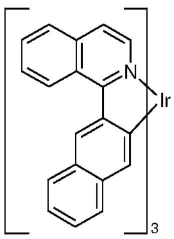
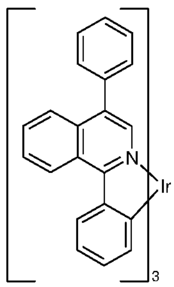
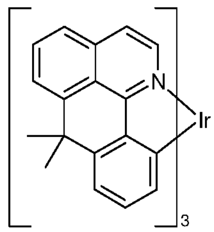

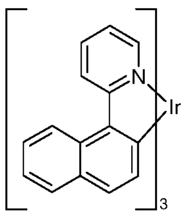
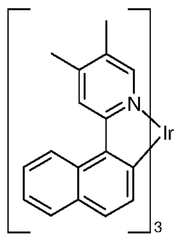
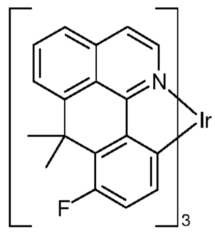

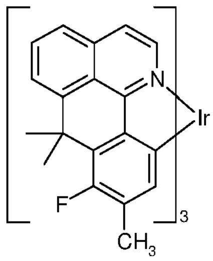
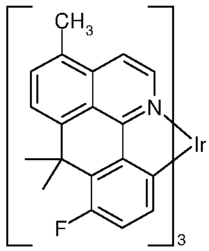
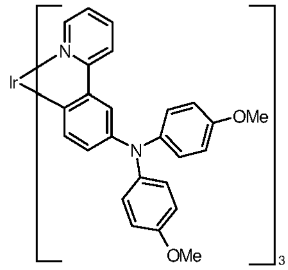

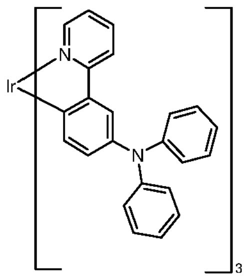
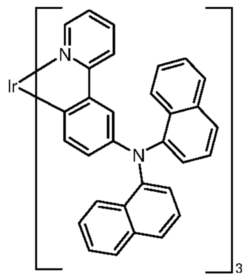
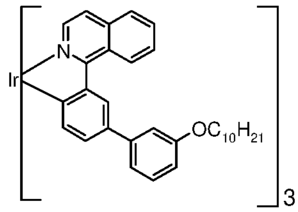

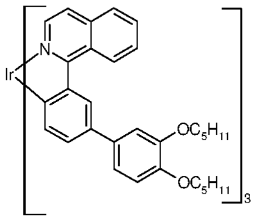
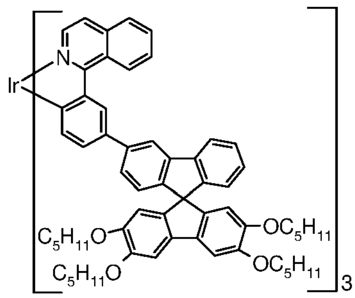
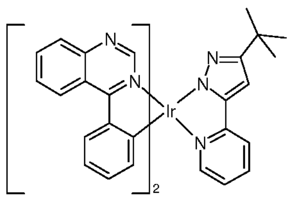

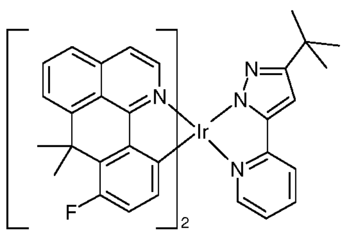
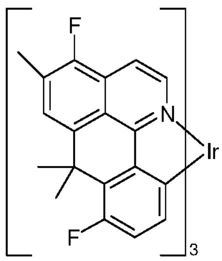
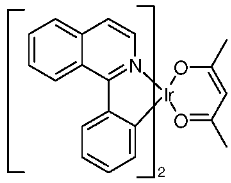

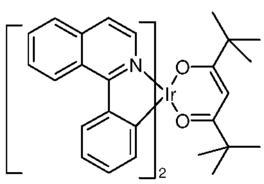
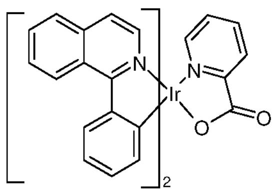
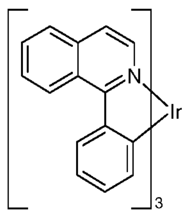

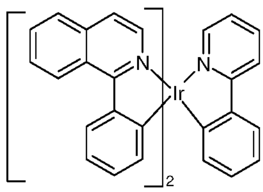
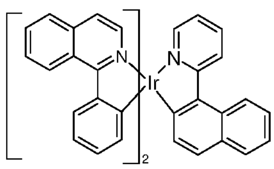
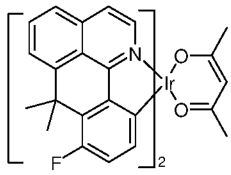

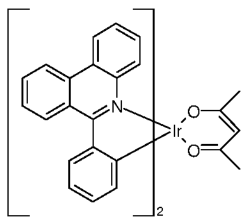
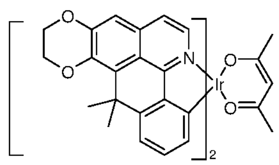
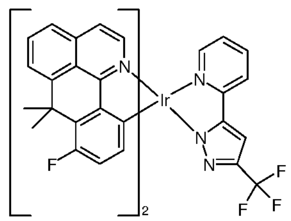

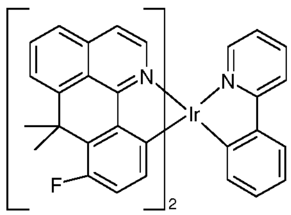
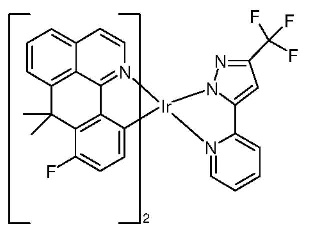
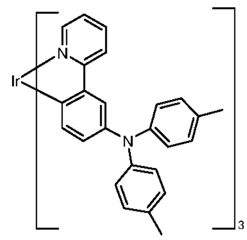

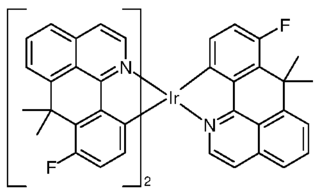
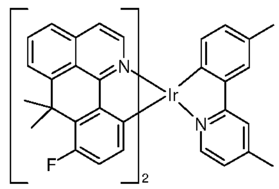
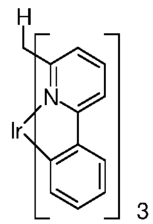

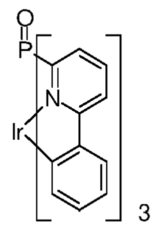
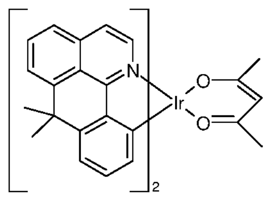
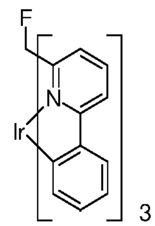

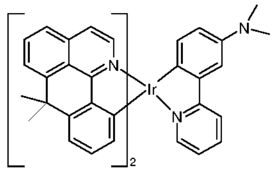
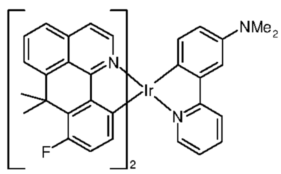
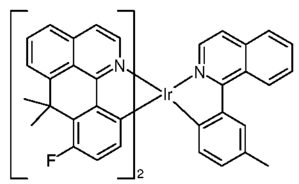

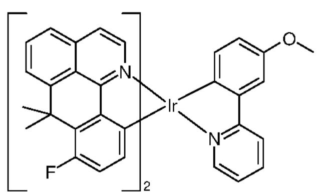
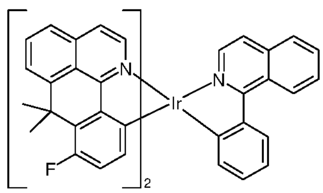
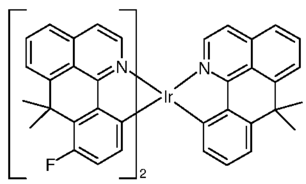

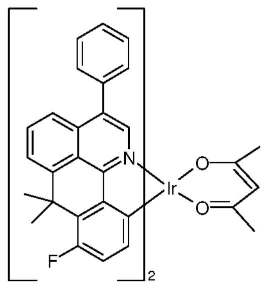
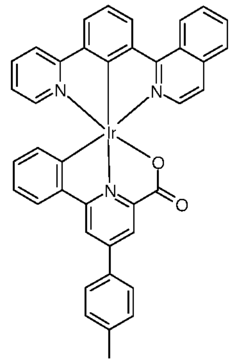
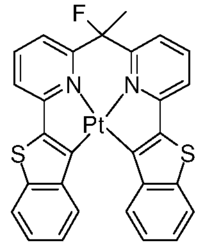

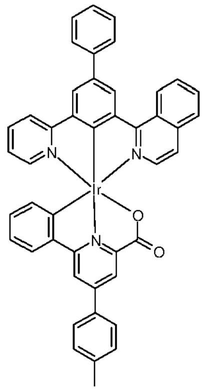
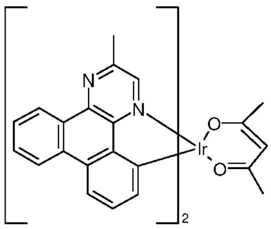
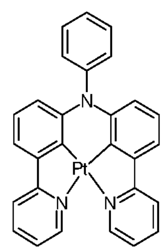

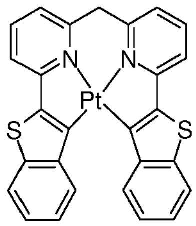
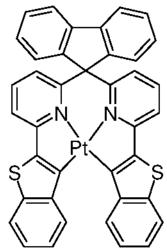
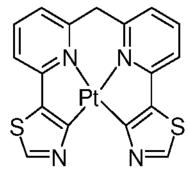

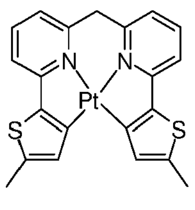
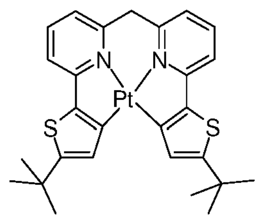
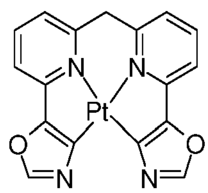

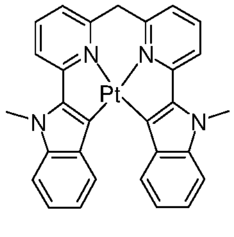
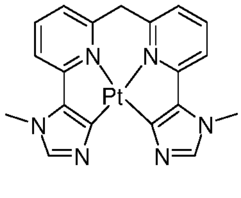
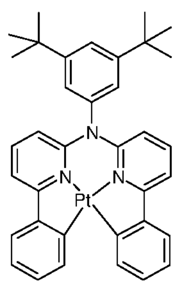

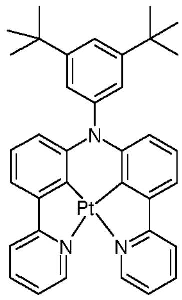
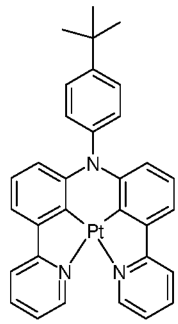
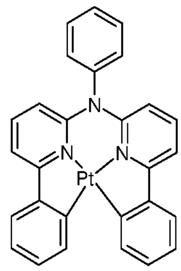

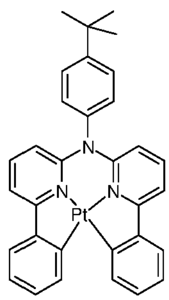
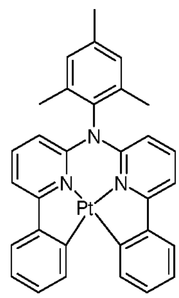
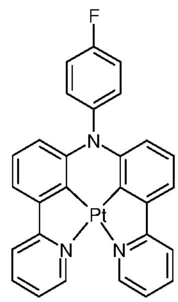

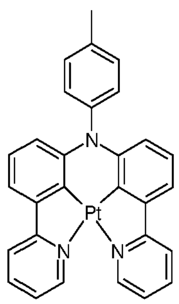
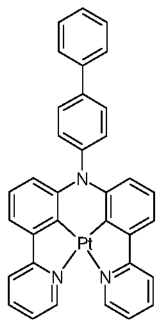
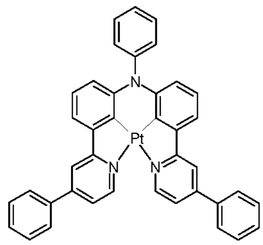

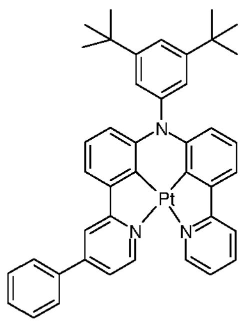
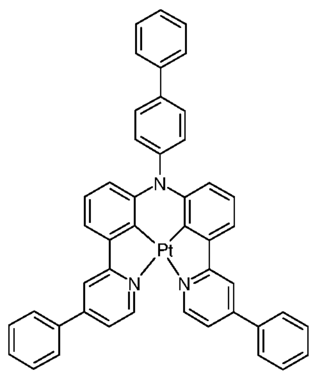
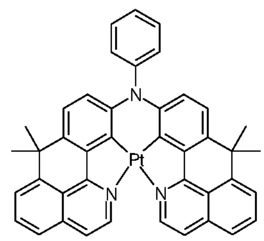

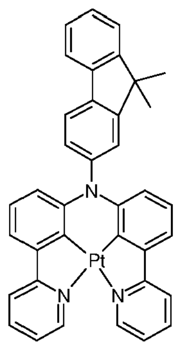
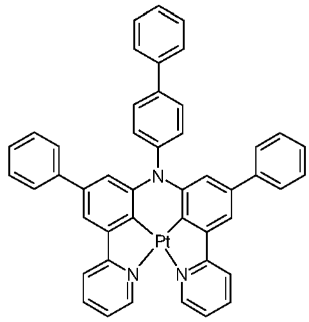
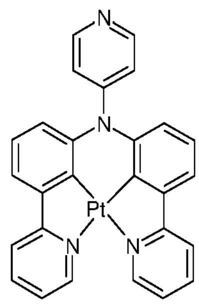

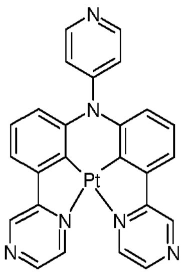
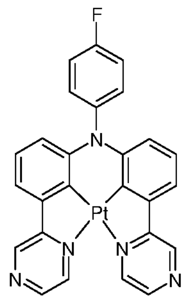
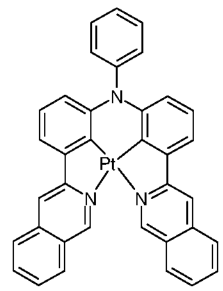

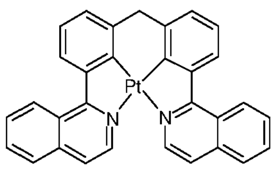
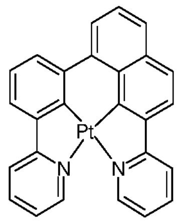
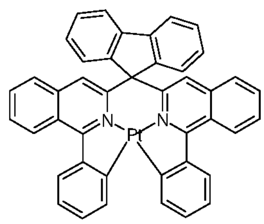

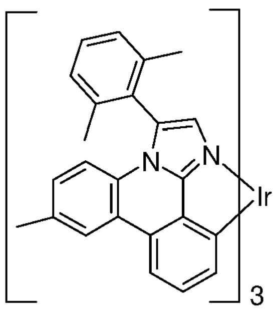
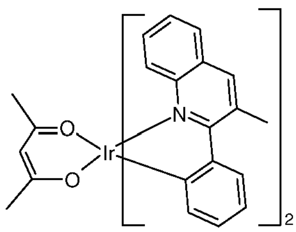
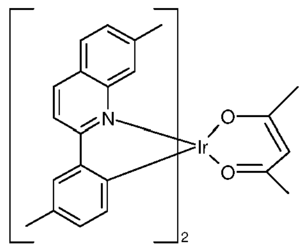

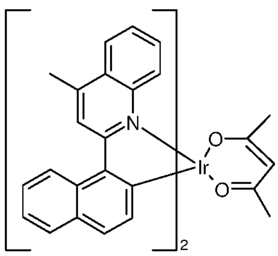
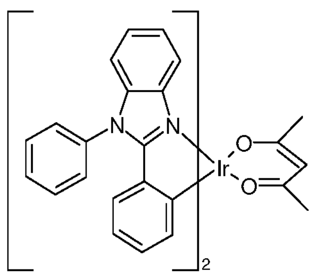
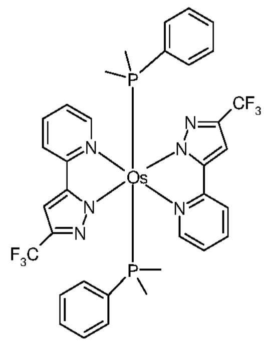

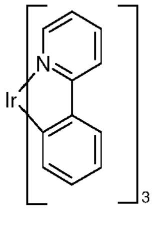
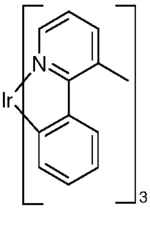
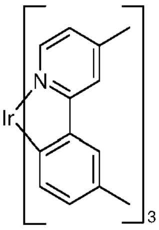

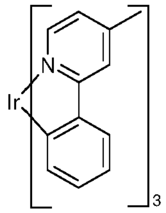
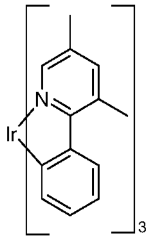
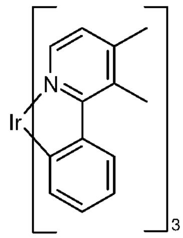

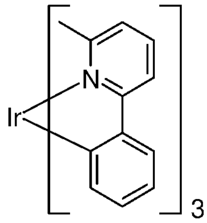
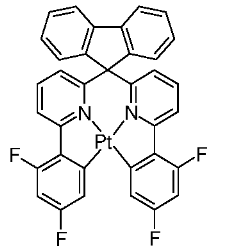
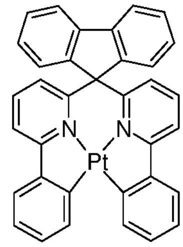

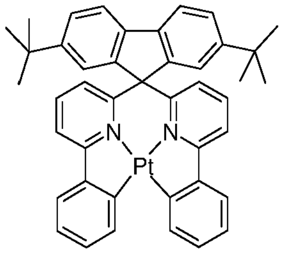
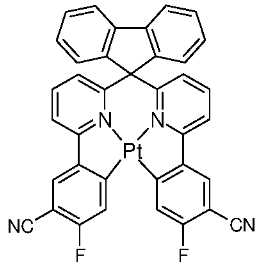
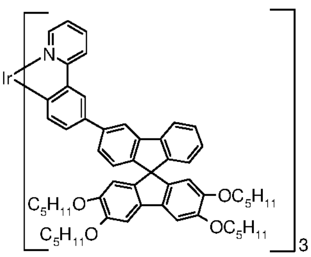

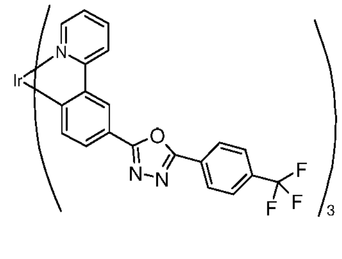
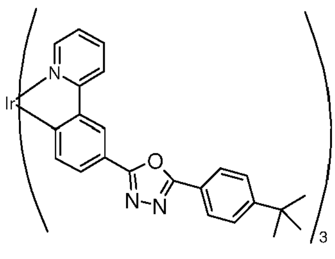
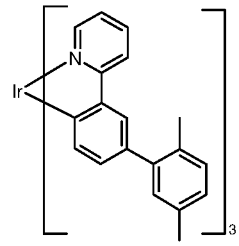

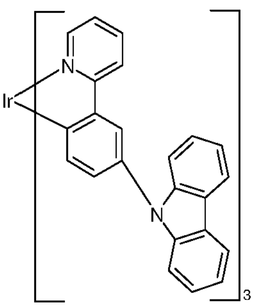
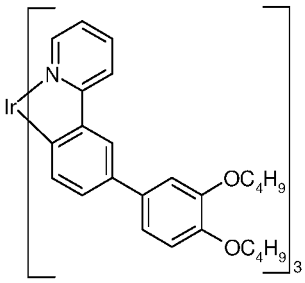
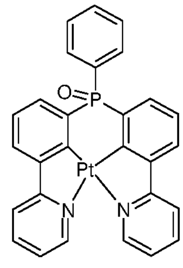

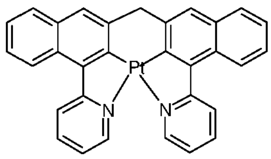
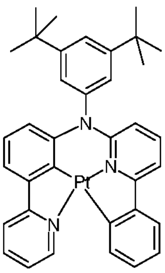
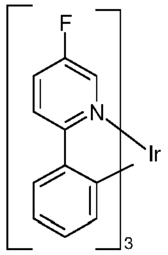

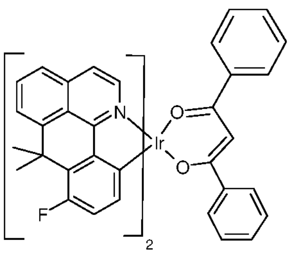
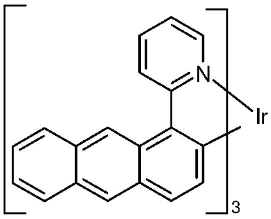
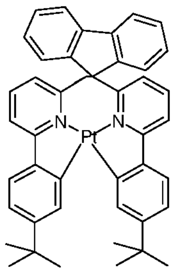

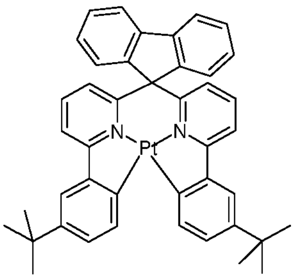
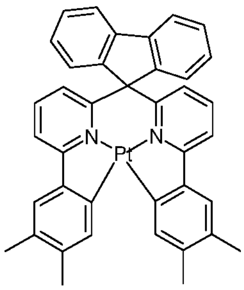
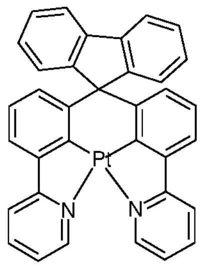

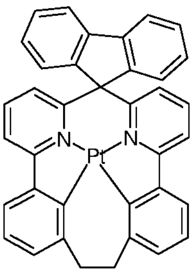
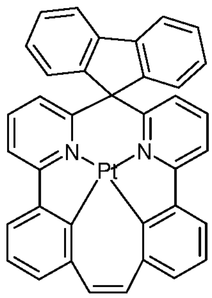
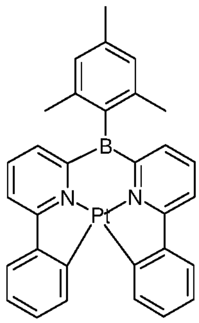

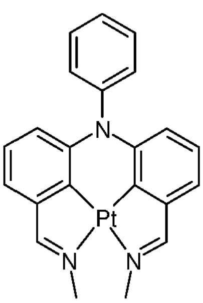
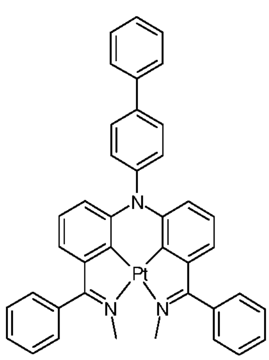
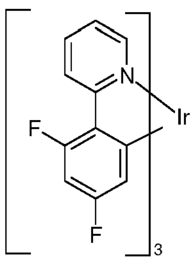

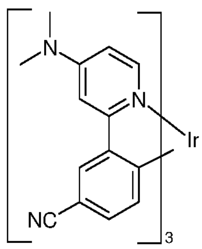
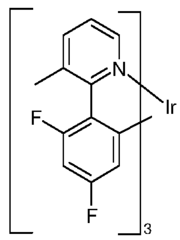
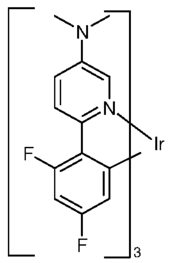

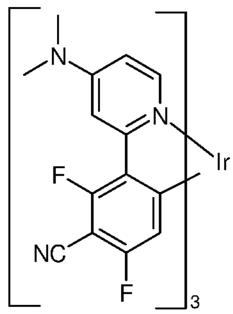
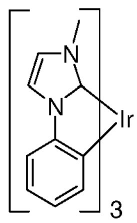
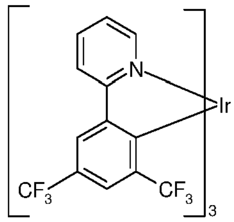

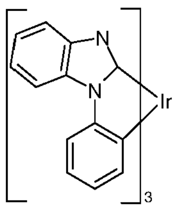
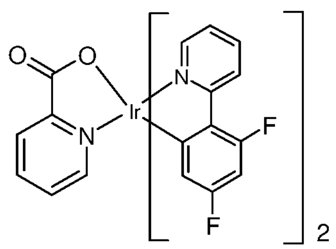
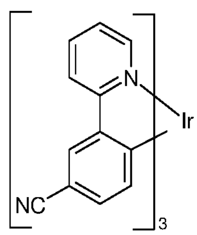

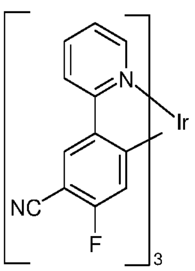
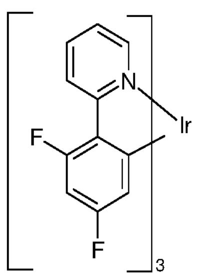
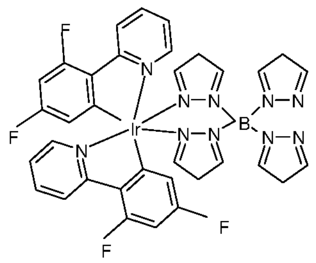

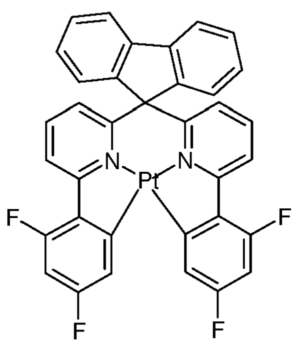
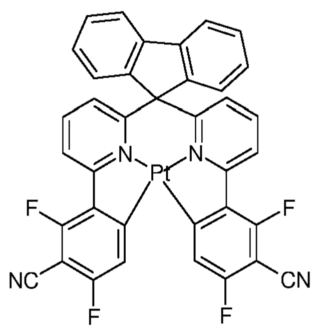
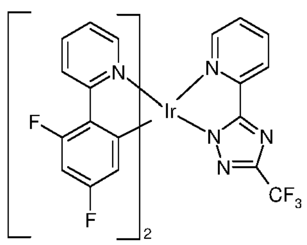

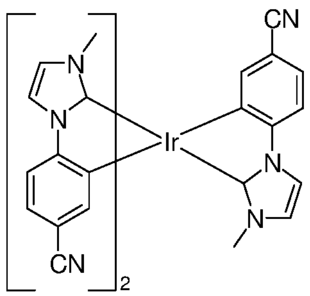 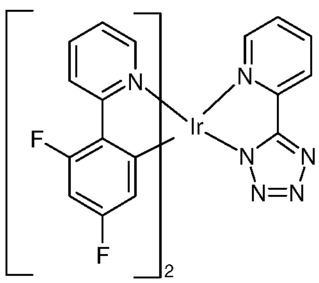 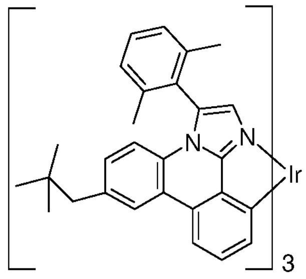

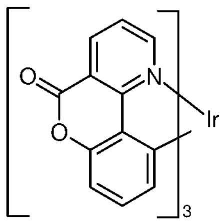 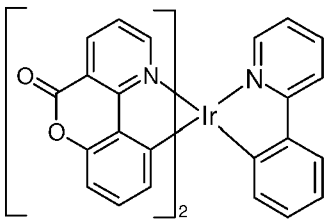 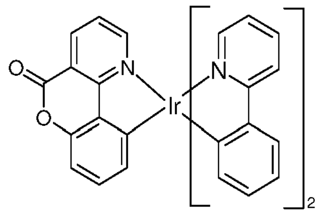

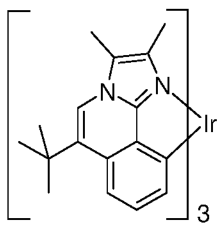 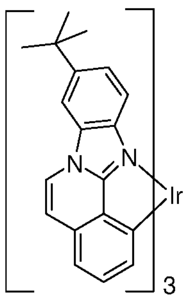 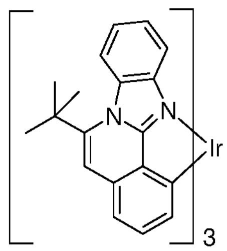

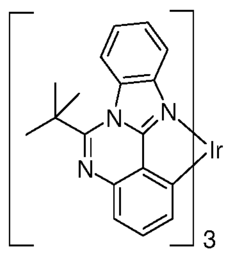 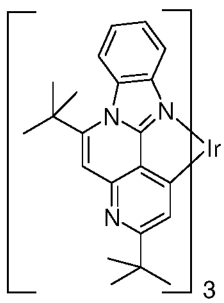 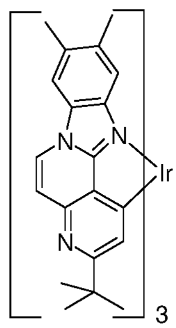

Die oben beschriebenen erfindungsgemäßen Verbindung können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine erfindungsgemäße Verbindung enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I) und/oder Formel (II). Besonders bevorzugt sind organische Elektrolumineszenzvorrichtungen. Aktive Komponenten sind generell die organischen oder anorganischen Materialien, welche zwischen

Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder der noch nicht offen gelegten Anmeldung EP 14002104.9. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

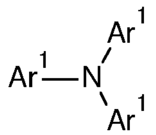

Formel (TA-1)

wobei $Ar^1$ gleich oder verschieden bei jedem Auftreten die oben, insbesondere für Formel (Q-1) genannte Bedeutung aufweist. Bevorzugt sind die Gruppen $Ar^1$ gleich oder verschieden bei jedem Auftreten ausgewählt aus den oben genannten Gruppen $R^1$-1 bis $R^1$-79, besonders bevorzugt $R^1$-1 bis $R^1$-51.

In einer bevorzugten Ausführungsform der Verbindungen der Formel (TA-1) ist mindestens eine Gruppe $Ar^1$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe Ar ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^1$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^1$ ausgewählt aus einer Fluoren- oder Spirobifluorengruppe, insbesondere einer 4-Fluoren- bzw. 4-Spirobifluorengruppe, und eine Gruppe $Ar^1$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^1$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

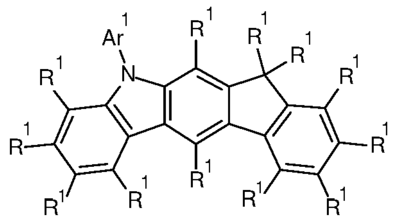

Formel (TA-2)

wobei $Ar^1$ und $R^1$ die oben insbesondere für Formeln (III), (IV) und/oder (Q-1) aufgeführten Bedeutungen aufweisen. Dabei sind bevorzugte Ausführungsformen der Gruppe $Ar^1$ die oben aufgeführten Strukturen $R^1$-1 bis $R^1$-79, besonders bevorzugt $R^1$-1 bis $R^1$-51.

Eine bevorzugte Ausführungsform der Verbindungen der Formel (TA-2) sind die Verbindungen der folgenden Formel (TA-2a),

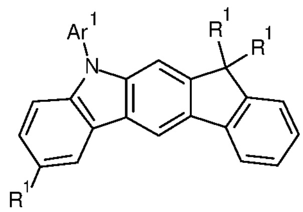

Formel (TA-2a)

wobei $Ar^1$ und $R^1$ die oben, insbesondere für Formeln (III), (IV) und/oder (Q-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

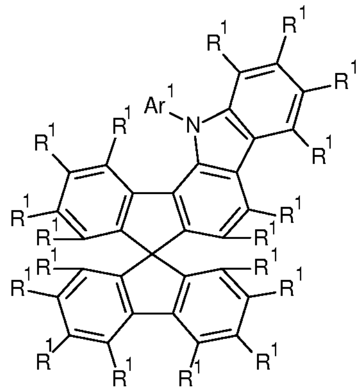

Formel (TA-3)

wobei $Ar^1$ und $R^1$ die oben, insbesondere für Formeln (III), (IV) und/oder (Q-1) aufgeführten Bedeutungen aufweisen. Dabei sind bevorzugte Ausführungsformen der Gruppe $Ar^1$ die oben aufgeführten Strukturen $R^1$-1 bis $R^1$-79, besonders bevorzugt $R^1$-1 bis $R^1$-51.

Eine bevorzugte Ausführungsform der Verbindungen der Formel (TA-3) sind die Verbindungen der folgenden Formel (TA-3a),

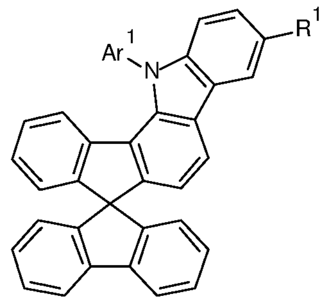

Formel (TA-3a)

wobei $Ar^1$ und $R^1$ die oben, insbesondere für Formeln (III), (IV) und/oder (Q-1) aufgeführten Bedeutungen aufweisen. Dabei sind bevorzugte Ausführungsformen der Gruppe $Ar^1$ die oben aufgeführten Strukturen $R^1$-1 bis $R^1$-79, besonders bevorzugt $R^1$-1 bis $R^1$-51.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

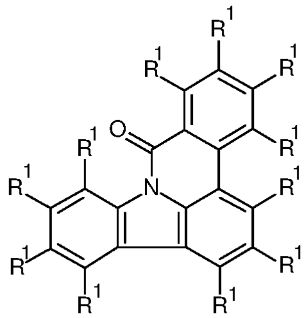

Formel (LAC-1)

wobei $R^1$ die oben, insbesondere für Formeln (III) und/oder (IV) aufgeführte Bedeutung aufweist.

Eine bevorzugte Ausführungsform der Verbindungen der Formel (LAC-1) sind die Verbindungen der folgenden Formel (LAC-1a),

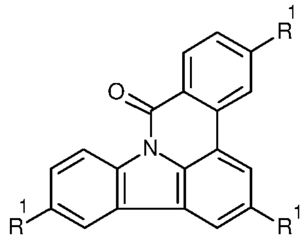

Formel (LAC-1a)

wobei $R^1$ die oben, insbesondere für Formeln (III) und/oder (IV) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (III) und/oder Formel (IV) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr TriplettEmittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend eine erfindungsgemäße Verbindung in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die erfindungsgemäßen Verbindung als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen

Vorrichtungen, insbesondere organische Elektrolumineszenzvorrichtungen enthaltend efindungsgemäße Verbindungen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend erfindungsgemäße Verbindungen, Oligomere, Polymere oder Dendrimere , insbesondere als elektronenleitende Materialien, weisen eine sehr gute Lebensdauer auf.

2. Elektronischen Vorrichtungen, insbesondere organische Elektrolumineszenzvorrichtungen enthaltend erfindungsgemäße Verbindungen, Oligomere, Polymere oder Dendrimere als elektronenleitende Materialien weisen eine hervorragende Effizienz auf. Insbesondere ist die Effizienz deutlich höher gegenüber analogen, nicht erfindungsgemäßen Verbindungen.

3. Die erfindungsgemäßen Verbindungen, Oligomere, Polymere oder Dendrimere zeigen eine sehr hohe Stabilität und führen zu Verbindungen mit einer sehr hohen Lebensdauer.

4. Mit erfindungsgemäßen Verbindungen, Oligomere, Polymere oder Dendrimere kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von erfindungsgemäßen Verbindungen, Oligomere, Polymere oder Dendrimere in Schichten elektronischer Vorrichtungen, insbesondere organischer Elektrolumineszenzvorrichtungen führt zu einer hohen Mobilität der Elektron-Leiterstrukturen.

6. Die erfindungsgemäßen Verbindungen, Oligomere, Polymere oder Dendrimere zeichnen sich durch eine ausgezeichnete thermische Stabilität aus, wobei Verbindungen mit einer Molmasse von weniger als ca. 1200 g/mol gut sublimierbar sind.

7. Die erfindungsgemäßen Verbindungen, Oligomere, Polymere oder Dendrimere weisen eine ausgezeichnete Glasfilmbildung auf.

8. Die erfindungsgemäßen Verbindungen, Oligomere, Polymere oder Dendrimere bilden aus Lösungen sehr gute Filme.

9. Die erfindungsgemäßen Verbindungen, Oligomere, Polymere oder Dendrimere weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Lochblockiermaterial, Elektroneninjektionsmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht

und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Dies gilt insbesondere für erfindungsgemäße Verbindungen, die keine Carbazolstruktur aufweisen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

## Synthesebeispiele

### a) 4-Brom-9-methyl-9-phenyl-9H-fluoren

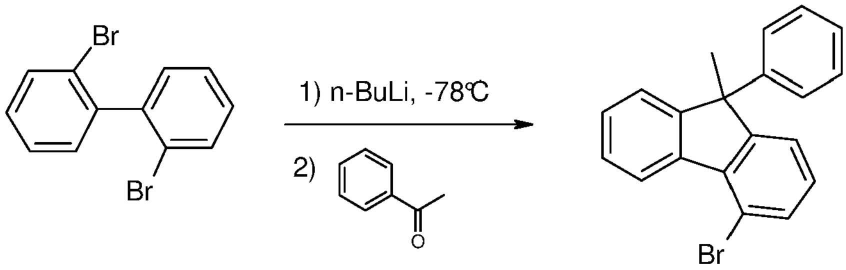


30 g (94 mmol) 2,2'-Dibrom-biphenyl werden in einem ausgeheizten Kolben in 200 mL getrocknetem THF gelöst. Die Reaktionsmischung wird auf -78°C gekühlt. Bei dieser Temperatur werden 37,7 mL einer 2,5 M-Lösung n-Butyllithium in Hexan (94 mmol) langsam zugetropft (Dauer: ca 1 h). Der Ansatz wird 1 h bei -70°C nachgerührt. An schließend werden 11,1 mL Acetophenon (94 mmol) in 100 ml THF gelöst und bei -70°C zugetropft. Nach beendeter Zugabe wird die Reaktionsmischung langsam auf Raumtemperatur erwärmt, mit $NH_4Cl$ gequencht und anschließend am Rotationsverdampfer eingeengt. Die einrotierte Lösung wird vorsichtig mit 300 ml Essigsäure versetzt und anschließend werden 50 ml rauchende HCl zugegeben. Der Ansatz wird auf 75°C erhitzt und 6 h dort gehalten. Dabei fällt ein weißer Feststoff aus.

Der Ansatz wird auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 25,3 g (75 mmol) (80% der Theorie)

**b) 4-Brom-9,9-diphenyl-9H-fluoren**

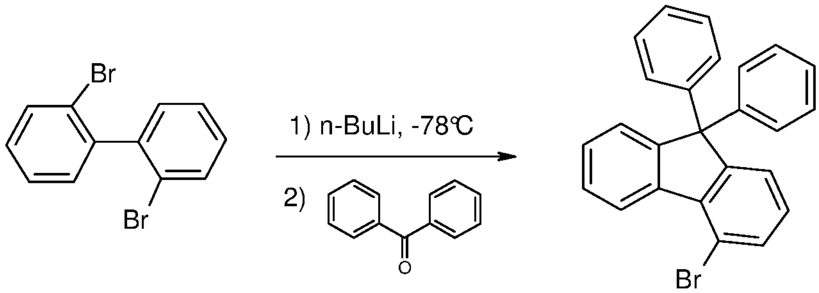


37 g (152 mmol) 2,2'-Dibrom-biphenyl werden in einem ausgeheizten Kolben in 300 mL getrocknetem THF gelöst. Die Reaktionsmischung wird auf -78°C gekühlt. Bei dieser Temperatur werden 75 mL einer 15%-igen Lösung n-Butyllithium in Hexan (119 mmol) langsam zugetropft (Dauer: ca 1 Stunde). Der Ansatz wird 1 h bei -70°C nachgerührt. Anschließend werden 21,8 g Benzophenon (119 mmol) in 100 ml THF gelöst und bei - 70°C zugetropft. Nach beendeter Zugabe wird die Reaktionsmischung langsam auf Raumtemperatur erwärmt, mit $NH_4Cl$ gequencht und anschließend am Rotationsverdampfer eingeengt. Die einrotierte Lösung wird vorsichtig mit 510 ml Essigsäure versetzt und anschließend werden 100 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Ausbe ute beträgt 33,2 g (83 mmol) (70% der Theorie)

Analog dazu werden die folgenden bromierten Verbindungen hergestellt:

| | Edukt 1 | Edukt 2 | Produkt | Ausbeute |
|---|---|---|---|---|
| b1 | 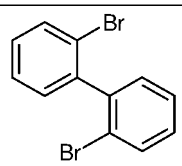 | 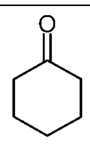 | 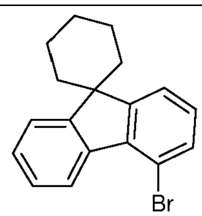 | 78% |

(fortgesetzt)

| | Edukt 1 | Edukt 2 | Produkt | Ausbeute |
|---|---|---|---|---|
| b2 | 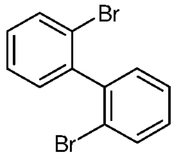 | 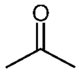 | 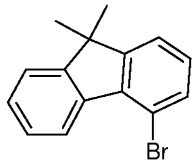 | 70% |
| b3 | 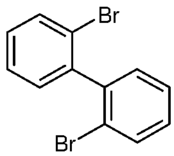 | 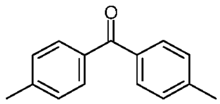 | 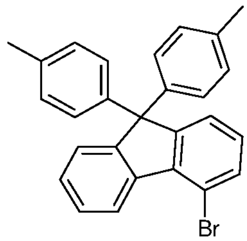 | 82% |

**c) 1-Brom-spiro-9,9'-bifluoren**

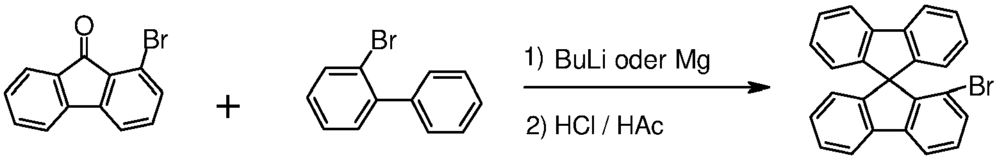


Aus mit Jod aktivierten 2.7 g (110 mmol) Magnesiumspänen und einer Mischung aus 25.6 g (110 mmol) 2-Brombiphenyl, 0.8 ml 1,2-Dichlorethan, 50 ml 1,2-Dimethoxyethan, 400 ml THF und 200 ml Toluol wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 25.9 g (100 mmol) 1-Brom-fluorenon, [36804-63-4] in 500 ml THF zu, erwärmt die Reaktionsmischung für 4 h auf 50 °C und rührt dann 12 h bei Raumtemperatur nach. Man gibt 100 ml Wasser zu, rührt kurz nach, trennt die organische Phase ab und entfernt das Lösungsmittel im Vakuum. Der Rückstand wird in der Wärme bei 40 °C in 500 ml Eisessig suspendiert, die Suspension wird mit 0.5 ml konz. Schwefelsäure versetzt, und anschließend 2 h bei 100 °C nachgerührt. Nach E rkalten saugt man vom ausgefallenen Feststoff ab, wäscht diesen einmal mit 100 ml Eisessig, dreimal mit je 100 ml Ethanol und kristallisiert abschließend aus Dioxan um. Ausbeute: 26.9 g (68 mmol), 68%; Reinheit ca. 98% nch $^{1}$H-NMR.

Analog wird die folgende Verbindung erhalten:

| | Edukt 1 | Edukt 2 | Produkt | Ausbeute |
|---|---|---|---|---|
| 1c | 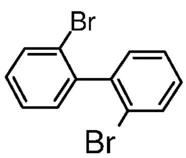 13029-09-9 | 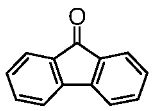 486-25-9 | 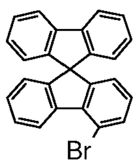 1161009-88-6 | 90% |

**d) 4-Biphenyl-4-yl-2-chloro-quinazolin**

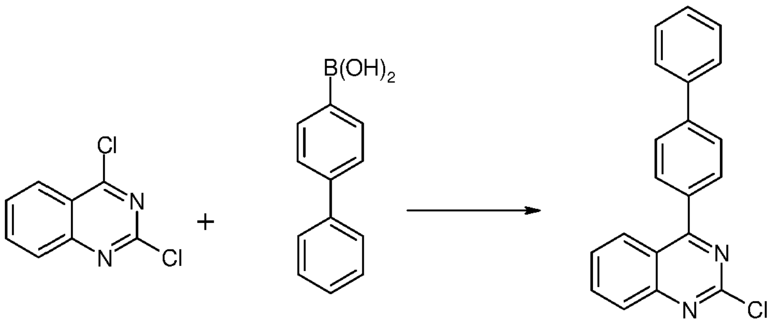

13 g (70 mmol) Biphenyl-4-boronsäure, 13,8 g (70 mmol) 2,4-Dichlorquinazolin und 14,7 g (139 mmol) Natriumcarbonat werden in 200 mL Toulol, 52 mL Ethanol und 100 mL Wasser suspendiert. Zu dieser Suspension werden 800 mg (0,69 mmol) Tetrakisphenylphosphinpalladium(0) gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Heptan / Dichlormethan umkristallisiert. Die Ausbeute beträgt 13 g (41 mmol), entsprechend 59% der Theorie.

Analog werden die folgenden Verbindungen erhalten:

| | Edukt 1 | Edukt 2 | Produkt | Ausbeute |
|---|---|---|---|---|
| 2d | 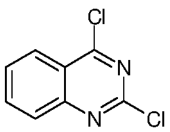 [607-68-1] | 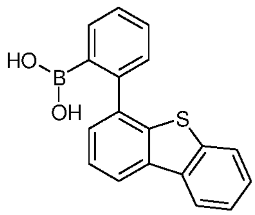 [1384699-53-9] | 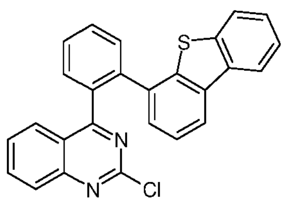 | 76% |
| 3d | 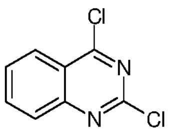 [607-68-1] | 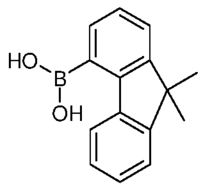 [1246022-50-3] | 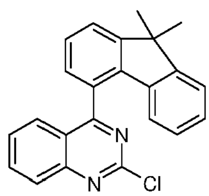 | 75% |
| 4d | 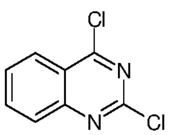 [607-68-1] | 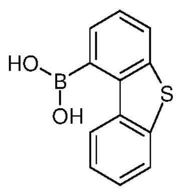 [1245943-60-5] | 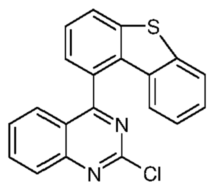 | 73% |
| 5d | 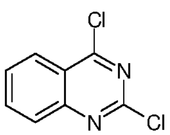 [607-68-1] | 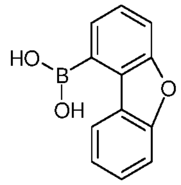 [162607-19-4] | 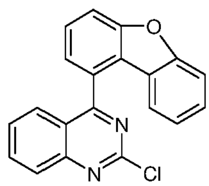 | 78% |

(fortgesetzt)

| | Edukt 1 | Edukt 2 | Produkt | Ausbeute |
|---|---|---|---|---|
| 6d | 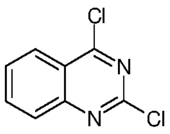 [607-68-1] | 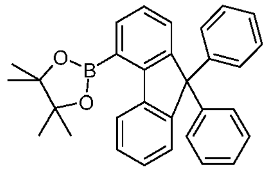 [1259280-37-9] | 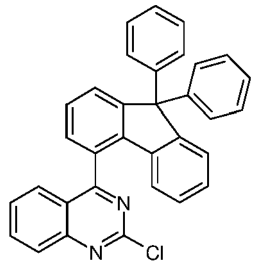 | 76% |
| 7d | 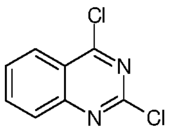 [607-68-1] | 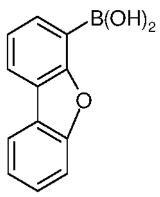 [100124-06-9] | 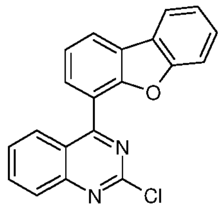 | 77% |
| 8d | 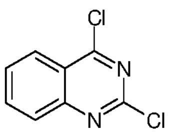 [607-68-1] | 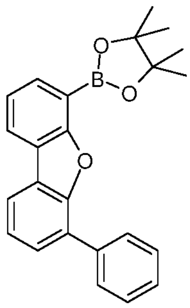 [1556069-49-8] | 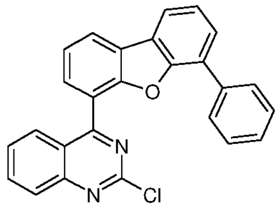 | 72% |
| 9d | 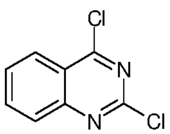 [607-68-1] | 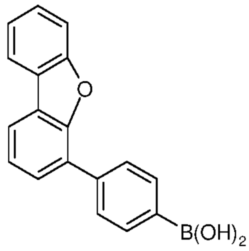 [796071-96-0] | 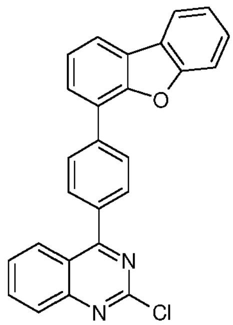 [607-68-1] | 73% |
| 10d | 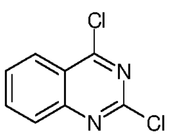 [607-68-1] | 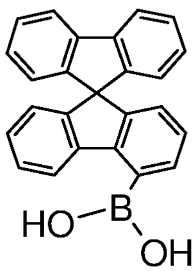 | 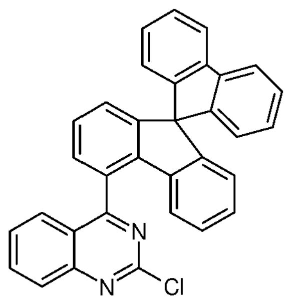 | 72% |

**e) Synthese von 4-(9H,9'H-[9,9']Bifluorenyl-1-yl)-2-phenyl-quinazoline**

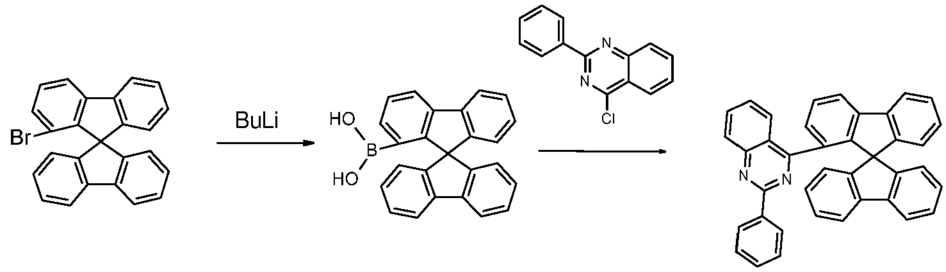


**Schritt 1) Synthese von Spiro-9,9'-bifluoren-1-boronsäure**

Eine auf -78 °C gekühlte Lösung von 106 g (270 mmol) 1-Brom-9-spirobifluoren in 1500 ml Diethylether wird tropfenweise mit 110 ml (276 mmol) n-Buthyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit ein er Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 94,5 g (255 mmol), 99 % d. Th.; Reinheit: 99 % nach HPLC.

Analog werden die folgenden Verbindungen erhalten:

| | Edukt 1 | Produkt | Ausbeute |
|---|---|---|---|
| 1e | 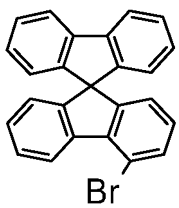 | 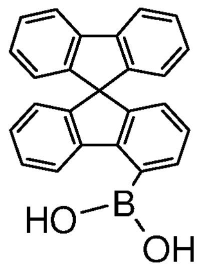 | 83% |
| 2e | 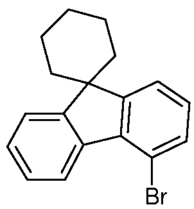 | 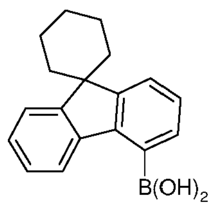 | 80% |
| 3e | 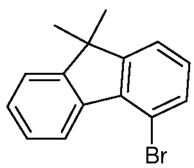 | 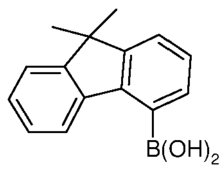 | 83% |

(fortgesetzt)

| | Edukt 1 | Produkt | Ausbeute |
|---|---|---|---|
| 4e | 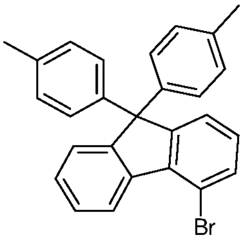 | 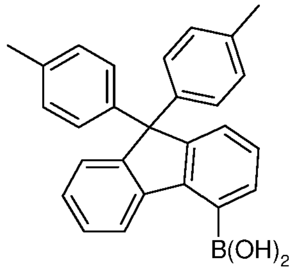 | 75% |
| 5e | 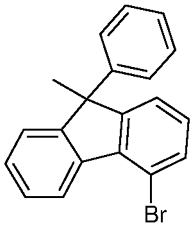 | 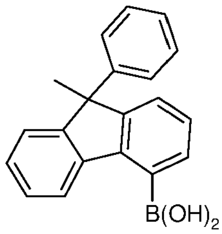 | 70% |
| 6e | 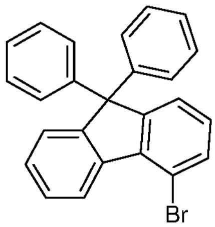 | 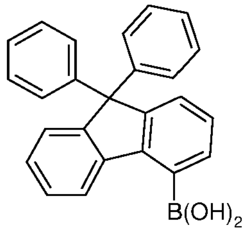 | 72% |
| 7e | 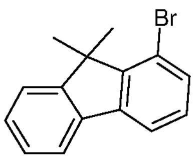 [1225053-54-2] | 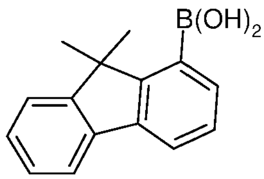 | 63% |
| 8e | 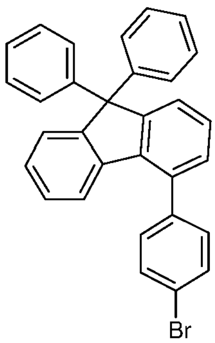 [ 1613372-04-5] | 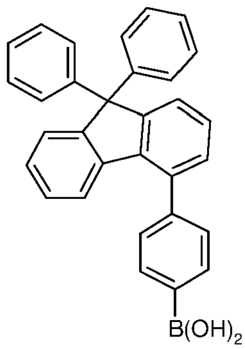 | 74% |

(fortgesetzt)

| | Edukt 1 | Produkt | Ausbeute |
|---|---|---|---|
| 9e | 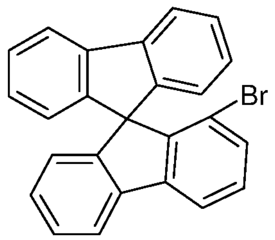 [1450933-18-2 ] | 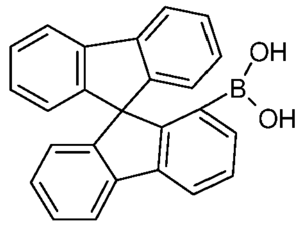 [1450933-18-2 ] | 70% |

**Schritt 2) 4-(9H,9'H-[9,9']Bifluorenyl-1-yl)-2-phenyl-quinazoline**

56,8 g (110 mmol) Spiro-9,9'-bifluoren-1 -boronsäure, 26 g (110.0 mmol) 4- -Chloro-2-phenyl-quinazolin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / iso-Propanol umkristallisiert und abschließend im Hochvakuum (p = 5 x $10^{-5}$ mbar, T = 350 °C) sublimiert. Die Ausbeute beträgt 64 g (43,5 mmol), entsprechend 80 % der Theorie.

Analog werden die folgenden Verbindungen erhalten:

| | Edukt 1 | Edukt 2 | Produkt | Ausbeute |
|---|---|---|---|---|
| 9e | 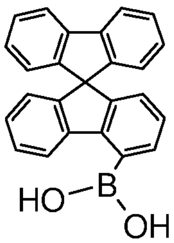 | 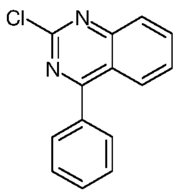 [29874-83-7] | 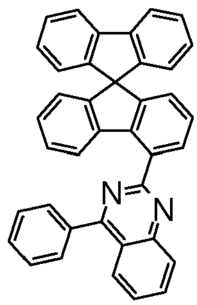 | 81% |
| 10e | 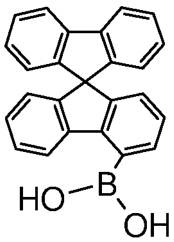 | 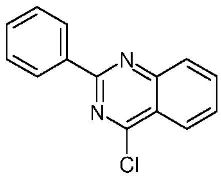 [6484-25-9] | 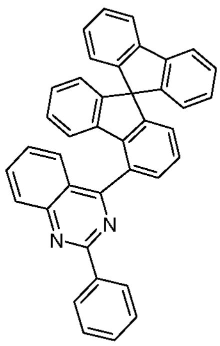 | 80% |

(fortgesetzt)

| | Edukt 1 | Edukt 2 | Produkt | Ausbeute |
|---|---|---|---|---|
| 11e | 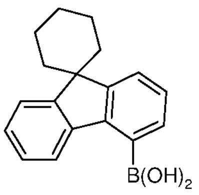 | 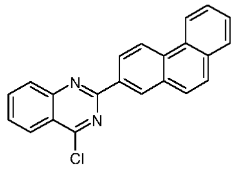 [1627575-10-3] | 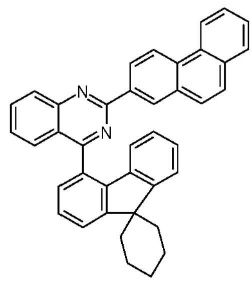 # | 72% |
| 12e | 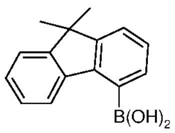 | 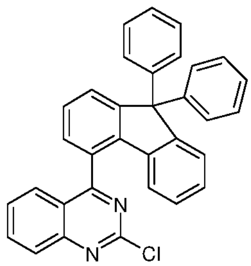 | 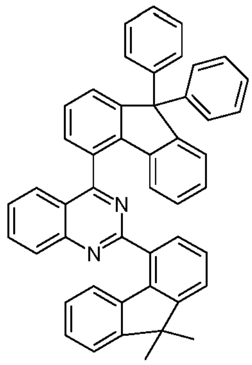 # | 70% |
| 13e | 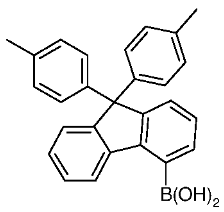 | 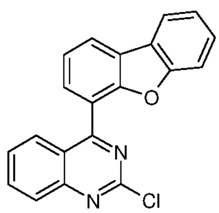 | 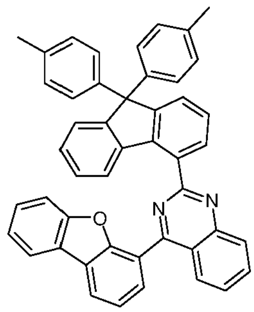 # | 82% |
| 14e | 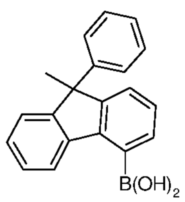 | 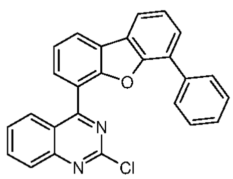 | 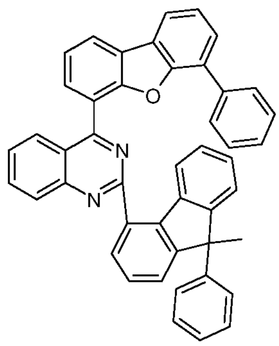 # | 78% |

(fortgesetzt)

| | Edukt 1 | Edukt 2 | Produkt | Ausbeute |
|---|---|---|---|---|
| 15e | 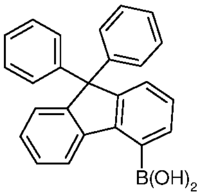 | 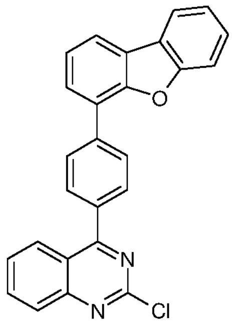 [607-68-1] | 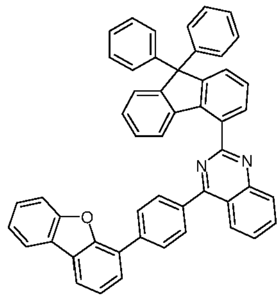 # | 79% |
| 16e | 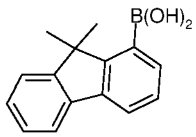 | 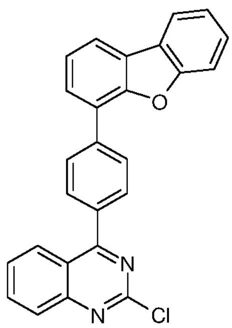 [607-68-1] | # 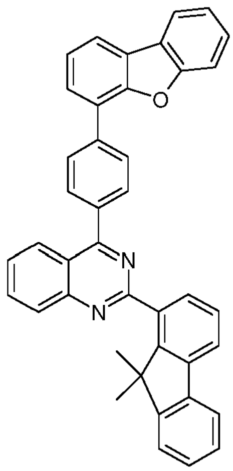 | 70% |
| 17e | 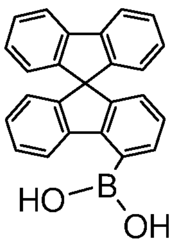 | 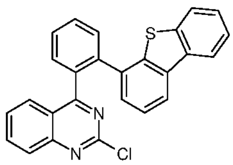 | 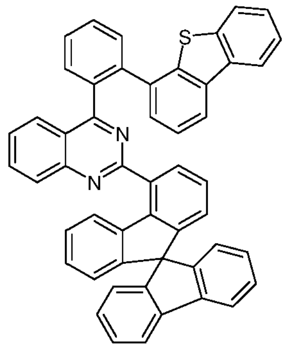 | 65% |
| 18e | 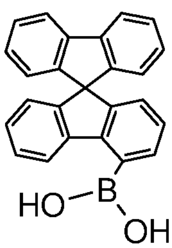 | 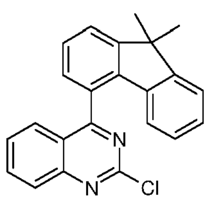 | 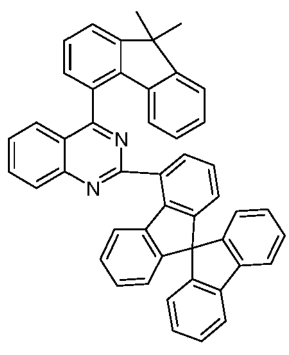 | 79% |

(fortgesetzt)

| | Edukt 1 | Edukt 2 | Produkt | Ausbeute |
|---|---|---|---|---|
| 19e | 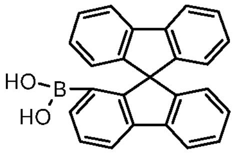 | 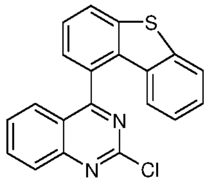 | 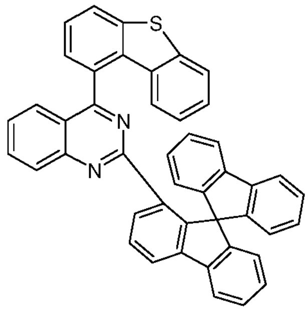 | 63% |
| 20e | 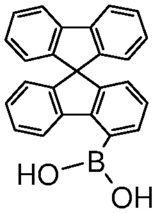 | 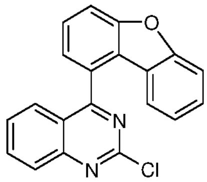 | 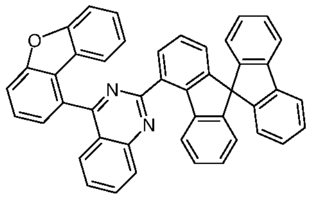 | 73% |
| 21e | 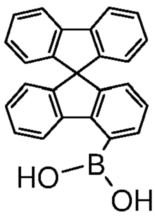 | 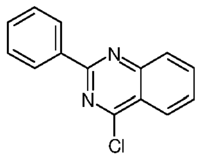 [6484-25-9] | 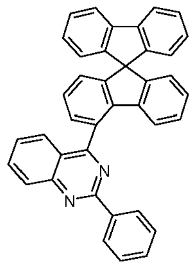 | 72% |
| 22e | 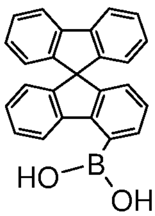 | 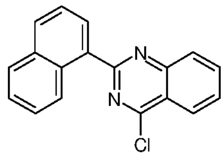 [133594-92-0] | 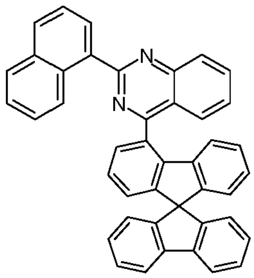 | 77% |
| 23e | 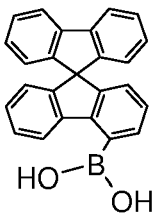 | 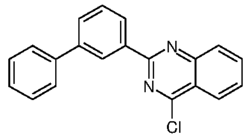 [1561960-15-3] | 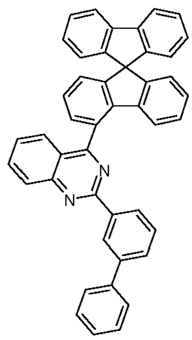 | 74% |

(fortgesetzt)

| | Edukt 1 | Edukt 2 | Produkt | Ausbeute |
|---|---|---|---|---|
| 24e | 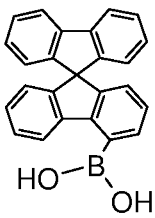 | 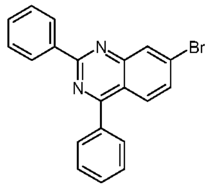 [1400697-34-8] | 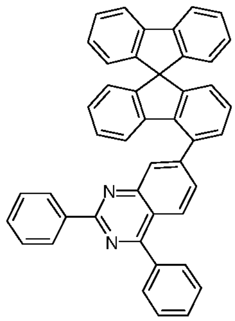 | 81% |
| 25e | 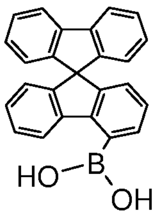 | 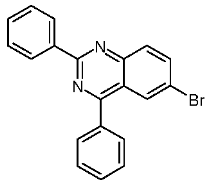 [1229609-99-7] | 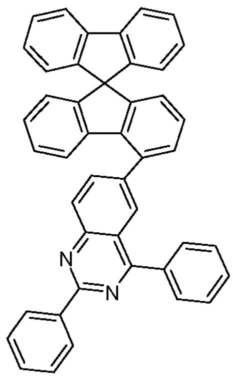 | 80% |
| 26e | 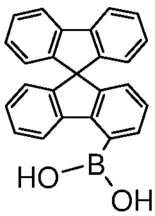 | 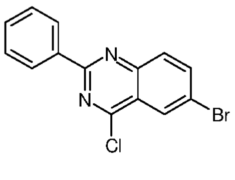 [6484-25-9] | 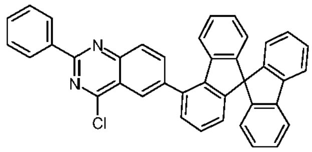 | 69% |
| 27e | 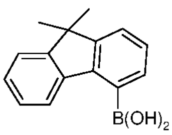 | 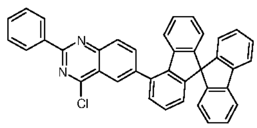 | 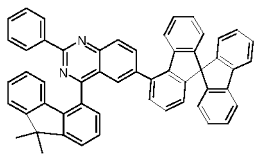 | 73% |
| 28f | 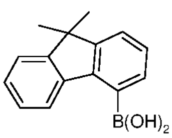 | 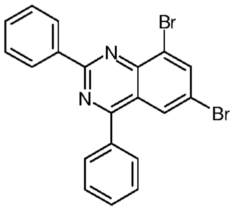 [107288-87-9] | 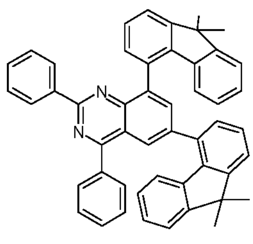 # | 68% |
| 29e | 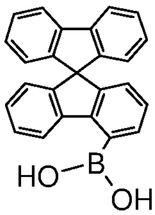 | 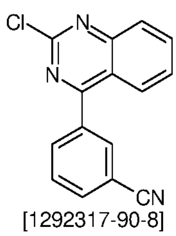 [1292317-90-8] | 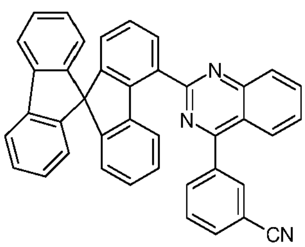 | 65% |

(fortgesetzt)

| | Edukt 1 | Edukt 2 | Produkt | Ausbeute |
|---|---|---|---|---|
| 30e | 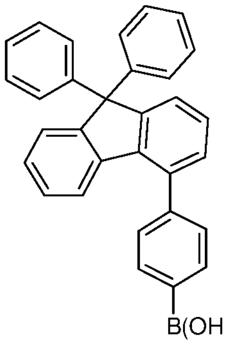 | 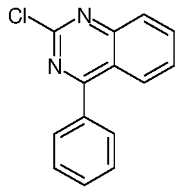 [29874-83-7] | 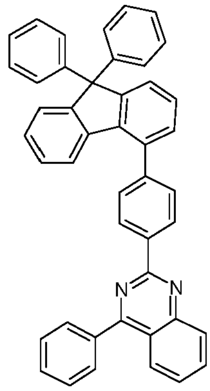 # | 68% |
| 31e | 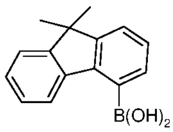 | 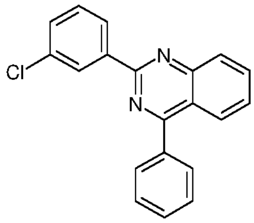 [540466-41-9] | 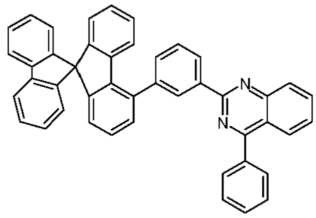 | 63% |
| 32e | 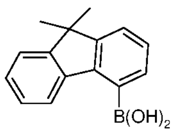 | 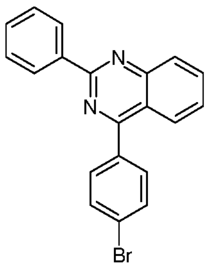 [160254-04-6] | 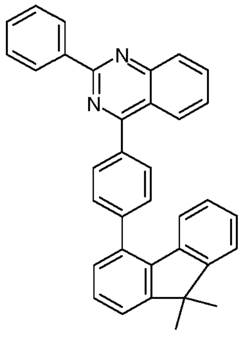 # | 79% |
| 33e | 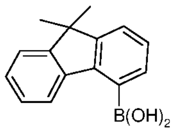 | 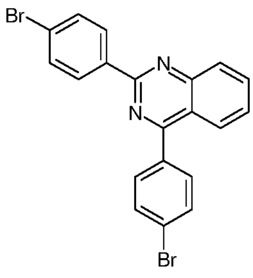 [1446785-97-2] | 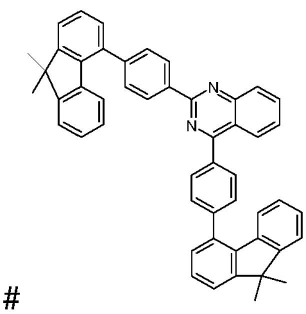 # | 81% |

(fortgesetzt)

| | Edukt 1 | Edukt 2 | Produkt | Ausbeute |
|---|---|---|---|---|
| 34e | 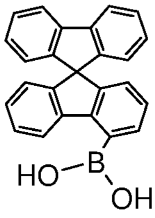 | 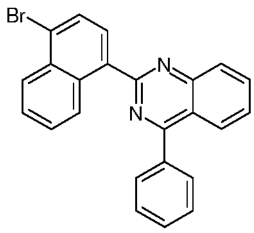 [1621469-45-1] | 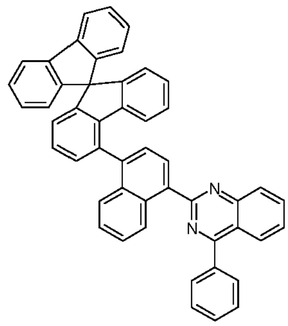 | 65% |
| 35e | 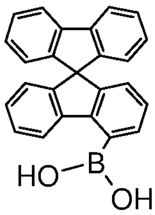 | 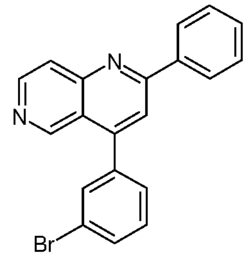 [1632307-99-3] | 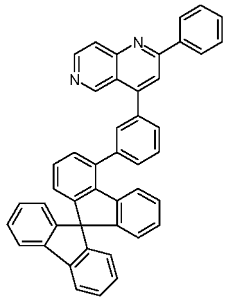 | 66% |
| 36e | 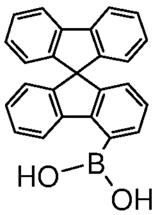 | 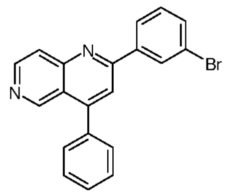 [1632307-97-1] | 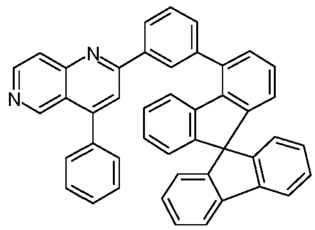 | 71% |
| 37e | 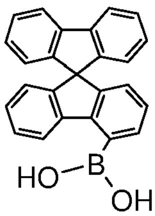 | 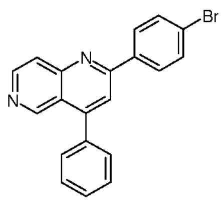 [1632307-96-0] | 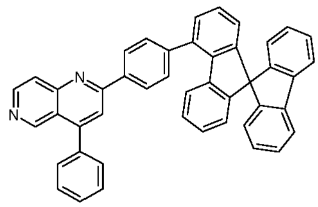 | 69% |
| 38e | 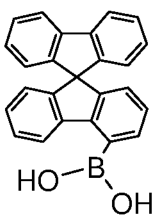 | 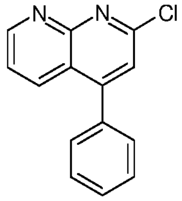 [33760-75-7] | 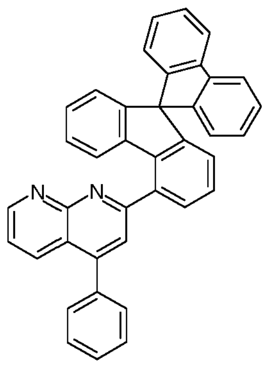 | 63% |

(fortgesetzt)

| | Edukt 1 | Edukt 2 | Produkt | Ausbeute |
|---|---|---|---|---|
| 39e | 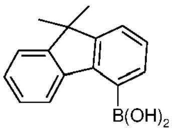 | 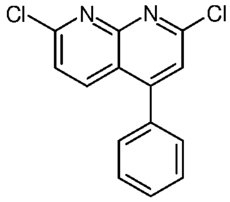 [58035-57-7] | 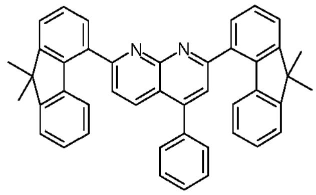 # | 65% |
| 40e | 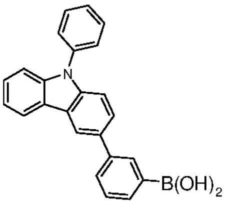 | 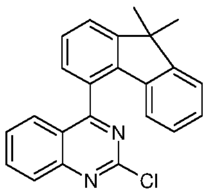 | 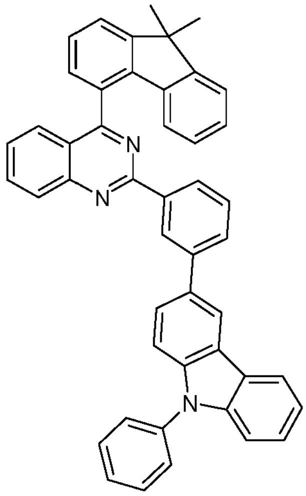 # | 60% |
| 41e | 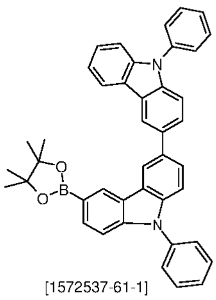 [1572537-61-1] | 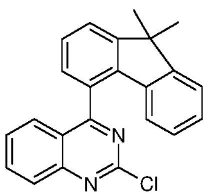 | 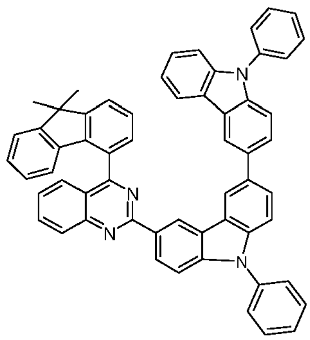 # | 61% |

(fortgesetzt)

| | Edukt 1 | Edukt 2 | Produkt | Ausbeute |
|---|---|---|---|---|
| 42e | 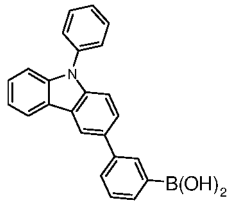 854952-60-6 | 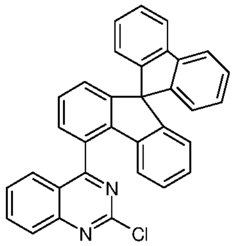 | 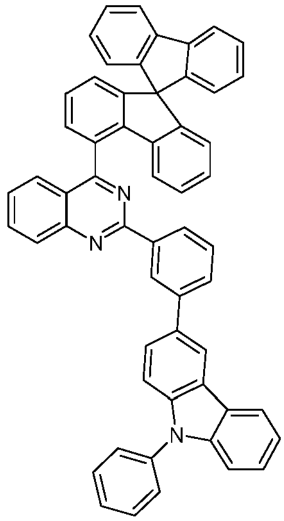 | 69% |
| 43e | 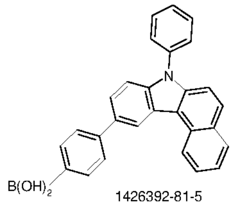 1426392-81-5 | 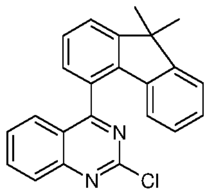 | 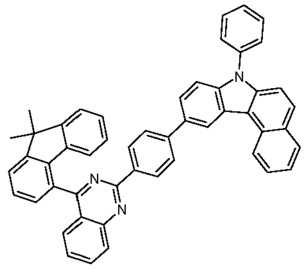 # | 64% |
| 44e | 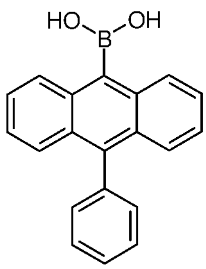 [334658-75-2] | 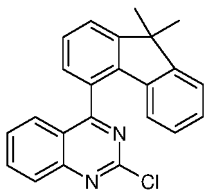 | 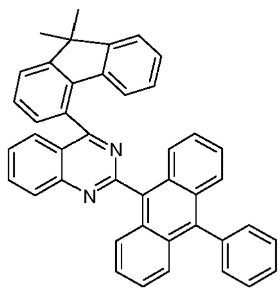 # | 66% |
| 45e | 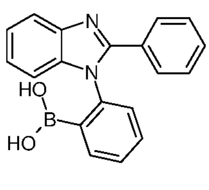 [1553408-82-4] | 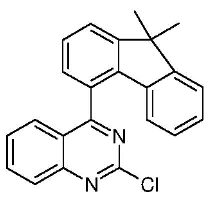 | 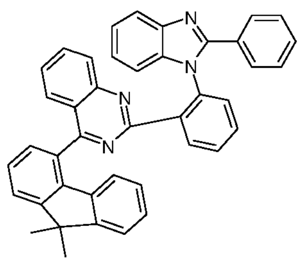 # | 69% |

(fortgesetzt)

| | Edukt 1 | Edukt 2 | Produkt | Ausbeute |
|---|---|---|---|---|
| 46e | 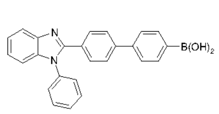 [1236181-90-0] | 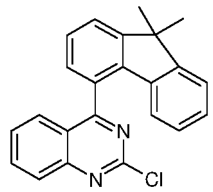 | 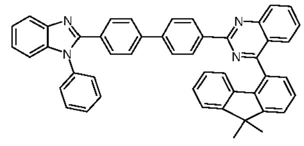 # | 70% |
| 47e | 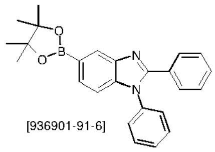 [936901-91-6] | 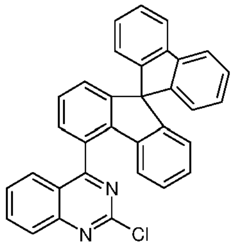 | 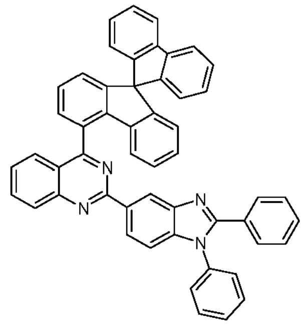 | 61% |
| 48e | 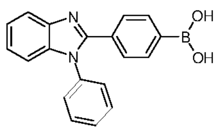 [952514-79-3] | 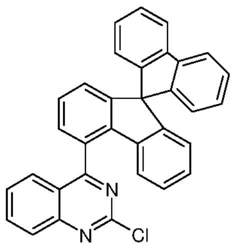 | 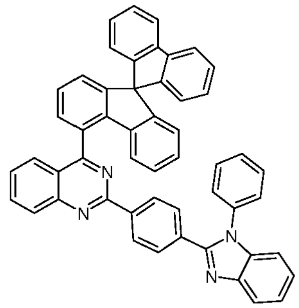 | 64% |
| 49e | 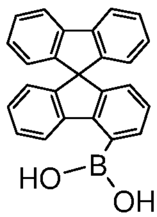 | 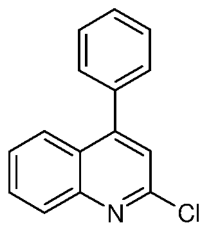 [5855-56-1] | 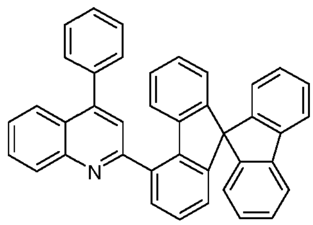 | 73% |
| 50e | 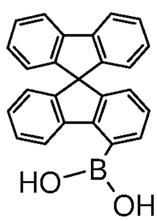 | 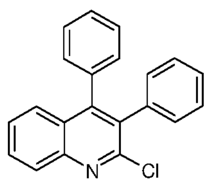 [19069-72-8] | 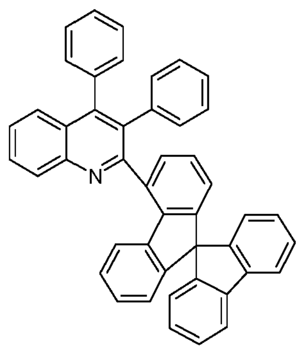 | 62% |

(fortgesetzt)

| | Edukt 1 | Edukt 2 | Produkt | Ausbeute |
|---|---|---|---|---|
| 51e | 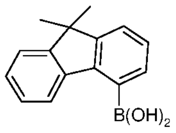 | 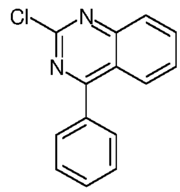 [29874-83-7] | 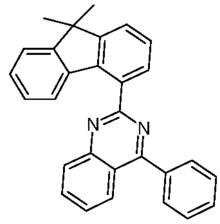 # | 75% |
| 52e | 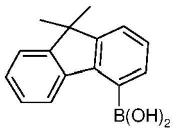 | 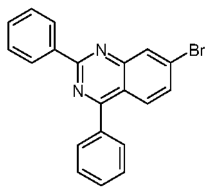 [1400697-34-8] | 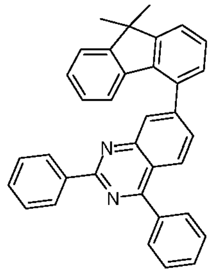 # | 68% |
| 53e | 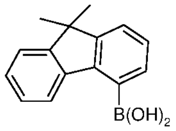 | 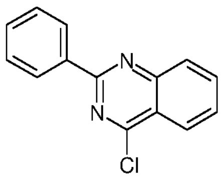 [6484-25-9] | 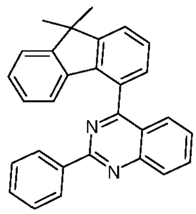 # | 60% |
| 54e | 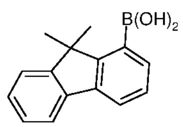 | 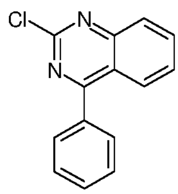 [29874-83-7] | 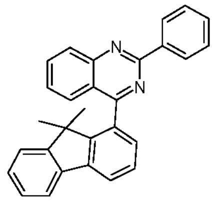 # | 61% |
| 55e | 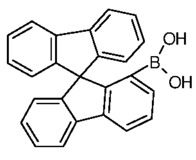 [1450933-18-2 ] | 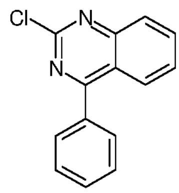 [29874-83-7] | 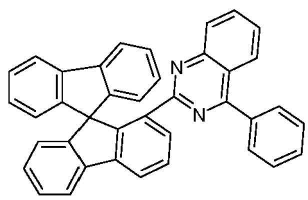 | 59% |

(fortgesetzt)

| | Edukt 1 | Edukt 2 | Produkt | Ausbeute |
|---|---|---|---|---|
| 56e | 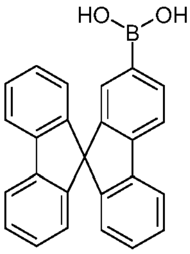 [236389-21-2 ] | 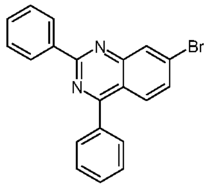 [1400697-34-8] | 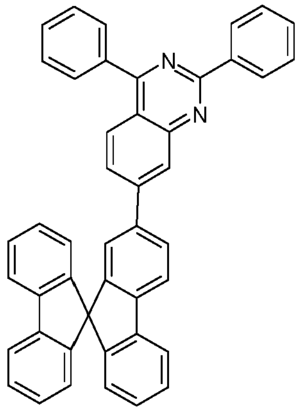 | 63% |
| 57e | 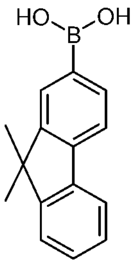 [333432-28-3 ] | 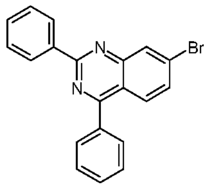 [1400697-34-8] | 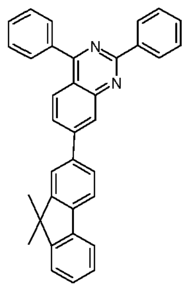 # | 65% |
| 58e | 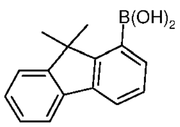 | 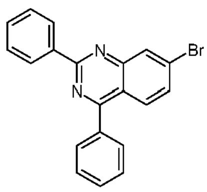 [1400697-34-8] | 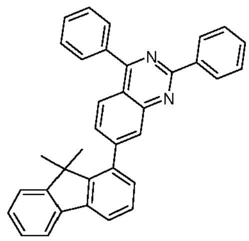 # | 62% |
| 59e | 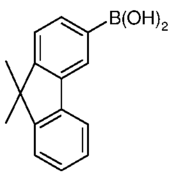 [1251773-34-8 ] | 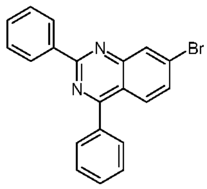 [1400697-34-8] | 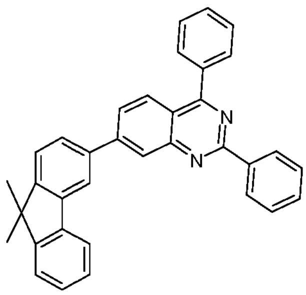 # | 60% |

(fortgesetzt)

| | Edukt 1 | Edukt 2 | Produkt | Ausbeute |
|---|---|---|---|---|
| 60e | 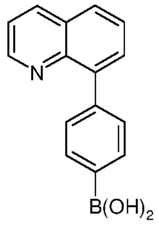 [1772611-33-2] | 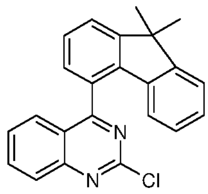 | 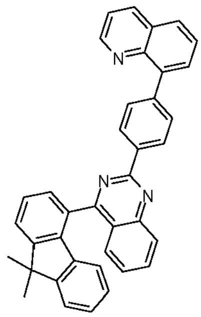 # | 64% |
| # nicht erfindungsgemäß | | | | |

**f) 2-(7-Bromo-9,9-dimethyl-9H-fluoren-4-yl)-4-phenyl-quinazolin**

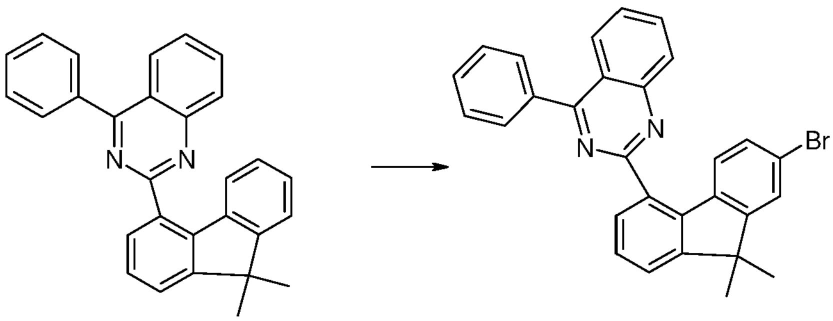

75,6 g (190.0 mmol) 2-(9,9-Dimethyl-9H-fluoren-4-yl)-4-phenyl-quinazolin weden 2000 mL Essigsäure(100%) und 2000 mL Schwefelsäure (95-98%) suspendiert. Zu dieser Suspension werden portionsweise 34 g (190 mmol) NBS zugegeben und 2 Stunden in Dunkelheit gerührt. Danach mit Wasser/Eis versetzt und Feststoff abgetrennt und mit Ethanol nachgewaschen. Der Rückstand wir im Toluol umkristallisiert. Die Ausbeute beträgt 68 g (142 mmol), entsprechend 76 % der Theorie. Analog dazu werden die folgenden Verbindungen hergestellt:

| | Edukt 1 | Produkt | Ausbeute |
|---|---|---|---|
| 1f | 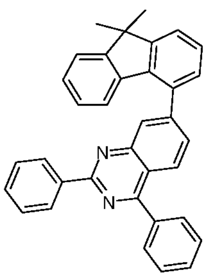 | 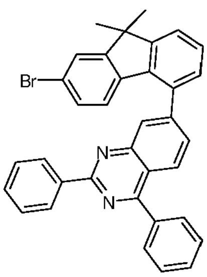 | 71%% |
| 2f | 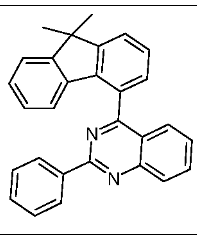 | 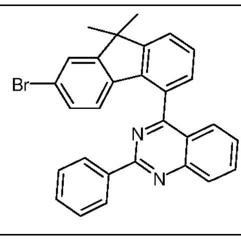 | 74% |

(fortgesetzt)

| | Edukt 1 | Produkt | Ausbeute |
|---|---|---|---|
| 36 | 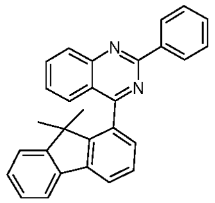 | 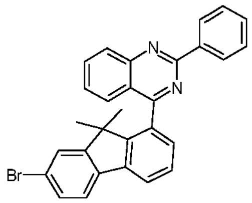 | 65% |

**g) 9,9-Dimethyl-5-(4-phenyl-quinazolin-2-yl)-9H-fluorene-2-boronsäure**

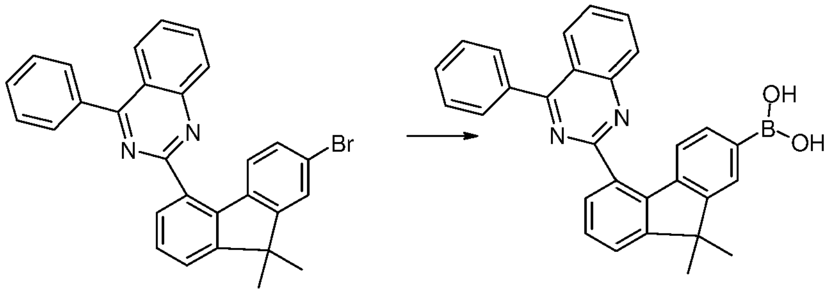

Eine auf -78°C gekühlte Lösung von 128 g (270 mmol) 4- [3-(7'-bromo-9,9'-spirobi[fluorene]-4'-yl)phenyl]-1-phenyl-benzimidazol in 1500 ml Diethylether wird tropfenweise mit 110 ml (276 mmol) n-Buthyllithium (2.5 M in Hexan) versetzt. Die Reaktionsmischung wird 30 min. bei -78°C gerührt. Man lässt auf Raumtemperatur kommen, kühlt erneut auf -78°C und versetzt dann schnell mit einer Mischung von 40 ml (351 mmol) Trimethylborat in 50 ml Diethylether. Nach Erwärmen auf -10°C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 126 g (241 mmol), 99% d. Th.; Reinheit: 90% nach HPLC.

Analog werden die folgenden Verbindungen erhalten:

| | Edukt 1 | Produkt | Ausbeute |
|---|---|---|---|
| 1g | 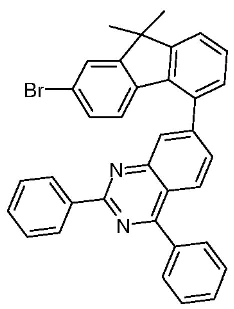 | 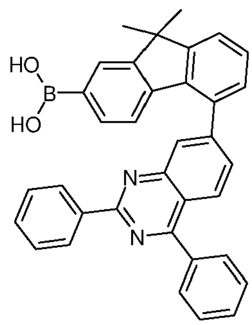 | 83% |

(fortgesetzt)

| | Edukt 1 | Produkt | Ausbeute |
|---|---|---|---|
| 2g | 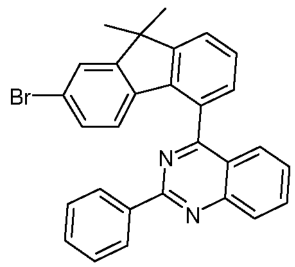 | 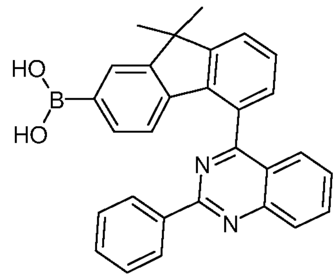 | 87% |
| 3g | 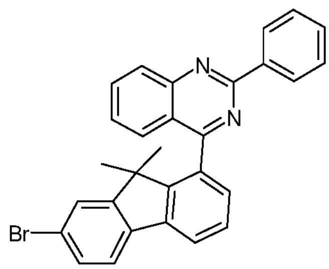 | 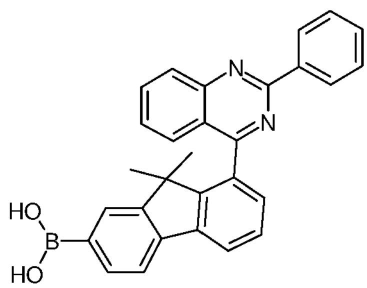 | 80% |

**h) 2-[7-(4,6-Diphenyl-[1,3,5]triazin-2-yl)-9,9-dimethyl-9H-fluoren-4-yl]-4-phenyl-quinazolin**

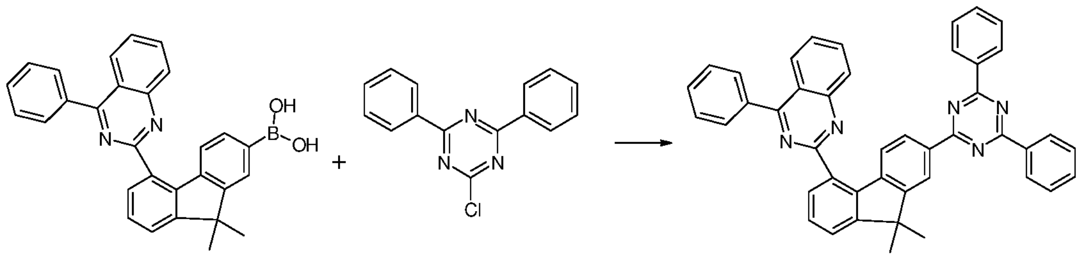

#

48g (110.0 mmol) 9,9-Dimethyl-5-(4-phenyl-quinazolin-2-yl)-9H-fluorene-2-boronsäure, 29.5 g (110.0 mmol) 2-Chlor-4,6-diphenyl-1,3,5-triazin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / iso-Propanol umkristallisiert und abschließend im Hochvakuum (p = 5 x $10^{-5}$ mbar, T = 350 °C) sublimiert. Die Ausbeute beträgt 58 g (93 mmol), entsprechend 86% der Theorie.

*# nicht erfindungsgemäß*

Analog dazu werden die folgenden Verbindungen hergestellt:

| | Edukt 1 | Edukt 2 | Produkt | Ausbeute |
|---|---|---|---|---|
| 1h | 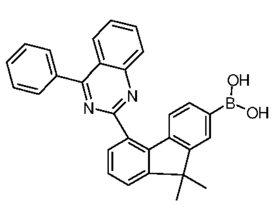 | 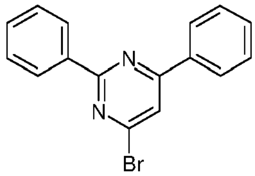 [40734-24-5] | 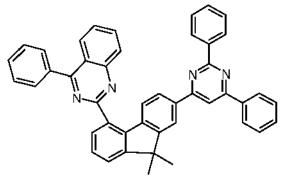 # | 73% |
| 2h | 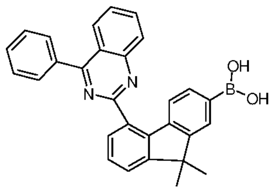 | 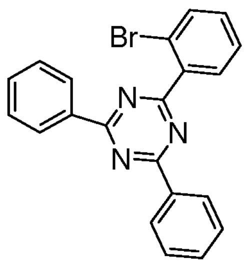 77989-15-2 | 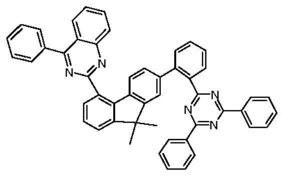 # | 82% |
| 3h | 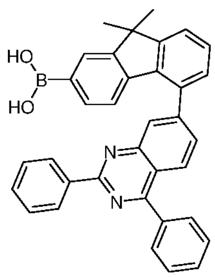 | 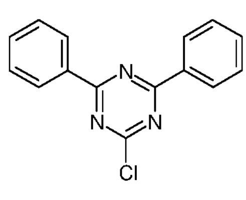 [3842-55-5] | 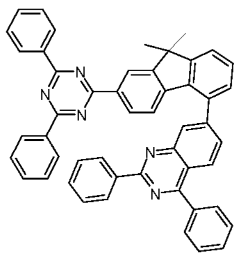 # | 73% |
| 4h | 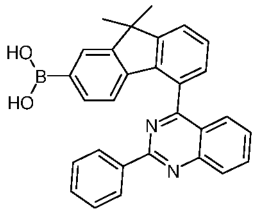 | 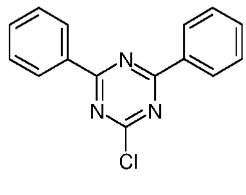 [3842-55-5] | 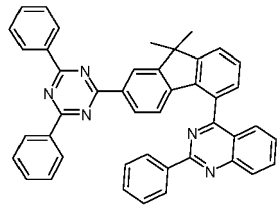 # | 70% |
| 5h | 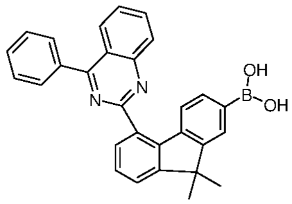 | 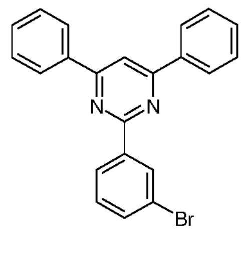 [864377-22-0] | 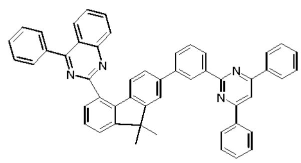 # | 72% |
| 6h | 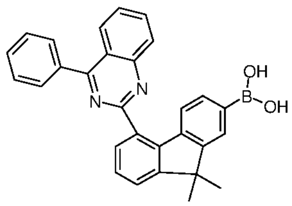 | 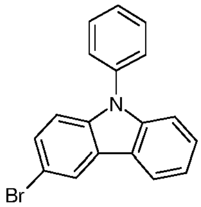 [1153-85-1] | 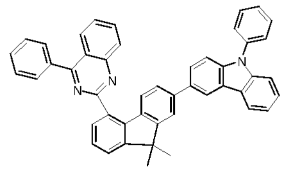 # | 70% |

(fortgesetzt)

| | Edukt 1 | Edukt 2 | Produkt | Ausbeute |
|---|---|---|---|---|
| 7h | 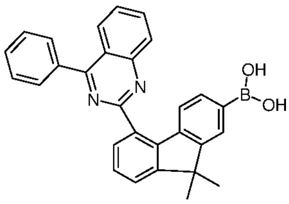 | 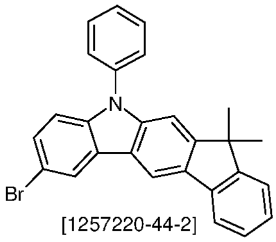 [1257220-44-2] | 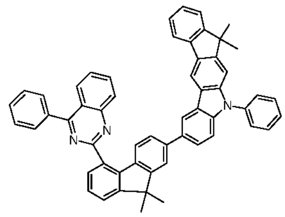 # | 69% |
| 8h | 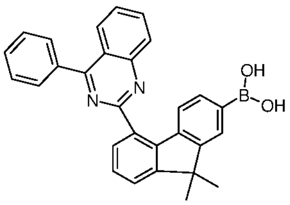 | 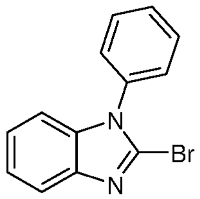 [1418123-78-0 ] | 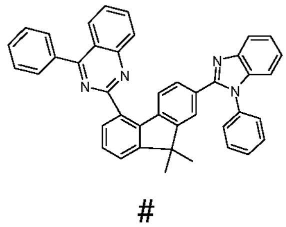 # | 68% |
| 9h | 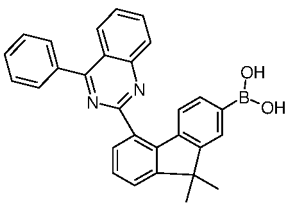 | 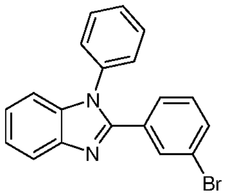 [760212-40-6 ] | 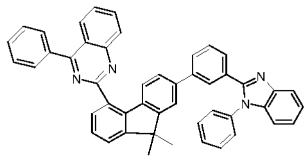 # | 70% |
| 10h | 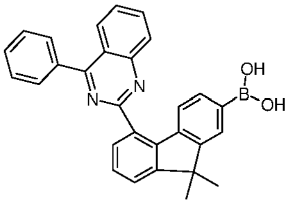 | 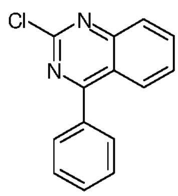 [29874-83-7] | 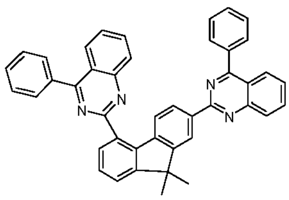 # | 71% |
| *# nicht erfindungsgemäß* | | | | |

**i) 4-[3-(7-bromo-9,9-spirobi[fluorene]-4-yl)phenyl]-1-phenyl-benzimidazol**

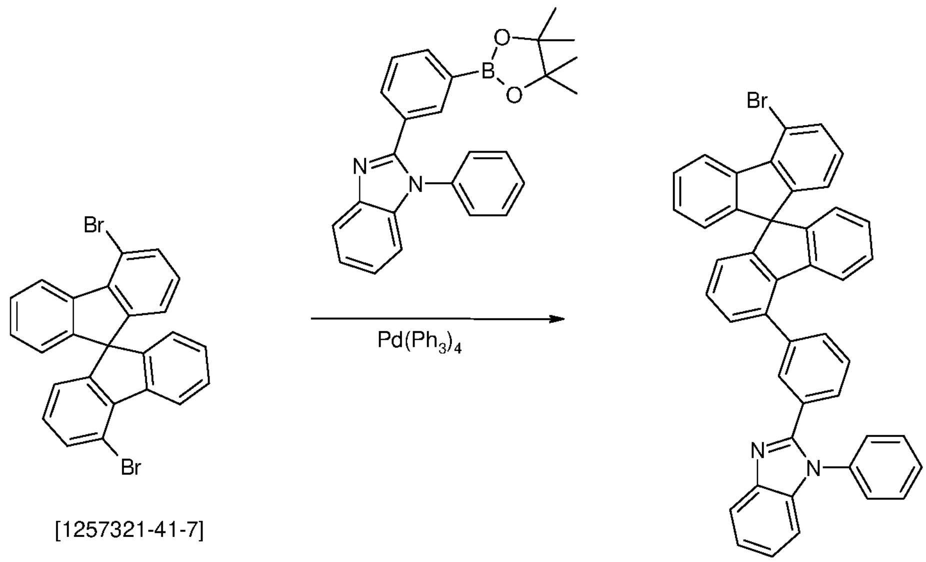

[1257321-41-7]

In einem 2L-Vierhalskolben werden unter Schutzgas 50.0g (105mmol, 1.00 eq) 4,4'-Dibromo-9,9'-spirobifluoren, 41.7g (105mmol, 1.00 eq) 1-Phenyl-2-[e-(4,4,5,5-tetramethyl-[1,3,2]dioxaborolan-y-yl)-phenyl]-1H-benzoimidazol, und 36.4g (263mmol, 2.50eq) Kaliumcarbonat in 400ml Toluol, 400ml 1,4-Dioxan und 200ml VE-Wasser vorgelegt und entgast. Anschließend wird 1.22g (1.05mmol, 0.01eq) Tetrakis(triphenylphosphin)-palladium(0) zugegeben und über Nacht am Rückfluss erhitzt. Nach beendeter Reaktion wird der Ansatz abgekühlt, über Celite filtriert und mit 1L Toluol verdünnt. Die Lösung wird 3x mit je 300ml halbgesättigter Natriumchloridlösung gewaschen und nach Trocknung über Natriumsulfat auf ca. 200ml am Rotationsverdampfer eingeengt. Der ausgefallene Feststoff wird abfiltriert und im Vakuum getrocknet. Die Abtrennung des disubstituierten Nebenproduktes erfolgt mittels Sublimation. Es werden 21.0g (32 mmol, 31%) des gewünschten Produktes erhalten.

Variante B:

Durchführung analog Variante A, anstelle von Tetrakis(triphenylphosphin)palladium(0) werden 0.01eq Palladium(II)-acetat und 0.01eq Dicyclohexyl-(2',6'-dimethoxy-biphenyl-2-yl-phophan (SPhos) verwendet.

Analog werden die folgenden Verbindungen erhalten:

| Nr. | Edukt 1 | Edukt 2 | Produkt 3 | Variante | Ausbeute |
|---|---|---|---|---|---|
| 1i | 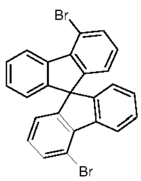 [1257321-41-7] | 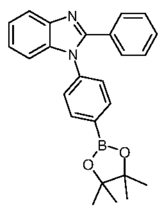 [1169709-19-6] | 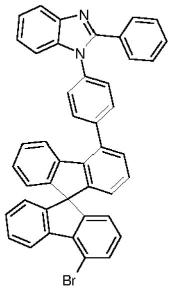 | B | 41% |
| 2i | 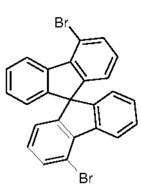 [1257321-41-7] | 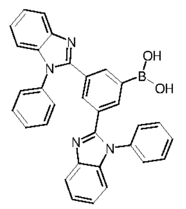 [1505512-90-2] | 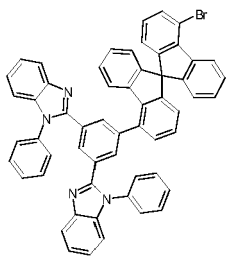 | B | 62% |

**j) 1-Phenyl-2-[3-[7'-(4,4,5,5-tetramethyl-1,3,2-dioxaborolan-4-yl)-9,9'-spirobi[fluorene]-4'-yl]phenyl]benzimidazol**

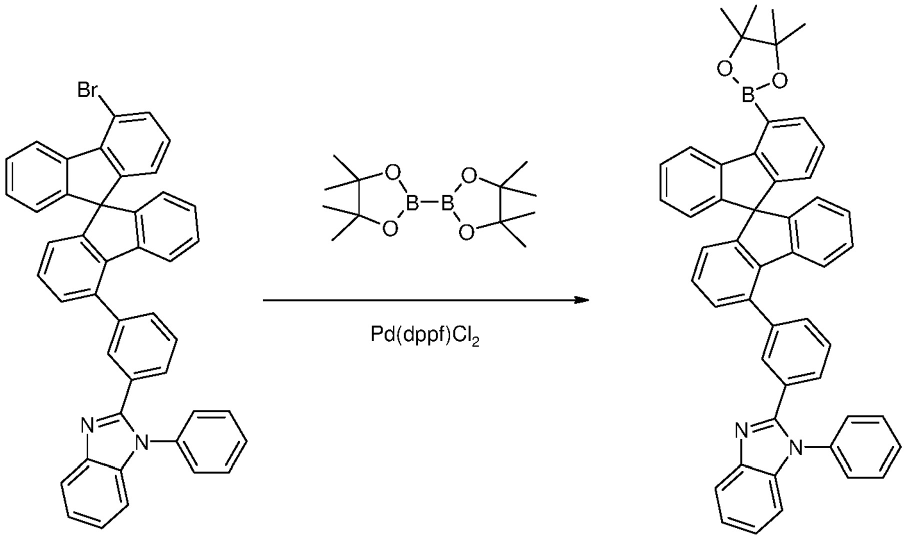


In einem 1L-Vierhalskolben werden 22.0g (33.1 mmol, 1.00eq) 2-[3-(7'-bromo-9,9'-spirobi[fluorene]-2'-yl)phenyl]-1-phenyl-benzimidazol, 8.84g (30.1 mmol, 0.91eq) Bis(pinacolato)diboron und 26.0g (265mmol, 8.00eq) Kaliumacetat in 500ml getrocknetem 1,4-Dioxan vorgelegt und für 30 Minuten entgast. Anschließend werden 812mg (0.995mmol, 0.0300eq) 1,1-Bis(diphenylphosphino)ferrocen-dichloropalladium(II)-Komplex mit DCM zugegeben und auf 80°C Innentemperatur erhitzt. Nach Rühren über Nacht wird der Ansatz abgekühlt und der ausgefallene Feststoff abgesaugt. Das Filtrat wird am Rotationsverdampfer auf ca. 50ml eingeengt und der ausgefallene Feststoff ebenfalls abgesaugt. Die Feststoffe werden vereint und getrocknet. Es werden 21.0g (29.5mmol, 89%) des Boronesters erhalten.

Analog werden die folgenden Verbindungen erhalten:

| Nr. | Edukt 3 | Produkt 4 | Ausbeute |
|---|---|---|---|
| 1j | 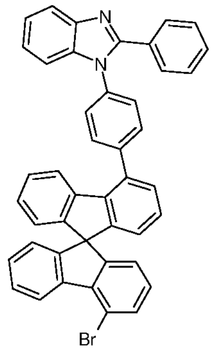 | 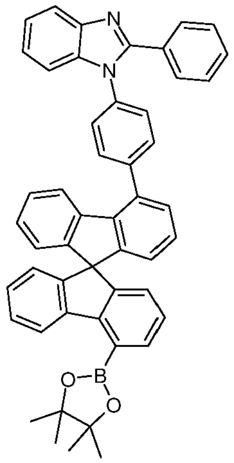 | 97% |

(fortgesetzt)

| Nr. | Edukt 3 | Produkt 4 | Ausbeute |
|---|---|---|---|
| 2j | 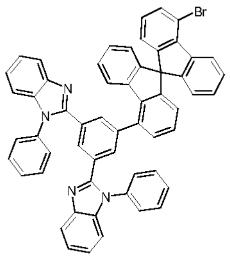 | 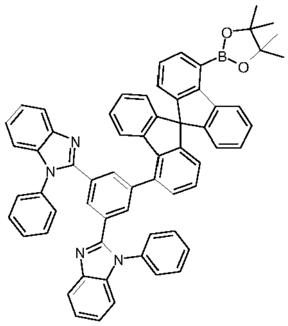 | 47% |

**k) 2-[3-[7'-(2-phenyl-quinazolin-4-yl)-9,9'-spirobi[fluorene]-4'-yl]phenyl]-1-phenyl-benzimidazole**

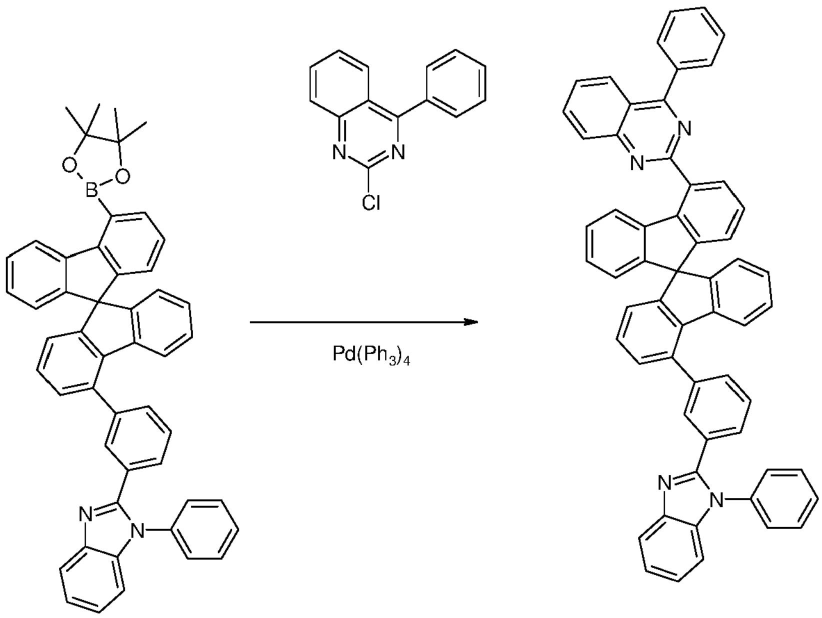


Variante A:

In einem 1L-Dreihalskolben werden 21.0g (29.5mmol, 1.00eq) 1-phenyl-2-[3-[7'-(4,4,5,5-tetramethyl-1,3,2-dioxaborolan-2-yl)-9,9'-spirobi[fluoren]-2'-yl]phenyl]benzimidazol und 7.2 g (29.5mmol, 1.00eq) 4--Chloro-2-phenyl-quinazolin zusammen mit 3.75g (35.4mmol, 1.20eq) Natriumcarbonat in 200ml Toluol, 200ml 1,4-Dioxan und 100ml VE-Wasser vorgelegt und für 20 Minuten entgast. Nach Zugabe von 1.02g (0.885mmol, 0.0300eq) Tetrakis(triphenylphosphin)palladium(0) wird der Ansatz für 2 Tage unter Rückfluss erhitzt und nach beendeter Reaktion abgekühlt. Der ausgefallene Feststoff wird abgesaugt, mit Wasser und etwas Toluol gewaschen und anschließend mehrfach aus Toluol/Heptan umkristallisert bis eine HPLC-Reinheit von >99.9% erreicht wird. Nach Sublimation werden 11.5g (14.0mmol, 46%) eines farblosen Feststoffs erhalten.

Variante B:

Durchführung analog Variante A, anstelle von Tetrakis(triphenylphosphin)palladium(0) werden 0.01eq Palladium(II)-acetat und 0.04eq Tri(o-Tolyl)phosphin verwendet.

Variante C:

Durchführung analog Variante A, anstelle von Tetrakis(triphenylphosphin)palladium(0) werden 0.01eq Palladium(II)-acetat und 0.01eq Dicyclohexyl-(2',6'-dimethoxy-biphenyl-2-yl-phophan (SPhos) verwendet.

Analog wurden hergestellt:

| Nr. | Edukt 4 | Edukt 5 | Produkt 6 | Variante | Ausbeute |
|---|---|---|---|---|---|
| **6k** | 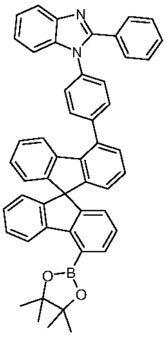 | 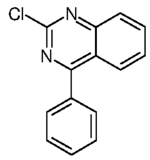 [29874-83-7] | 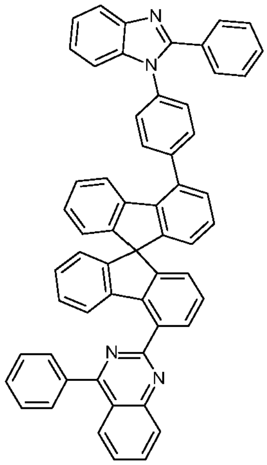 | B | 62% |
| **7k** | 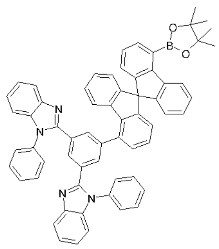 | 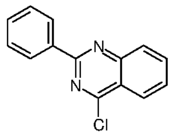 [6484-25-9] | 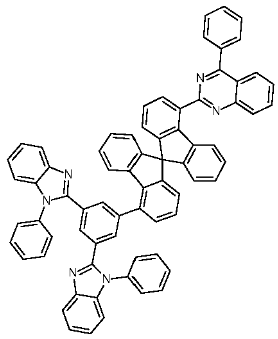 | A | 41% |
| **8k** | 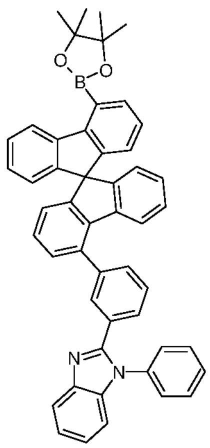 | 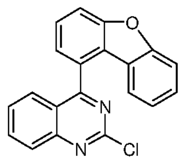 | 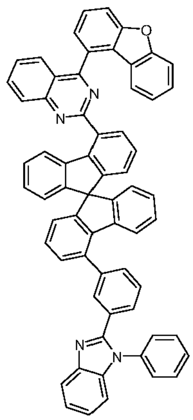 | A | 40% |

(fortgesetzt)

| Nr. | Edukt 4 | Edukt 5 | Produkt 6 | Variante | Ausbeute |
|---|---|---|---|---|---|
| **9k** | 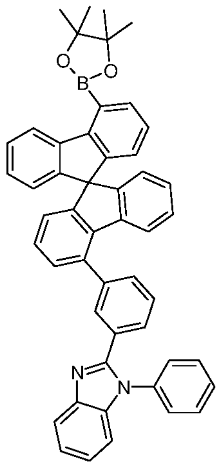 | 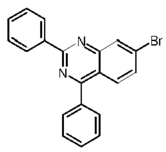 [1400697-34-8] | 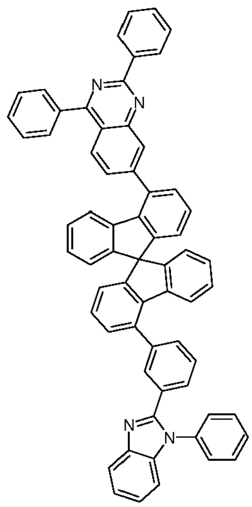 | B | 57% |
| **10k** | 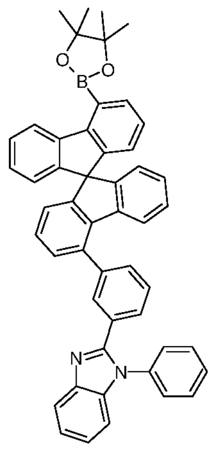 | 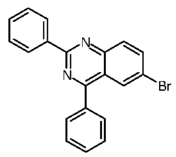 [1229609-99-7] | 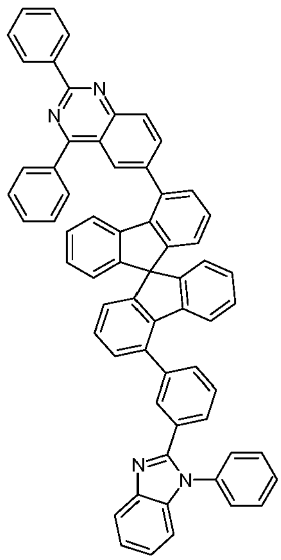 | A | 61% |

**Herstellung der OLEDs**

In den folgenden Beispielen V1 bis E14 (siehe Tabellen 1 und 2) werden die Daten verschiedener OLEDs vorgestellt.

**Vorbehandlung für die Beispiele V1-E14:** Glasplättchen, die mit strukturiertem ITO (Indium Zinn Oxid) der Dicke 50 nm beschichtet sind, bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1 :IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die

Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$.

Die Daten der verschiedenen OLEDs sind in Tabelle 2 zusammengefasst. Die Beispiele V1- V4 sind Vergleichsbeispiele gemäß dem Stand der Technik, die Beispiele E1-E14 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Materialien in phosphoreszenten OLEDs**

Die erfindungsgemäßen Materialien ergeben bei Einsatz als Lochblockierschicht (HBL) in phosphoreszierenden OLEDs eine wesentliche Verbesserung der Effizienz gegenüber dem Stand der Technik Durch Einsatz der erfindungsgemäßen Verbindungen 24e, 52e und 58e lässt sich eine Erhöhung der externen Quanteneffizienz um ca. 10% gegenüber dem Stand der Technik SdT1, SdT2 und SdT3 beobachten. Auch bei Verwendung der erfindungsgemäßen Materialien in der Elektronentransportschicht (ETL), lässt sich eine verbesserte externe Quanteneffizienz gegenüber dem Stand der Technik erzielen (Vergleich Beispiel V4 mit Beispiel E4).

Tabelle 1: Aufbau der OLEDs

| Bsp | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 110nm | IC1:TEG1 (85%:15%) 30nm | SdT1 10nm | ST2:LiQ (50%:50%) 30nm | --- |
| V2 | SpA1 70nm | HATCN 5nm | SpMA1 110nm | IC1:IC3:TEG1 (50%:45%:5%) 30nm | SdT2 10nm | ST2:LiQ (50%:50%) 30nm | --- |
| V3 | SpA1 70nm | HATCN 5nm | SpMA1 110nm | IC1:TEG1 (85%:15%) 30nm | SdT3 10nm | ST2:LiQ (50%:50%) 30nm | --- |
| V4 | SpA1 70nm | HATCN 5nm | SpMA1 110nm | IC1 :IC3:TEG1 (50%:45%:5%) 30nm | - | ST2:SdT4 (40%:60%) 40nm | LiQ 3nm |
| E1 | SpA1 70nm | HATCN 5nm | SpMA1 110nm | IC1:TEG1 (85%:15%) 30nm | 24e 10nm | ST2:LiQ (50%:50%) 30nm | --- |
| E2 # | SpA1 70nm | HATCN 5nm | SpMA1 110nm | IC1:IC3:TEG1 (50%:45%:5%) 30nm | 52e 10nm | ST2:LiQ (50%:50%) 30nm | --- |
| E3 # | SpA1 70nm | HATCN 5nm | SpMA1 110nm | IC1:TEG1 (85%:15%) 30nm | 58e 10nm | ST2:LiQ (50%:50%) 30nm | --- |
| E4 # | SpA1 70nm | HATCN 5nm | SpMA1 110nm | IC1 :IC3:TEG1 (50%:45%:5%) 30nm | --- | ST2: 60e (40%:60%) 40nm | LiQ 3nm |
| E5 # | SpMA1 140nm | --- | --- | 16e:TER4 (95%:5%) 40nm | --- | ST2:LiQ (50%:50%) 35nm | --- |

(fortgesetzt)

| Bsp | HTL Dicke | IL Dicke | EBL Dicke | EML Dicke | HBL Dicke | ETL Dicke | EIL Dicke |
|---|---|---|---|---|---|---|---|
| E6 | SpMA1 140nm | --- | --- | IC1:TER4 (95%:5%) 40nm | 19e 10nm | ST2:LiQ (50%:50%) 25nm | --- |
| E7 | SpMA1 140nm | --- | --- | 22e:TER4 (97%:3%) 40nm | --- | ST2:LiQ (50%:50%) 35nm | --- |
| E8 | SpA1 70nm | HATCN 5nm | SpMA1 110nm | IC1:TEG1 (90%:10%) 30nm | 10k 10nm | ST2:LiQ (50%:50%) 30nm | --- |
| E9 # | SpMA1 140nm | --- | --- | 41e:SpMA1: TER4 (55%:22%:3%) 40nm | --- | ST2:LiQ (50%:50%) 35nm | --- |
| E10 # | SpMA1 140nm | --- | --- | 43e:TER4 (95%:5%) 40nm | IC1 10nm | ST2:LiQ (50%:50%) 25nm | --- |
| E11 # | SpA1 70nm | HATCN 5nm | SpMA1 110nm | IC1:TEG1 (90%:10%) 30nm | IC1 10nm | ST2:44e (40%:60%) 40nm | LiQ 3nm |
| E12 | SpMA1 140nm | --- | --- | IC1:TER4 (95%:5%) 40nm | 48e 10nm | ST2:LiQ (50%:50%) 25nm | --- |
| E13 # | SpA1 70nm | HATCN 5nm | SpMA1 110nm | IC1:TEG1 (90%:10%) 30nm | --- | 1 h:LiQ (50%:50%) 30nm | --- |
| E14 # | SpMA1 140nm | --- | --- | 7h:TEG1:TER4 (85%:10%:5%) 40nm | | ST2:LiQ (50%:50%) 35nm | --- |
| *# nicht erfindungsgemäß* | | | | | | | |

Tabelle 2: Messeraebnisse der verschiedenen OLEDs

| Bsp. | U1000 (V) | SE1000 (cd/A) | LE1000 (lm/W) | EQE 1000 | CIE x/y bei 1000 cd/m$^2$ |
|---|---|---|---|---|---|
| V1 | 3.6 | 55 | 48 | 15.2% | 0.31/0.64 |
| V2 | 3.4 | 55 | 51 | 14.9% | 0.34/0.63 |
| V3 | 3.5 | 56 | 50 | 15.5% | 0.31/0.64 |
| V4 | 3.7 | 52 | 44 | 14.1% | 0.34/0.62 |
| E1 | 3.6 | 64 | 56 | 17.2% | 0.33/0.63 |
| E2 # | 3.3 | 59 | 56 | 16.2% | 0.32/0.64 |
| E3 # | 3.5 | 63 | 57 | 17.1% | 0.33/0.63 |
| E4 # | 3.7 | 56 | 48 | 15.3% | 0.33/0.63 |
| E5 # | 4.6 | 17 | 12 | 14.6% | 0.66/0.34 |
| E6 | 4.9 | 16 | 10 | 14.4% | 0.67/0.33 |
| E7 | 4.7 | 16 | 11 | 14.9% | 0.67/0.33 |

(fortgesetzt)

| Bsp. | U1000 (V) | SE1000 (cd/A) | LE1000 (lm/W) | EQE 1000 | CIE x/y bei 1000 cd/m$^2$ |
|---|---|---|---|---|---|
| E8 | 3.5 | 64 | 57 | 17.4% | 0.32/0.64 |
| E9 # | 4.5 | 16 | 11 | 15.3% | 0.67/0.33 |
| E10 # | 4.6 | 17 | 12 | 15.1% | 0.66/0.34 |
| E11 # | 3.6 | 58 | 51 | 15.8% | 0.31/0.64 |
| E12 | 5.0 | 15 | 9 | 14.8% | 0.67/0.33 |
| E13 # | 3.7 | 63 | 53 | 17.2% | 0.31/0.64 |
| E14 # | 4.4 | 18 | 13 | 16.5% | 0.67/0.33 |
| *# nicht erfindungsgemäß* | | | | | |

Tabelle 3: Strukturformeln der Materialien für die OLEDs

| | |
|---|---|
| 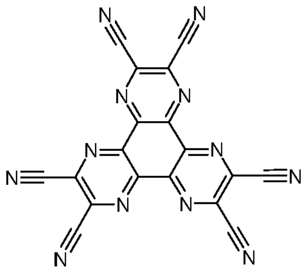 | 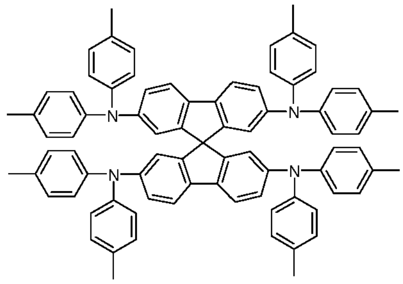 |
| HATCN | SpA1 |
| 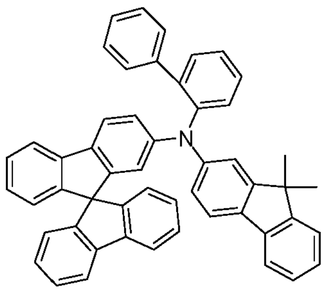 | 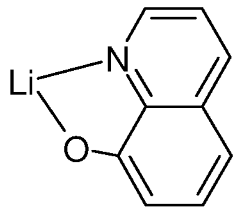 |
| SpMA1 | LiQ |
| 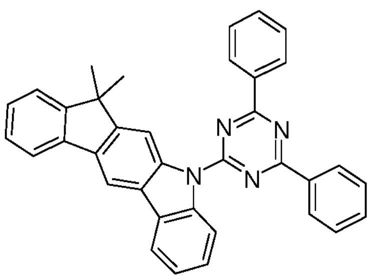 | 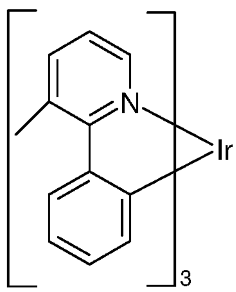 |
| IC1 | TEG1 |

(fortgesetzt)

| | |
|---|---|
| 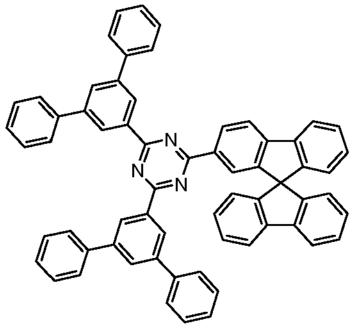 | 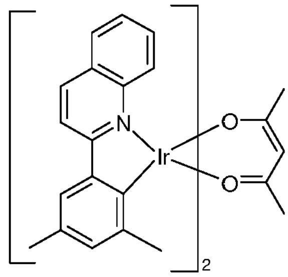 |
| ST2 | TER4 |
| 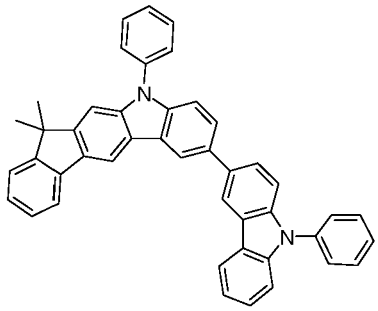 | |
| IC3 | |
| 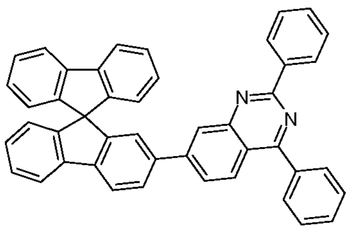 | 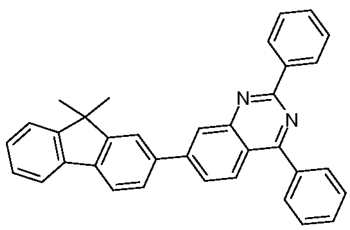 |
| SdT1 | SdT2 |
| 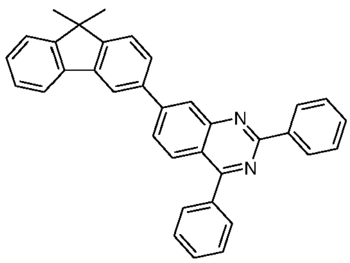 | 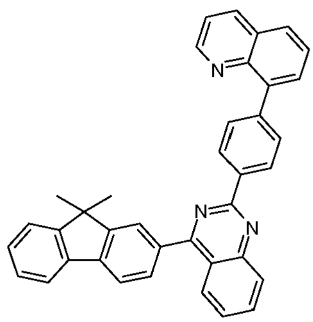 |
| SdT3 | SdT4 |

(fortgesetzt)

| | |
|---|---|
| 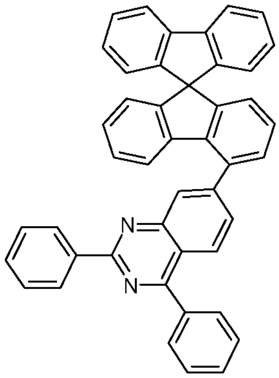 | 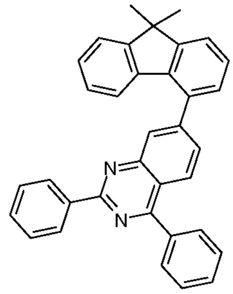 |
| 24e | 52e # |
| 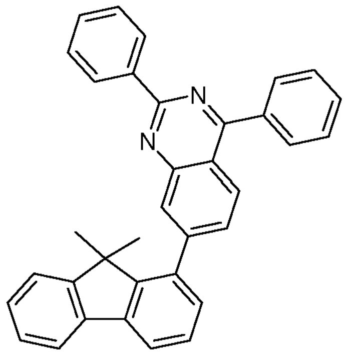 | 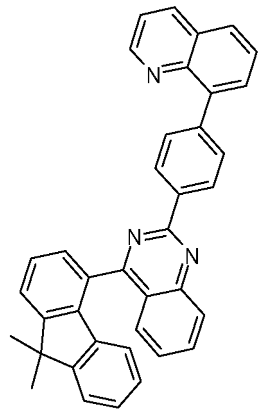 |
| 58e # | 60e # |
| 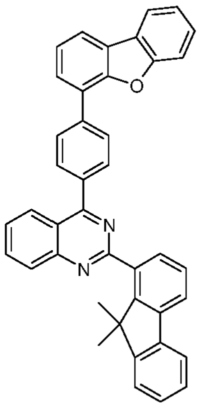 | 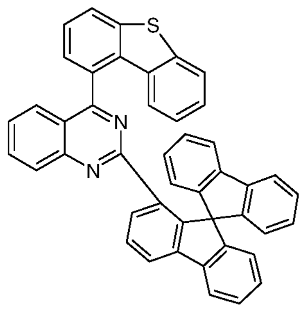 |
| 16e # | 19e |
| 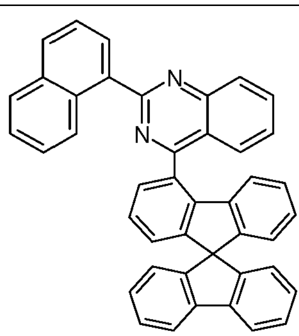 | 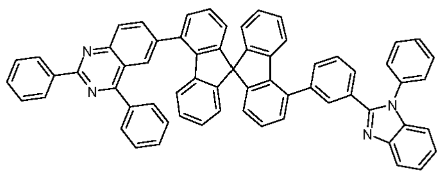 |

(fortgesetzt)

| 22e | 10k |
|---|---|
| 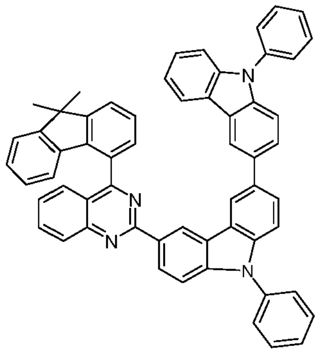 | 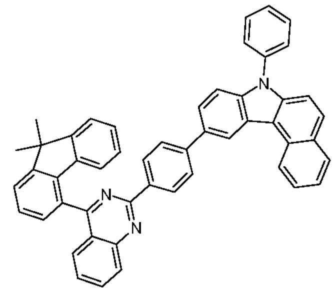 |
| 41e # | 43e # |
| 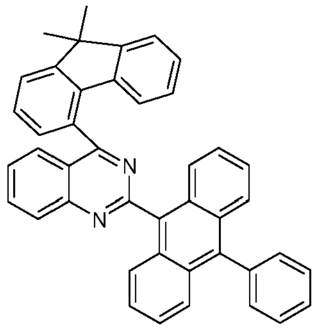 | 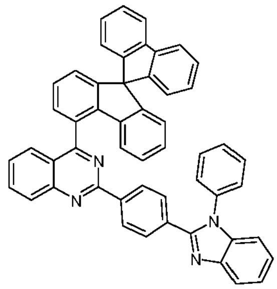 |
| 44e # | 48e |
| 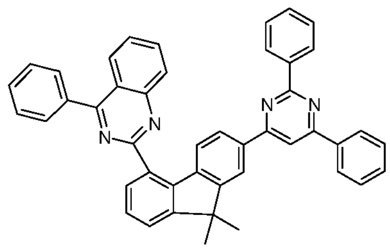 | 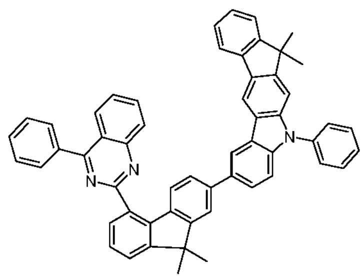 |
| 1h # | 7h # |

**Patentansprüche**

**1.** Verbindung, umfassend Strukturen der Formel (III) und/oder Formel (IV)

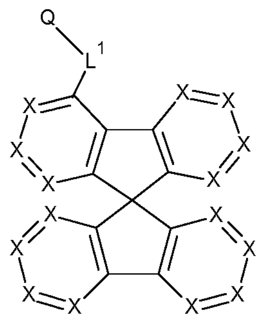

Formel (III)

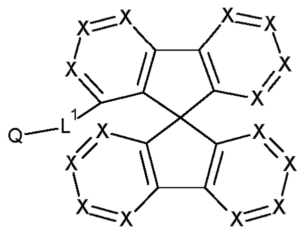

Formel (IV)

wobei für die verwendeten Symbole gilt:

X ist bei jedem Auftreten gleich oder verschieden N oder $CR^1$, vorzugsweise $CR^1$, mit der Maßgabe, dass nicht mehr als zwei der Gruppen X in einem Cyclus für N stehen;

Q ist eine Elektronentransportgruppe, die ausgewählt ist aus Strukturen der Formeln (Q-1), (Q-2) und/oder (Q-3)

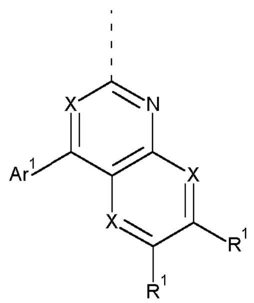

Formel (Q-1)

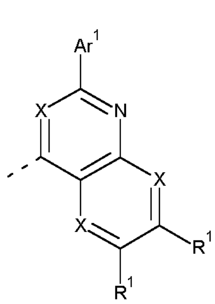

Formel (Q-2)

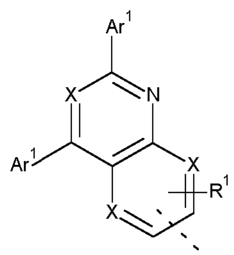

Formel (Q-3)

wobei die gestrichelte Bindung die Anbindungsposition markiert und $Ar^1$ ein aromatisches oder heteroaromatisches Ringsystem mit 6 bis 40 C-Atomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^1$ bzw. $R^2$ ein mono- oder polycyclisches, aliphatisches Ringsystem bilden können, das mit einem oder mehreren Resten $R^3$ substituiert sein kann;

$L^1$ ist eine Bindung, C(=O) oder ein aromatisches Ringsystem mit 5 bis 24 aromatischen Ringatomen, welches durch einen oder mehrere Reste $R^1$ substituiert sein kann;
$R^1$ ist bei jedem Auftreten gleich oder verschieden H, D, eine geradkettige Alkyl-, Alkoxy- oder Thioalkoxygruppe mit 1 bis 40 C-Atomen oder eine verzweigte oder cyclische Alkyl-, Alkoxy-oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch-$R^2C=CR^2$-, -C≡C-, $Si(R^2)_2$, C=O, C=S, $C=NR^2$, $-C(=O)O-$, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy-oder Heteroaryloxygruppe mit 5 bis 40 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;
$R^2$ ist bei jedem Auftreten gleich oder verschieden H, D, eine geradkettige Alkyl-, Alkoxy- oder Thioalkoxygruppe mit 1 bis 40 C-Atomen oder eine verzweigte oder cyclische Alkyl-, Alkoxy-oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch-$R^3C=CR^3$-, -C≡C-, $Si(R^3)_2$, C=O, C=S, $C=NR^3$, $-C(=O)O-$, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy-oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;
$R^3$ ist bei jedem Auftreten gleich oder verschieden H, D, F oder ein aliphatischer Kohlenwasserstoffrest mit 1 bis 20 C-Atomen, in dem ein oder mehrere H-Atome durch D oder F ersetzt sein können, oder ein aromatisches und/oder heteroaromatisches Ringsystem mit 5 bis 30 Kohlenstoffatomen, in dem ein oder mehrere H-Atome durch D oder F ersetzt sein können; dabei können zwei oder mehrere benachbarte Substituenten $R^3$ auch miteinander ein mono- oder polycyclisches, aliphatisches oder aromatisches Ringsystem bilden.

2. Verbindung gemäß Anspruch 1, **dadurch gekennzeichnet, dass** die Verbindung mindestens eine der Strukturen der Formeln (IIIa) und/oder (IVa) umfasst

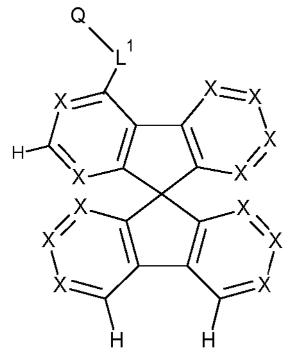

Formel (IIIa)

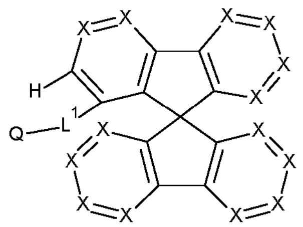

Formel (IVa)

worin die Symbole X, $R^1$, $L^1$ und Q die in Anspruch 1 oder 2 dargelegte Bedeutung haben.

3. Verbindungen gemäß Anspruch 1 oder 2, **dadurch gekennzeichnet, dass** in Formeln (III), (IIIa), (IV) und (IVa) nicht mehr als zwei, vorzugsweise nicht mehr als eine Gruppe X für N steht, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist.

4. Verbindungen gemäß einem oder mehreren der Ansprüche 1-3, **dadurch gekennzeichnet, dass** die Verbindung mindestens eine der Strukturen der Formeln (IIIb) und/oder (IVb) umfasst

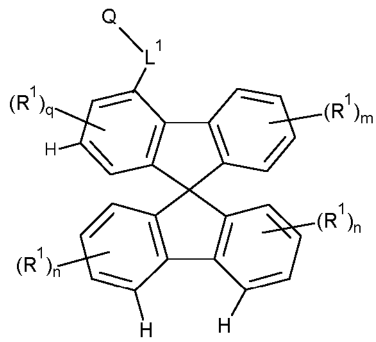

Formel (IIIb)

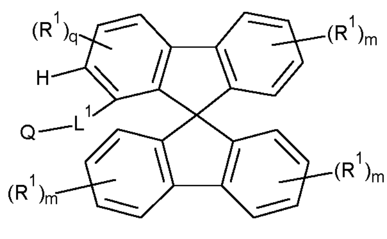

Formel (IVb)

wobei die Symbole Q, $L^1$ und $R^1$ die in Anspruch 1 genannte Bedeutung aufweisen, m 0, 1, 2, 3 oder 4, vorzugsweise 0, 1 oder 2, n 0, 1, 2 oder 3, vorzugsweise 0, 1 oder 2 und q 0, 1 oder 2, vorzugsweise 0 oder 1 ist.

5. Verbindungen gemäß einem oder mehreren der Ansprüche 1-4, **dadurch gekennzeichnet, dass** die Gruppe Q ein heteroaromatisches Ringsystem mit mindestens zwei kondensierten Ringen ist, welches durch einen oder mehrere Reste $R^1$ substituiert sein kann, wobei die Ringatome der mindestens zwei kondensierten Ringe mindestens ein Stickstoffatom umfassen.

6. Verbindungen gemäß einem oder mehreren der Ansprüche 1-5, **dadurch gekennzeichnet, dass** die Gruppe Q ein heteroaromatisches Ringsystem mit 9 bis 14, vorzugsweise 10 Ringatomen darstellt, welches durch einen oder mehrere Reste $R^1$ substituiert sein kann.

7. Verbindungen gemäß einem oder mehreren der Ansprüche 1-6, **dadurch gekennzeichnet, dass** die Gruppe Q ausgewählt ist aus Strukturen der Formeln (Q-4), (Q-5), (Q-6), (Q-7), (Q-8), (Q-9), (Q-10), (Q-11), (Q-12), (Q-13), (Q-14) und/oder (Q-15)

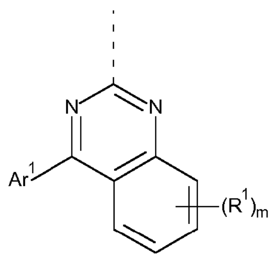

Formel (Q-4)

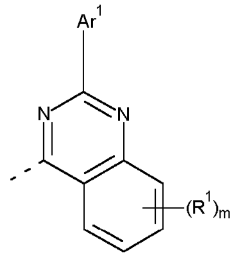

Formel (Q-5)

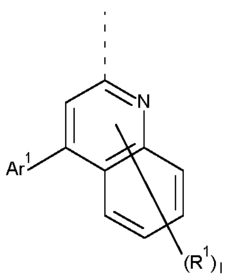

Formel (Q-6)

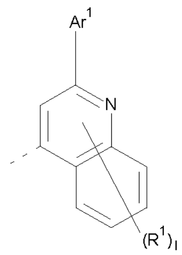

Formel (Q-7)

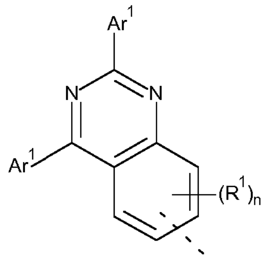

Formel (Q-8)

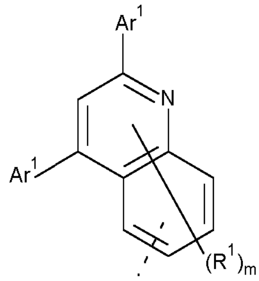

Formel (Q-9)

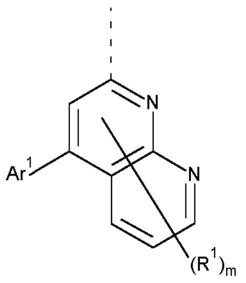

Formel (Q-10)

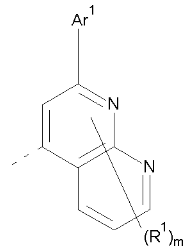

Formel (Q-11)

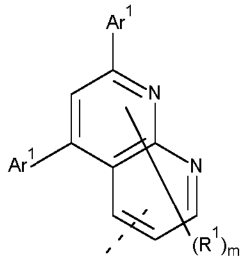

Formel (Q-12)

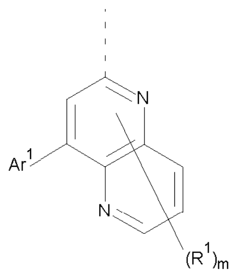

Formel (Q-13)

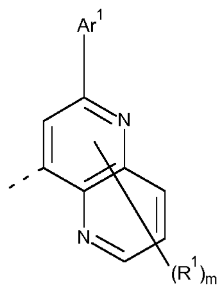

Formel (Q-14)

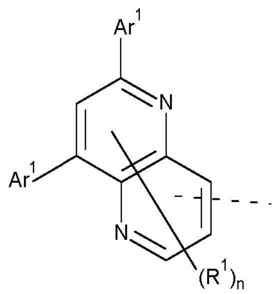

Formel (Q-15)

worin die Symbole $Ar^1$ und $R^1$ die in Anspruch 8 dargelegt Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1 und l 1, 2, 3, 4 oder 5, vorzugsweise 0, 1 oder 2 ist.

**8.** Verbindungen gemäß mindestens einem der vorhergehenden Anspruch 6 oder 7, **dadurch gekennzeichnet, dass** $Ar^1$ und/oder ein an $Ar^1$ gebundener Substituent $R^2$ gemäß den Formeln (Q-1) bis (Q-15) ein Strukturelement umfasst, welches ausgewählt ist aus Strukturen gemäß Formeln Q1 bis Q15 und/oder aus Strukturen gemäß Formel Q16 oder Q17

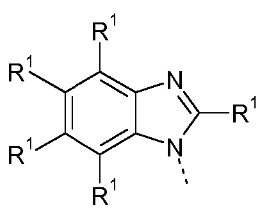

Formel (Q-16)

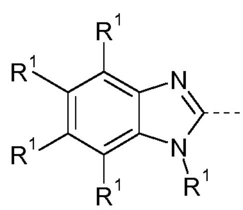

Formel (Q-17)

wobei das Symbol $R^1$ die in Anspruch 1 genannte Bedeutung aufweist und die gestrichelten Bindungen jeweils die

Anbindungspositionen markieren, an die das Strukturelement gemäß Formel (Q-16) oder (Q-17) an andere Strukturelemente des Rests $Ar^1$ oder an den Substituent $R^2$ oder an eine Struktur gemäß Formeln (Q-1) bis (Q-15) binden.

9. Verbindung gemäß einem oder mehreren der Ansprüche 1-8, **dadurch gekennzeichnet, dass** die Verbindung keine Carbazol- und/oder Triarylamin-Gruppe umfasst.

10. Verbindung gemäß einem oder mehreren der Ansprüche 1-9, **dadurch gekennzeichnet, dass** die Verbindung keine lochtransportierende Gruppe umfasst.

11. Oligomer, Polymer oder Dendrimer enthaltend eine oder mehrere Verbindungen gemäß einem oder mehreren der Ansprüche 1-10, wobei ein oder mehrere Bindungen der Verbindung zum Polymer, Oligomer oder Dendrimer vorhanden sind.

12. Zusammensetzung enthaltend wenigstens eine Verbindung gemäß einem oder mehreren der Ansprüche 1-10 und/oder ein Oligomer, Polymer oder Dendrimer gemaäß Anspruch 11 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

13. Formulierung enthaltend mindestens eine Verbindung gemäß einem oder mehreren der Ansprüche 1-10, ein Oligomer, Polymer oder Dendrimer gemäß Anspruch 11 und/oder wenigstens eine Zusammensetzung gemäß Anspruch 12 und mindestens ein Lösungsmittel.

14. Verfahren zur Herstellung einer Verbindung gemäß einem oder mehreren der Ansprüche 1-10 oder eines Oligomers, Polymers oder Dendrimers gemäß Anspruch 11, **dadurch gekennzeichnet, dass** in einer Kupplungsreaktion eine Verbindung, umfassend mindestens eine elektronentransportierende Gruppe, mit einer Verbindung, umfassend mindestens einen Fluoren-Rest, umgesetzt wird.

15. Verwendung einer Verbindung gemäß einem oder mehreren der Ansprüche 1-10, eines Oligomers, Polymers oder Dendrimers gemäß Anspruch 11 oder einer Zusammensetzung gemäß Anspruch 12 in einer elektronischen Vorrichtung als Lochblockiermaterial, Elektroneninjektionsmaterial und/oder Elektronentransportmaterial.

16. Elektronische Vorrichtung, enthaltend mindestens eine Verbindung gemäß einem oder mehreren der Ansprüche 1-10, ein Oligomer, Polymer oder Dendrimer gemäß Anspruch 11 oder eine Zusammensetzung gemäß Anspruch 12, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laser-dioden.

## Claims

1. Compound comprising structures of the formula (III) and/or formula (IV)

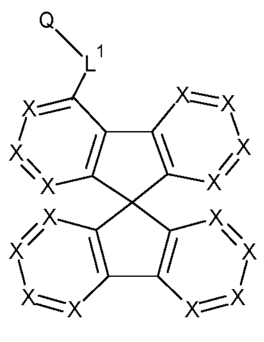

formula (III)

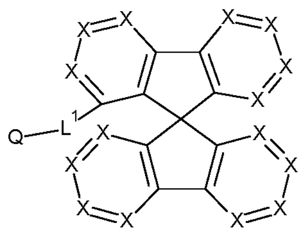

formula (IV)

where the following applies to the symbols used:

X is on each occurrence, identically or differently, N or $CR^1$, preferably $CR^1$, with the proviso that not more than two of the groups X in a ring stand for N;
Q is an electron-transport group selected from structures of the formulae (Q-1), (Q-2) and/or (Q-3)

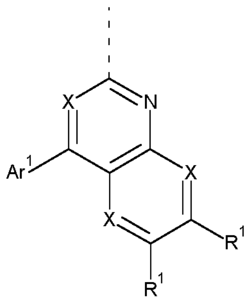

formula (Q-1)

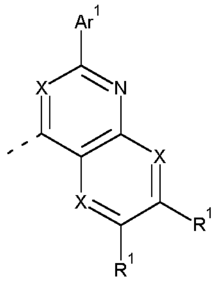

formula (Q-2)

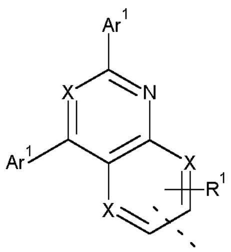

formula (Q-3)

where the dashed bond marks the bonding position and $Ar^1$ represents an aromatic or heteroaromatic ring system having 6 to 40 C atoms, which may in each case be substituted by one or more radicals $R^2$, an aryloxy group having 5 to 60 aromatic ring atoms, which may be substituted by one or more radicals $R^2$, or an aralkyl group having 5 to 60 aromatic ring atoms, which may in each case be substituted by one or more radicals $R^2$, where two or more adjacent substituents $R^1$ or $R^2$ may optionally form a mono- or polycyclic, aliphatic ring system, which may be

substituted by one or more radicals $R^3$;

$L^1$ is a bond, C(=O) or an aromatic ring system having 5 to 24 aromatic ring atoms, which may be substituted by one or more radicals $R^1$;

$R^1$ is on each occurrence, identically or differently, H, D, a straightchain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by-$R^2C=CR^2$-, -C=C-, $Si(R^2)_2$, C=O, C=S, $C=NR^2$, -C(=O)O-, -C(=O)$NR^2$-, $NR^2$, P(=O)($R^2$), -O-, -S-, SO or $SO_2$ and where one or more H atoms may be replaced by D, F, Cl, Br, I, CN or $NO_2$, or an aromatic or heteroaromatic ring systems having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or a combination of these systems; two or more adjacent substituents $R^1$ may also form a mono- or polycyclic, aliphatic or aromatic ring system with one another;

$R^2$ is on each occurrence, identically or differently, H, D, a straightchain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by -$R^3C=CR^3$-, -C≡C-, $Si(R^3)_2$, C=O, C=S, $C=NR^3$, -C(=O)O-, -C(=O)$NR^3$-, $NR^3$, P(=O)($R^3$), -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more adjacent substituents $R^2$ may also form a mono- or polycyclic, aliphatic or aromatic ring system with one another;

$R^3$ is on each occurrence, identically or differently, H, D, F or an aliphatic hydrocarbon radical having 1 to 20 C atoms, in which one or more H atoms may be replaced by D or F, or an aromatic and/or heteroaromatic ring system having 5 to 30 carbon atoms, in which one or more H atoms may be replaced by D or F; two or more adjacent substituents $R^3$ may also form a mono- or polycyclic, aliphatic or aromatic ring system with one another.

2. Compound according to Claim 1, **characterised in that** the compound comprises at least one of the structures of the formulae (IIIa) and/or (IVa)

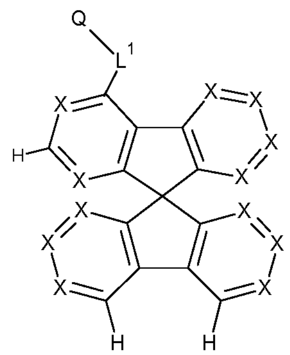

formula (IIIa)

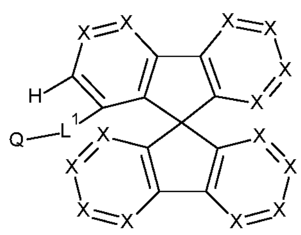

formula (IVa)

in which the symbols X, $R^1$, $L^1$ and Q have the meaning described in Claim 1 or 2.

3. Compound according to Claim 1 or 2, **characterised in that**, in formulae (III), (IIIa), (IV) and (IVa), not more than two, preferably not more than one, group X stands for N, preferably all X stand for $CR^1$, where preferably at most

4, particularly preferably at most 3 and especially preferably at most 2 of the groups $CR^1$ for which X stands is not equal to the group CH.

4. Compound according to one or more of Claims 1-3, **characterised in that** the compound comprises at least one of the structures of the formulae (IIIb) and/or (IVb)

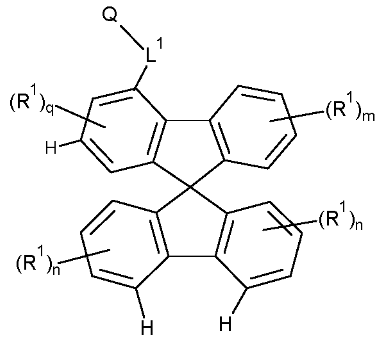

formula (IIIb)

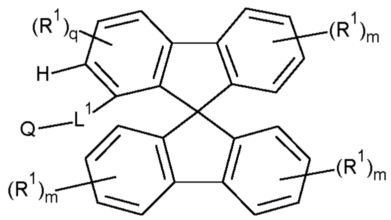

formula (IVb)

where the symbols Q, $L^1$ and $R^1$ have the meaning given in Claim 1, m is 0, 1, 2, 3 or 4, preferably 0, 1 or 2, n is 0, 1, 2 or 3, preferably 0, 1 or 2, and q is 0, 1 or 2, preferably 0 or 1.

5. Compounds according to one or more of Claims 1-4, **characterised in that** the group Q is a heteroaromatic ring system having at least two condensed rings, which may be substituted by one or more radicals $R^1$, where the ring atoms of the at least two condensed rings include at least one nitrogen atom.

6. Compounds according to one or more of Claims 1-5, **characterised in that** the group Q represents a heteroaromatic ring system having 9 to 14, preferably 10, ring atoms, which may be substituted by one or more radicals $R^1$.

7. Compound according to one or more of Claims 1-6, **characterised in that** the group Q is selected from structures oft he formulae (Q-4), (Q-5), (Q-6), (Q-7), (Q-8), (Q-9), (Q-10), (Q-11), (Q-12), (Q-13), (Q-14) and/or (Q-15)

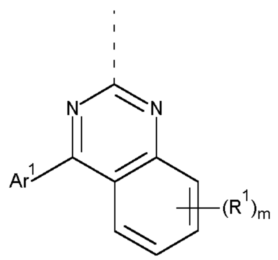

formula (Q-4)

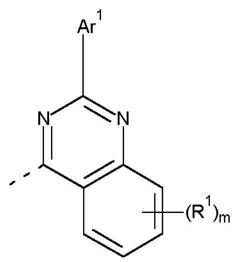

formula (Q-5)

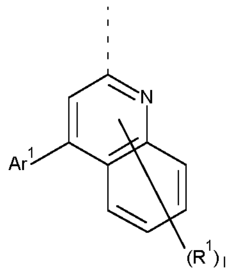

formula (Q-6)

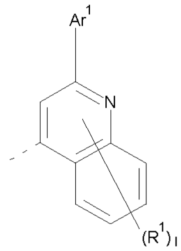

formula (Q-7)

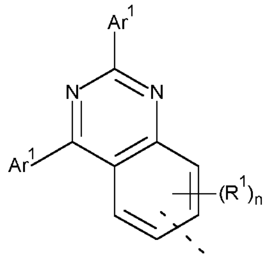

formula (Q-8)

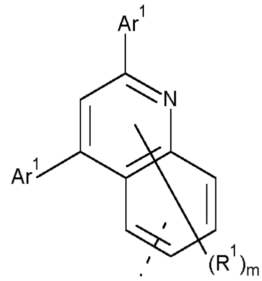

formula (Q-9)

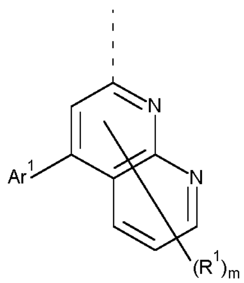

formula (Q-10)

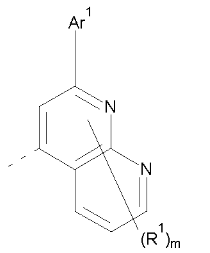

formula (Q-11)

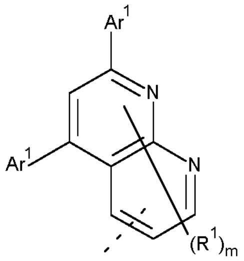

formula (Q-12)

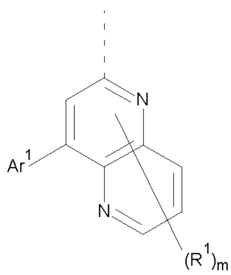

formula (Q-13)

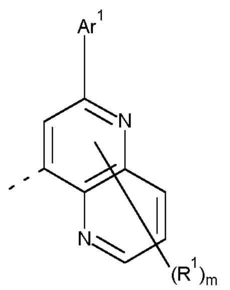

formula (Q-14)

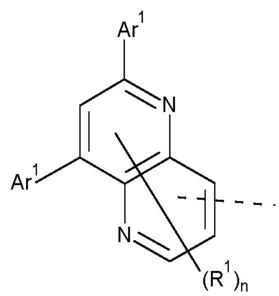

formula (Q-15)

in which the symbols $Ar^1$ and $R^1$ have the meaning described in Claim 8, the dashed bond marks the bonding position and m is 0, 1, 2, 3 or 4, preferably 0, 1 or 2, n is 0, 1, 2 or 3, preferably 0 or 1, and l is 1, 2, 3, 4 or 5, preferably 0, 1 or 2.

**8.** Compound according to at least one of the preceding Claims 6 and 7, **characterised in that** $Ar^1$ and/or a substituent $R^2$ of the formulae (Q-1) to (Q-15) which is bonded to $Ar^1$ comprises a structural element selected from structures of the formulae (Q-1) to (Q-15) and/or from structures of the formula (Q-16) or (Q-17)

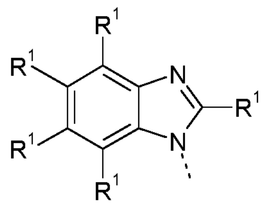

formula (Q-16)

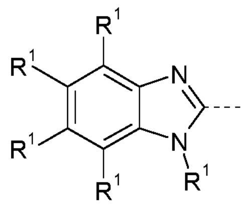

formula (Q-17)

where the symbol $R^1$ has the meaning given in Claim 1 and the dashed bonds in each case mark the bonding positions at which the structural element of the formula (Q-16) or (Q-17) is bonded to other structural elements of the radical $Ar^1$ or to the substituent $R^2$ or to a structure of the formulae (Q-1) to (Q-15).

**9.** Compound according to one or more of Claims 1-8, **characterised in that** the compound does not comprise a carbazole and/or triarylamine group.

**10.** Compound according to one or more of Claims 1-9, **characterised in that** the compound does not comprise a hole-transporting group.

**11.** Oligomer, polymer or dendrimer containing one or more compounds according to one or more of Claims 1-10, where one or more bonds are present from the compound to the polymer, oligomer or dendrimer.

**12.** Composition comprising at least one compound according to one or more of Claims 1-10 and/or an oligomer, polymer or dendrimer according to Claim 11 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**13.** Formulation comprising at least one compound according to one or more of Claims 1-10, an oligomer, polymer or dendrimer according to Claim 11 and/or at least one composition according to Claim 12 and at least one solvent.

**14.** Process for the preparation of a compound according to one or more of Claims 1-10 or an oligomer, polymer or dendrimer according to Claim 11, **characterised in that** a compound comprising at least one electron-transporting group is reacted with a compound comprising at least one fluorene radical in a coupling reaction.

**15.** Use of a compound according to one or more of Claims 1-10, an oligomer, polymer or dendrimer according to Claim

11 or a composition according to Claim 12 as hole-blocking material, electron-injection material and/or electron-transport material in an electronic device.

**16.** Electronic device containing at least one compound according to one or more of Claims 1-10, an oligomer, polymer or dendrimer according to Claim 11 or a composition according to Claim 12, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Composé comprenant les structures de la formule (III) et/ou de la formule (IV)

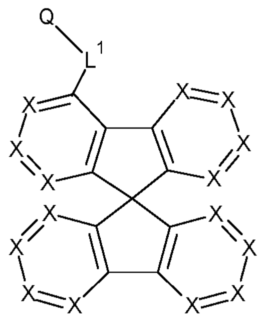

formule (III)

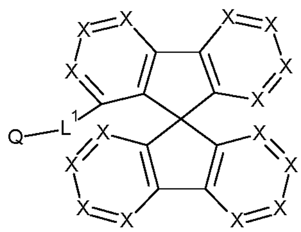

formule (IV)

dans lesquelles ce qui suit s'applique aux symboles qui sont utilisés :

X est pour chaque occurrence, de manière identique ou différente, N ou $CR^1$, de préférence $CR^1$, étant entendu que pas plus de deux des groupes X dans un cycle représentent N ;
Q est un groupe de transport d'électrons qui est sélectionné parmi les structures des formules (Q-1), (Q-2) et/ou (Q-3)

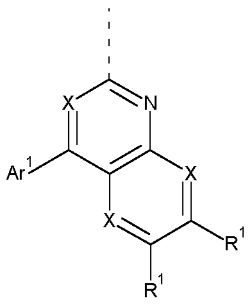

formule (Q-1)

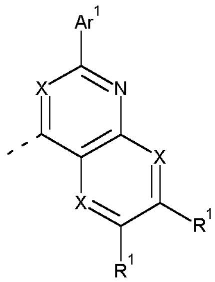

formule (Q-2)

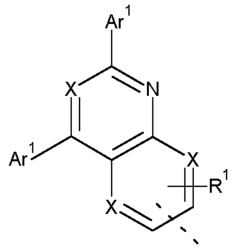

formule (Q-3)

dans lesquelles le lien en pointillés indique la position de liaison et $Ar^1$ représente un système de cycle aromatique ou hétéroaromatique qui comporte de 6 à 40 atomes de C, lequel peut dans chaque cas être substitué par un radical ou par plusieurs radicaux $R^2$, un groupe aryloxy qui comporte de 5 à 60 atomes de cycle aromatique, lequel peut être substitué par un radical ou par plusieurs radicaux $R^2$, ou un groupe aralkyle qui comporte de 5 à 60 atomes de cycle aromatique, lequel peut dans chaque cas être substitué par un radical ou par plusieurs radicaux $R^2$, où deux substituants $R^1$ ou $R^2$ adjacents ou plus peuvent en option former un système de cycle aliphatique mono- ou polycyclique, lequel peut être substitué par un radical ou par plusieurs radicaux $R^3$ ;

$L^1$ est une liaison, C(=O) ou un système de cycle aromatique qui comporte de 5 à 24 atomes de cycle aromatique, lequel peut être substitué par un radical ou par plusieurs radicaux $R^1$ ;

$R^1$ est pour chaque occurrence, de manière identique ou différente, H, D, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $-R^2C=CR^2-$, -C≡C-, $Si(R^2)_2$, C=O, C=S, $C=NR^2$, -C(=O)O-, $-C(=O)NR^2-$, $NR^2$, $P(=O)(R^2)$, -O-, -S-, SO ou $SO_2$ et où un ou plusieurs atome(s) de H peut/peuvent être remplacé(s) par D, F, Cl, Br, I, CN ou $NO_2$, ou des systèmes de cycle aromatique ou hétéroaromatique qui comportent de 5 à 40 atomes de cycle aromatique, lesquels peuvent dans chaque cas être substitués par un radical ou par plusieurs radicaux $R^2$, ou un groupe aryloxy ou hétéroaryloxy qui comporte de 5 à 40 atomes de cycle aromatique, lequel peut être substitué par un radical ou par plusieurs radicaux $R^2$, ou une combinaison de ces systèmes ; deux substituants adjacents $R^1$ ou plus peuvent également former un système de cycle aliphatique ou aromatique mono- ou polycyclique l'un avec l'autre ou les uns avec les autres ;

$R^2$ est pour chaque occurrence, de manière identique ou différente, H, D, un groupe alkyle, alcoxy ou thioalcoxy en chaîne droite qui comporte de 1 à 40 atome(s) de C ou un groupe alkyle, alcoxy ou thioalcoxy ramifié ou cyclique qui comporte de 3 à 40 atomes de C, lequel peut dans chaque cas être substitué par un radical ou par plusieurs radicaux $R^3$, où un ou plusieurs groupe(s) $CH_2$ non adjacents peut/peuvent être remplacé(s) par $-R^3C=CR^3-$, -C≡C-, $Si(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 40 atomes de cycle aromatique, lequel peut dans chaque cas être substitué par un radical ou par plusieurs radicaux $R^3$, ou un groupe aryloxy ou hétéroaryloxy qui comporte de 5 à 40 atomes de cycle aromatique, lequel peut être substitué par un radical ou par plusieurs radicaux $R^3$, ou une combinaison de ces systèmes ; deux substituants $R^2$ adjacents ou plus peuvent également former un système de cycle aliphatique ou aromatique mono- ou polycyclique l'un avec l'autre ou les uns avec les autres ;

$R^3$ est pour chaque occurrence, de manière identique ou différente, H, D, F ou un radical hydrocarbone aliphatique qui comporte de 1 à 20 atome(s) de C, où un ou plusieurs atome(s) de H peut/ peuvent être remplacé(s) par D ou par F, ou un système de cycle aromatique et/ou hétéroaromatique qui comporte de 5 à 30 atomes de carbone, où un ou plusieurs atome(s) de H peut/ peuvent être remplacé(s) par D ou par F ; deux substituants $R^3$ adjacents ou plus peuvent également former un système de cycle aliphatique ou aromatique mono- ou polycyclique l'un avec l'autre ou les uns avec les autres.

2. Composé selon la revendication 1, **caractérisé en ce que** le composé comprend au moins l'une des structures des formules (IIIa) et/ou (IVa)

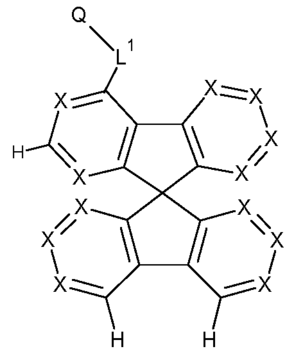

formule (IIIa)

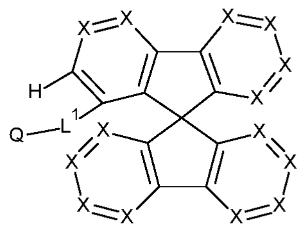

formule (IVa)

dans lesquelles les symboles X, $R^1$, $L^1$ et Q présentent la signification qui a été décrite selon la revendication 1 ou 2.

3. Composé selon la revendication 1 ou 2, **caractérisé en ce que**, dans les formules (III), (IIIa), (IV) et (IVa), pas plus de deux groupes X, de préférence pas plus d'un groupe X représentent/représente N, de préférence tous les groupes X représentent $CR^1$, où de préférence, au plus 4, de façon particulièrement préférable, au plus 3 et de façon tout particulièrement préférable, au plus 2 des groupes $CR^1$ que X représente ne sont pas égaux au groupe CH.

4. Composé selon une ou plusieurs des revendications 1 à 3, **caractérisé en ce que** le composé comprend au moins l'une des structures des formules (IIIb) et/ou (IVb)

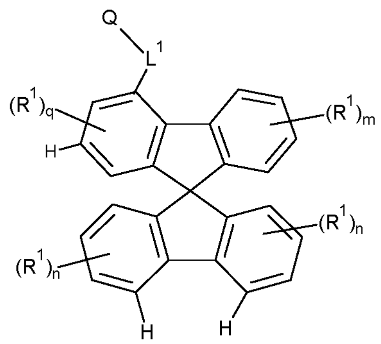

formule (IIIb)

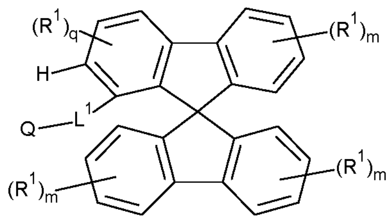

formule (IVb)

dans lesquelles les symboles Q, $L^1$ et $R^1$ présentent la signification qui a été donnée selon la revendication 1, m est 0, 1, 2, 3 ou 4, de préférence 0, 1 ou 2, n est 0, 1, 2 ou 3, de préférence 0, 1 ou 2, et q est 0, 1 ou 2, de préférence 0 ou 1.

5. Composé selon une ou plusieurs des revendications 1 à 4, **caractérisé en ce que** le groupe Q est un système de cycle hétéroaromatique qui comporte au moins deux cycles condensés, lequel peut être substitué par un radical ou par plusieurs radicaux $R^1$, où les atomes de cycle des au moins deux cycles condensés incluent au moins un atome d'azote.

6. Composé selon une ou plusieurs des revendications 1 à 5, **caractérisé en ce que** le groupe Q représente un système de cycle hétéroaromatique qui comporte de 9 à 14, de préférence 10, atomes de cycle, lequel peut être substitué par un radical ou par plusieurs radicaux $R^1$.

7. Composé selon une ou plusieurs des revendications 1 à 6, **caractérisé en ce que** le groupe Q est sélectionné parmi les structures des formules (Q-4), (Q-5), (Q-6), (Q-7), (Q-8), (Q-9), (Q-10), (Q-11), (Q-12), (Q-13), (Q-14) et/ou (Q-15)

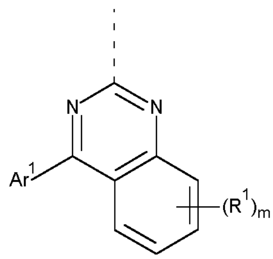

formule (Q-4)

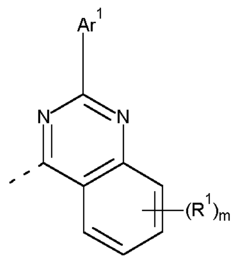

formule (Q-5)

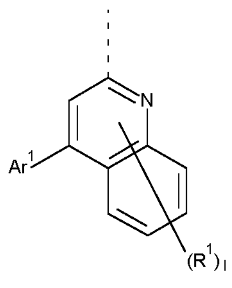

formule (Q-6)

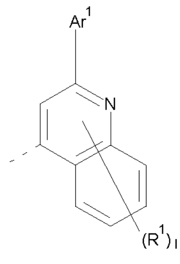

formule (Q-7)

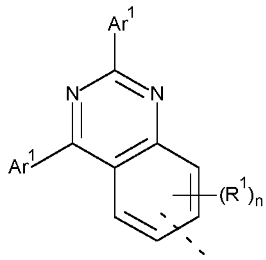

formule (Q-8)

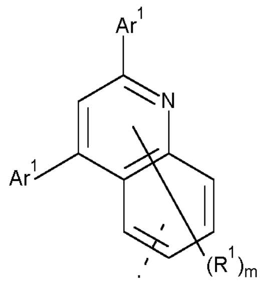

formule (Q-9)

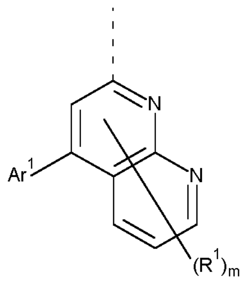

formule (Q-10)

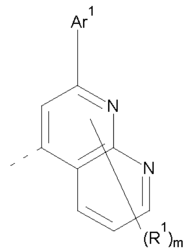

formule (Q-11)

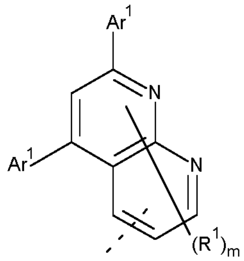

formule (Q-12)

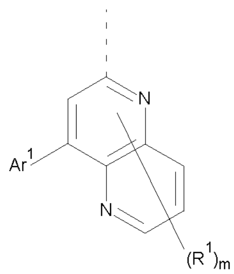

formule (Q-13)

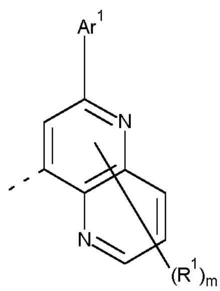

formule (Q-14)

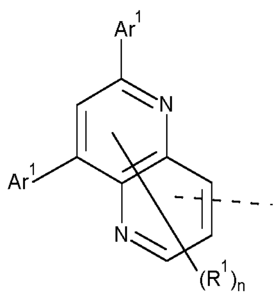

formule (Q-15)

dans lesquelles les symboles $Ar^1$ et $R^1$ présentent la signification qui a été décrite selon la revendication 8, le lien en pointillés indique la position de liaison et m est 0, 1, 2, 3 ou 4, de préférence 0, 1 ou 2, n est 0, 1, 2 ou 3, de préférence 0 ou 1, et l est 1, 2, 3, 4 ou 5, de préférence 0, 1 ou 2.

**8.** Composé selon au moins l'une des revendications précédentes 1 à 6 et 7, **caractérisé en ce que** $Ar^1$ et/ou un substituant $R^2$ des formules (Q-1) à (Q-15) qui est lié à $Ar^1$ comprennent/comprend un élément structurel qui est sélectionné parmi les structures des formules (Q-1) à (Q-15) et/ou parmi les structures de la formule (Q-16) ou (Q-17)

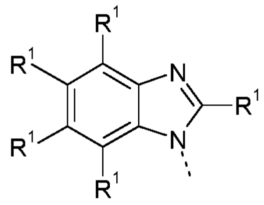

formule (Q-16)

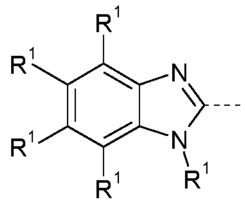

formule (Q-17)

dans lesquelles le symbole $R^1$ présente la signification qui a été donnée selon la revendication 1 et les liens en pointillés indiquent dans chaque cas les positions de liaison au niveau desquelles l'élément structurel de la formule (Q-16) ou (Q-17) est lié à d'autres éléments structurels du radical $Ar^1$ ou du substituant $R^2$ ou à une structure des formules (Q-1) à (Q-15).

9. Composé selon une ou plusieurs des revendications 1 à 8, **caractérisé en ce que** le composé ne comprend pas un groupe carbazole et/ou triarylamine.

10. Composé selon une ou plusieurs des revendications 1 à 9, **caractérisé en ce que** le composé ne comprend pas un groupe de transports de trous.

11. Oligomère, polymère ou dendrimère contenant un ou plusieurs composé(s) selon une ou plusieurs des revendications 1 à 10, dans lequel une ou plusieurs liaison(s) est/sont présentes depuis le composé jusqu'au polymère, à l'oligomères ou au dendrimère.

12. Composition comprenant au moins un composé selon une ou plusieurs des revendications 1 à 10 et/ou un oligomère, un polymère ou un dendrimère selon la revendication 11 et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

13. Formulation comprenant au moins un composé selon une ou plusieurs des revendications 1 à 10, un oligomère, un polymère ou un dendrimère selon la revendication 11 et/ou au moins une composition selon la revendication 12 et au moins un solvant.

14. Procédé pour la préparation d'un composé selon une ou plusieurs des revendications 1 à 10 ou d'un oligomère, d'un polymère ou d'un dendrimère selon la revendication 11, **caractérisé en ce qu'**un composé qui comprend au moins un groupe de transports d'électrons est amené à réagir avec un composé qui comprend au moins un radical fluorène selon une réaction de couplage.

15. Utilisation d'un composé selon une ou plusieurs des revendications 1 à 10, d'un oligomère, d'un polymère ou d'un dendrimère selon la revendication 11 ou d'une composition selon la revendication 12 en tant que matériau de blocage de trous, matériau d'injection d'électrons et/ou matériau de transport d'électrons dans un dispositif électronique.

16. Dispositif électronique contenant au moins un composé selon une ou plusieurs des revendications 1 à 10, un oligomère, un polymère ou un dendrimère selon la revendication 11 ou une composition selon la revendication 12, dans lequel le dispositif électronique est de préférence sélectionné parmi le groupe qui est constitué par les dispositifs électroluminescents organiques, les circuits intégrés organiques, les transistors à effet de champ organiques, les transistors à film mince organiques, les transistors à émission de lumière ou électroluminescents organiques, les cellules solaires organiques, les détecteurs optiques organiques, les photorécepteurs organiques, les dispositifs à extinction de champ organiques, les cellules électrochimiques à émission de lumière ou électroluminescentes ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4539507 A
- US 5151629 A
- EP 0676461 A
- WO 9827136 A
- WO 2010015306 A
- WO 2010072300 A
- JP 2014183315 A
- EP 2842954 A1
- WO 2012165832 A1
- WO 2012141499 A1
- KR 20120116272 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- EP 13004411
- EP 14000345
- EP 14000417
- EP 14002623
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- EP 14002104
- WO 2010108579 A
- WO 2005053051 A
- WO 2009030981 A

**In der Beschreibung aufgeführte Nicht-Patentliteratur**


- **D. M. KOLLER et al.** organic plasmon emitting devices. *Nature Photonics,* 2008, 1-4
- **M. S. ARNOLD et al.** *Appl. Phys. Lett.,* 2008, vol. 92, 053301